# EUROPEAN PATENT SPECIFICATION

(11) **EP 4 431 500 B1**
(45) Date of publication and mention of the grant of the patent: **05.08.2026**
(21) Application number: 22919025.1
(22) Date of filing: 06.12.2022
(51) Int. Cl.: C07D 307/79, C07D 333/54, C07D 405/04, C07D 409/04, H10K 50/11, H10K 99/00, C09K 11/06

(54) **COMPOUND AND ORGANIC LIGHT-EMITTING DEVICE COMPRISING SAME**
VERBINDUNG UND ORGANISCHE LICHTEMITTIERENDE VORRICHTUNG DAMIT
COMPOSÉ ET DISPOSITIF ÉLECTROLUMINESCENT ORGANIQUE LE COMPRENANT

(30) Priority: 07.01.2022 KR 20220002817
(43) Date of publication of application: 18.09.2024
(73) Proprietor: LG Chem, Ltd., Yeongdeungpo-gu Seoul 07336 (KR)
(72) Inventor: HA, Jae Seung, Daejeon 34122 (KR); GEUM, Sujeong, Daejeon 34122 (KR); KIM, Seonwoo, Daejeon 34122 (KR); LEE, Woochul, Daejeon 34122 (KR); CHO, Woo Jin, Daejeon 34122 (KR); CHA, Yongbum, Daejeon 34122 (KR)
(74) Representative: Hoffmann Eitle
(86) International application number: PCT/KR2022/019720
(87) International publication number: WO 2023/132488

(56) References cited:
- EP-A1- 4 011 872
- EP-A1- 4 137 486
- EP-A1- 4 299 568
- WO-A1-2021/021840
- WO-A1-2022/191570
- CN-A- 113 149 943
- KR-A- 20100 017 136
- KR-A- 20190 056 338
- KR-A- 20210 125 937
- KR-A- 20210 125 937
- KR-B1- 101 356 654
- US-A1- 2023 111 485

## Description

### Technical Field

This application claims priority to and the benefit of Korean Patent Application No. 10-2022-0002817 filed in the Korean Intellectual Property Office on January 7, 2022.

The present specification relates to a compound and an organic light emitting device.

### Background Art

An organic light emission phenomenon generally refers to a phenomenon converting electrical energy to light energy using an organic material. An organic light emitting device using an organic light emission phenomenon normally has a structure including a positive electrode, a negative electrode, and an organic material layer therebetween. Here, the organic material layer has in many cases a multi-layered structure composed of different materials in order to improve the efficiency and stability of the organic light emitting device, and for example, may be composed of a hole injection layer, a hole transport layer, a light emitting layer, an electron transport layer, an electron injection layer, and the like. In such a structure of the organic light emitting device, if a voltage is applied between the two electrodes, holes are injected from the positive electrode into the organic material layer and electrons are injected from the negative electrode into the organic material layer, and when the injected holes and electrons meet each other, an exciton is formed, and light is emitted when the exciton falls down again to a ground state.

There is a continuous need for developing a new material for the aforementioned organic light emitting device.

KR 2021-0125937 A, WO 2022/191570 A, EP 4 011 872 A, CN 113149943, KR 2019-0056338 A, WO 2021/021840 A and KR 101 356 654 A represent background art.

### Detailed Description of the Invention

### Technical Problem

The present specification provides a compound and an organic light emitting device.

### Technical Solution

An aspect of the invention is a compound selected from the following:

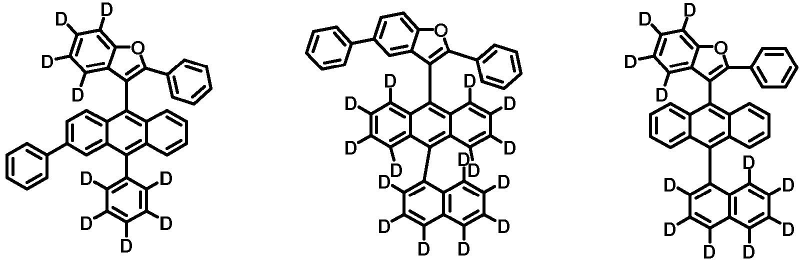

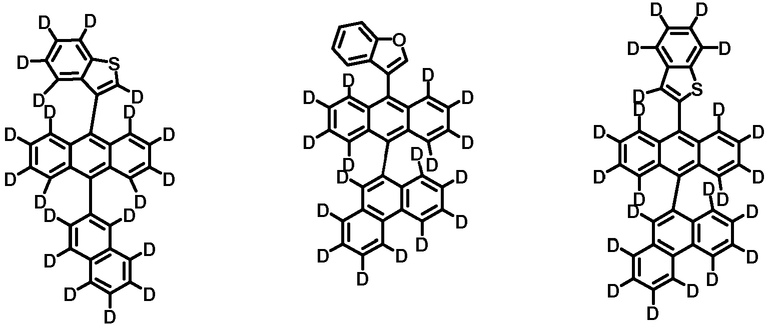

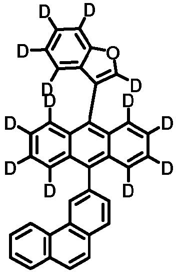

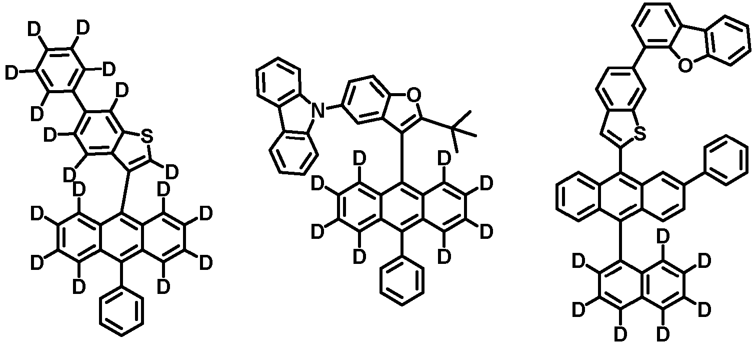

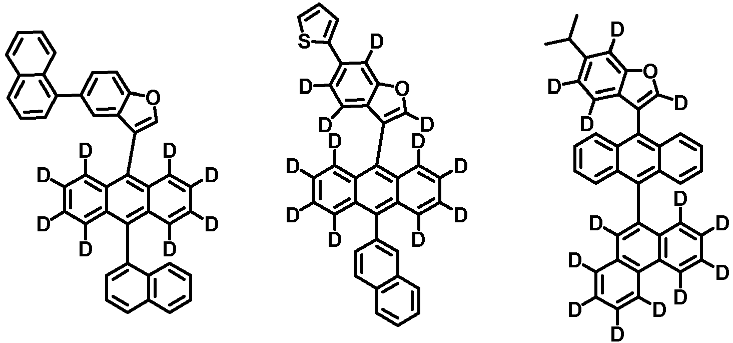

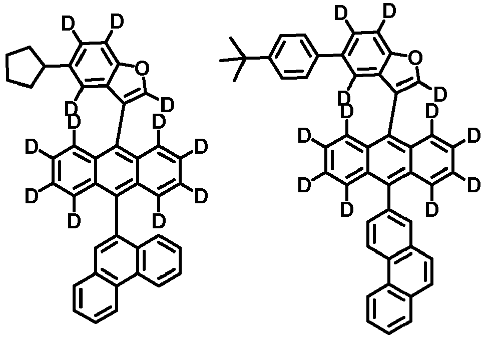

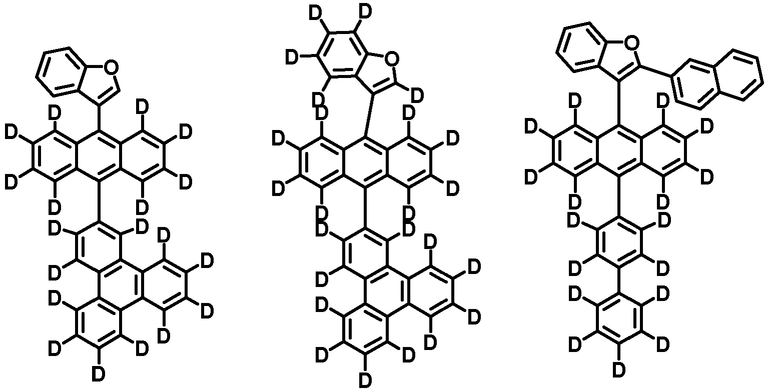

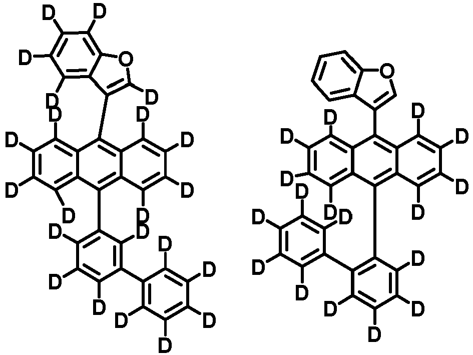

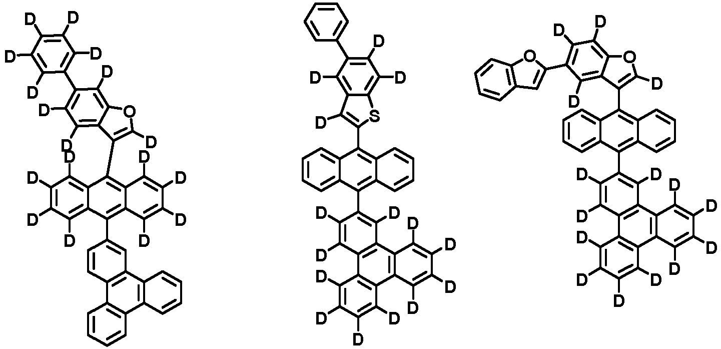

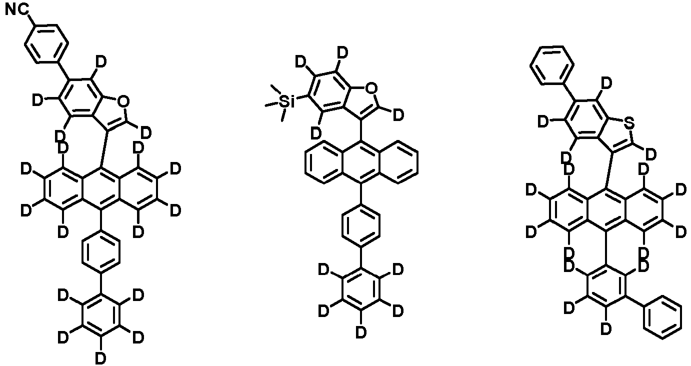

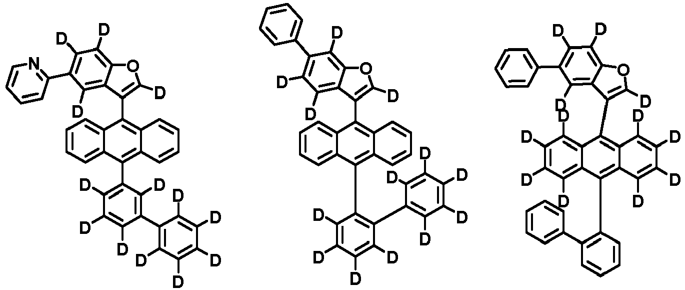

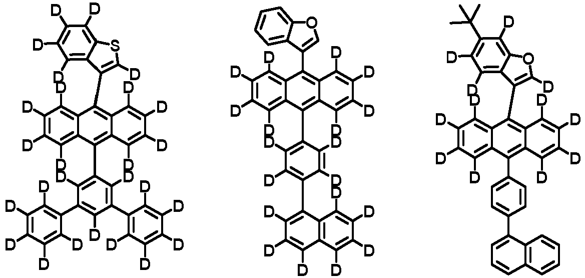

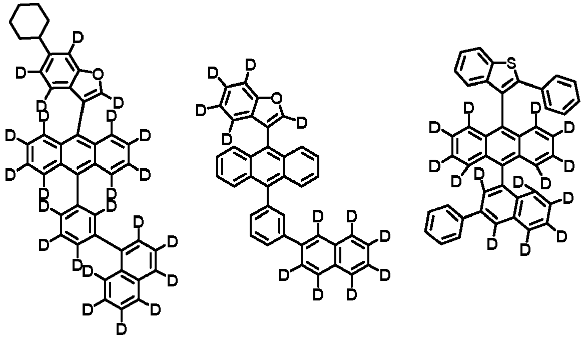

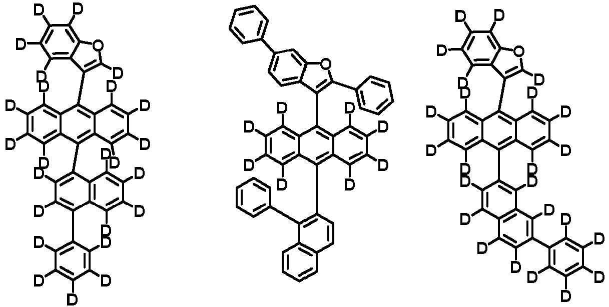

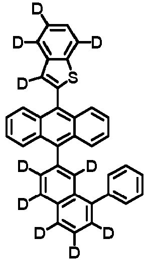

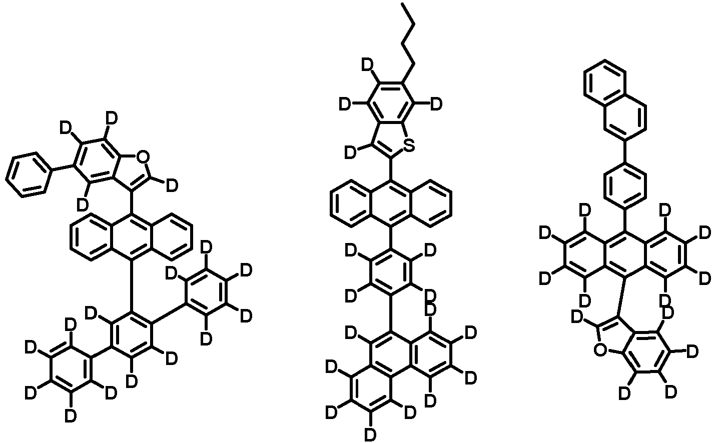

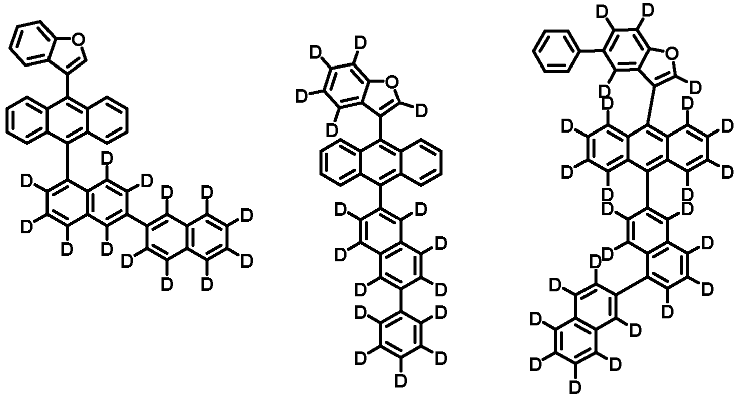

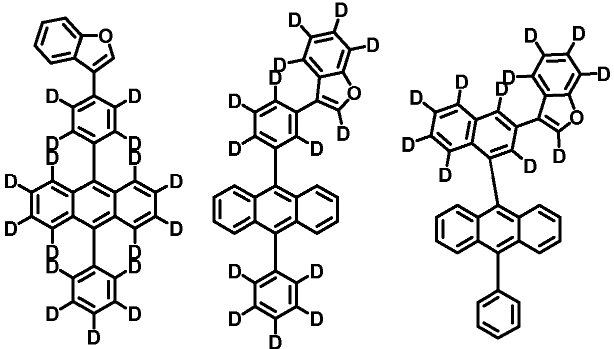

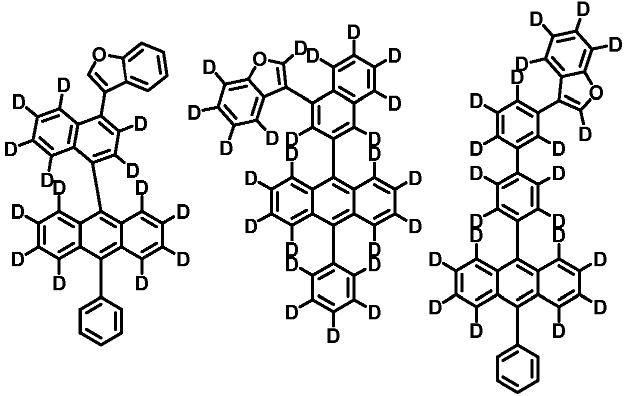

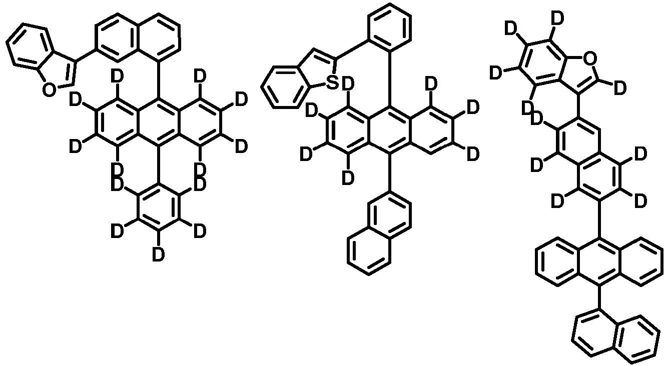

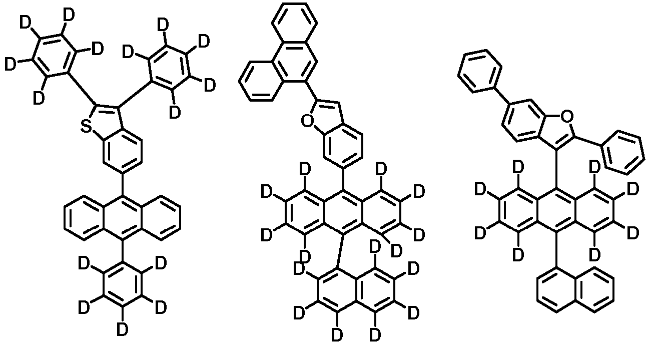

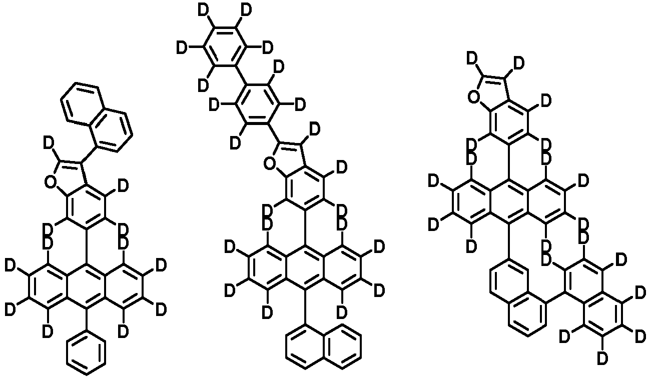

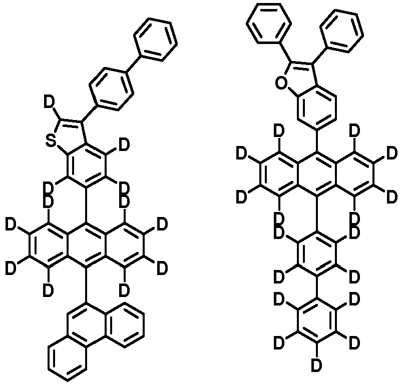

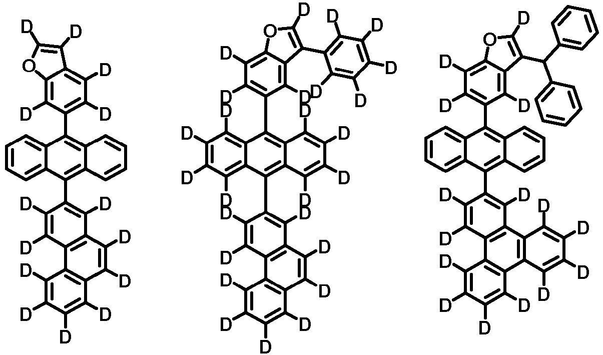

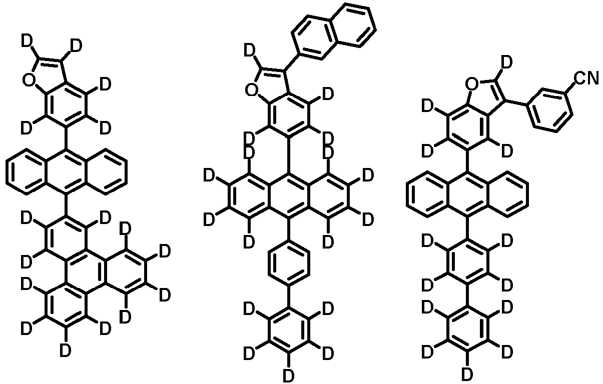

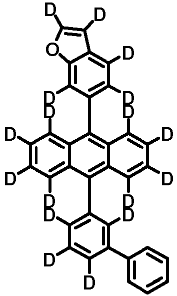

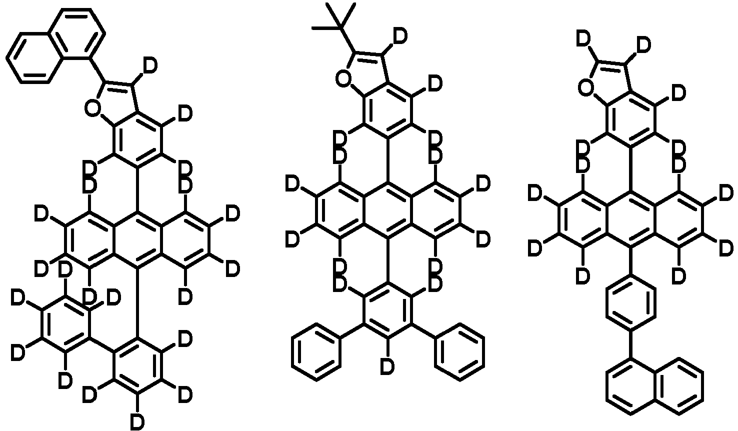

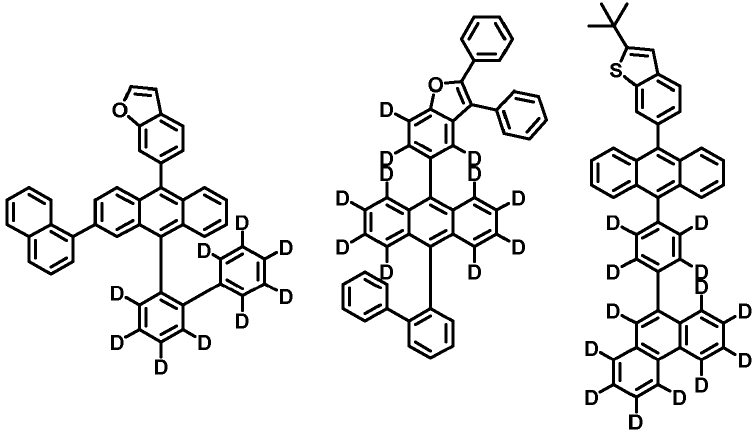

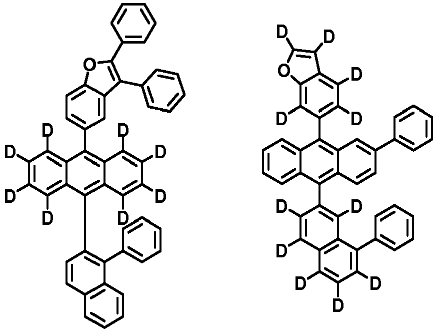

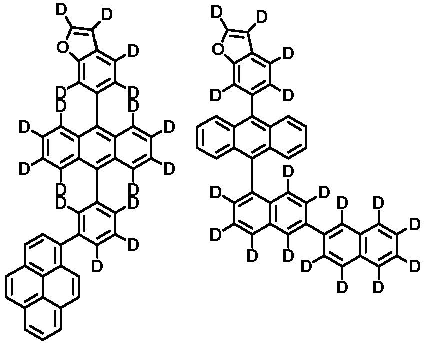

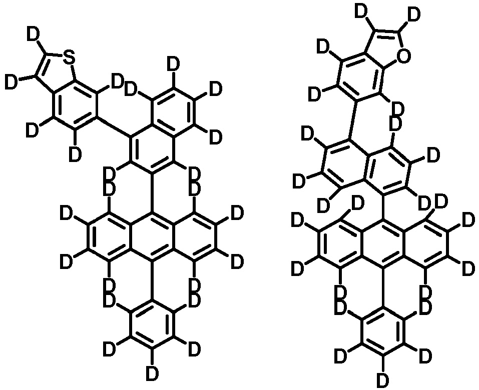

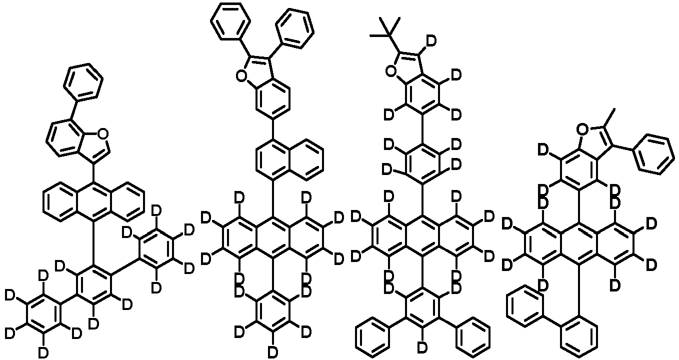

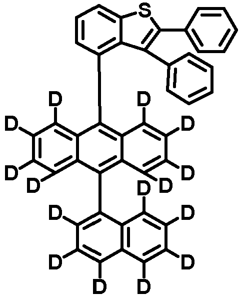

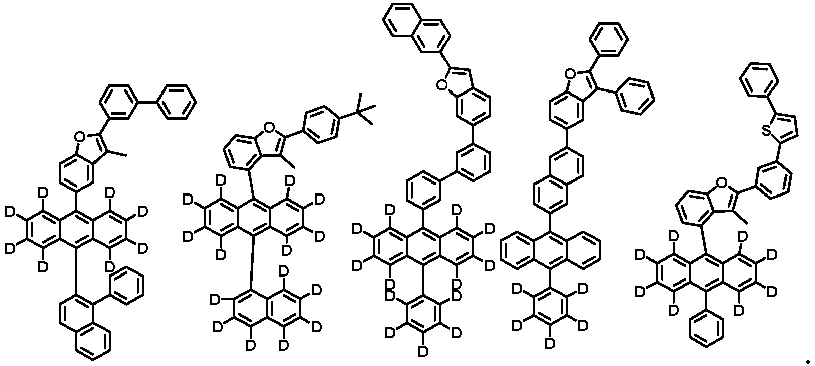

Another aspect of the invention is an organic light emitting device comprising a first electrode, a second electrode, and one or more organic material layers provided between the first electrode and the second electrode; wherein the one or more organic material layers comprise a light emitting layer and an electron transport layer; the light emitting layer comprises a compound of any one of the following Chemical Formulae 1-1 to 1-5 as a host compound; and the electron transport layer comprises a compound of the following Chemical Formula ET-1: 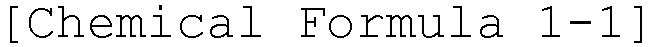 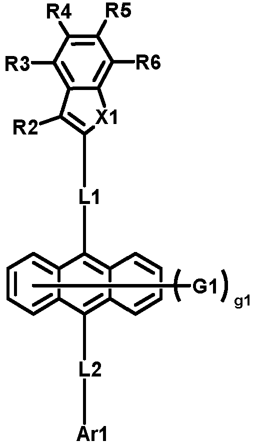 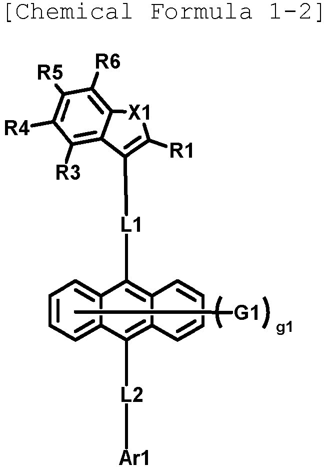 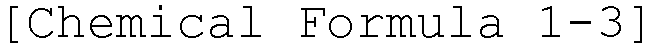 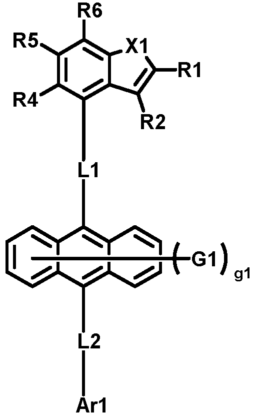 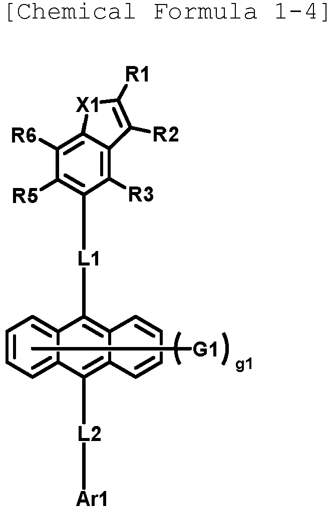 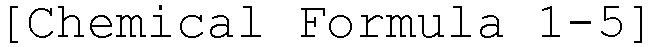 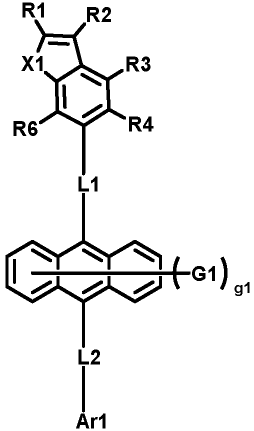 in Chemical Formulae 1-1 to 1-5,
R1 to R6 are the same as or different from each other, and are each independently hydrogen, deuterium, a halogen group, a cyano group, a substituted or unsubstituted alkyl group, a substituted or unsubstituted cycloalkyl group, a substituted or unsubstituted alkoxy group, a substituted or unsubstituted alkenyl group, a substituted or unsubstituted silyl group, a substituted or unsubstituted aryl group, or a substituted or unsubstituted heterocyclic group;
L1 and L2 are the same as or different from each other, and are each independently a direct bond, a substituted or unsubstituted arylene group, or a substituted or unsubstituted divalent heterocyclic group;
Ar1 is a substituted or unsubstituted aryl group;
each G1 is independently hydrogen, deuterium, a halogen group, a cyano group, a substituted or unsubstituted alkyl group, a substituted or unsubstituted cycloalkyl group, a substituted or unsubstituted alkoxy group, a substituted or unsubstituted alkenyl group, a substituted or unsubstituted silyl group, a substituted or unsubstituted aryl group, or a substituted or unsubstituted heterocyclic group;
g1 is 8;
G1's are the same as or different from each other;
in Chemical Formulae 1-2 to 1-6, X1 is O or S;
in Chemical Formula 1-1, X1 is S;
in Chemical Formulae 1-3 and 1-4, R2 is a substituted or unsubstituted alkyl group, a substituted or unsubstituted cycloalkyl group, a substituted or unsubstituted alkoxy group, a substituted or unsubstituted alkenyl group, a substituted or unsubstituted silyl group, a substituted or unsubstituted aryl group, or a substituted or unsubstituted heterocyclic group; and
Chemical Formulae 1-1 to 1-5 comprise deuterium, wherein the deuterium substitution rate is 40% to 100%; 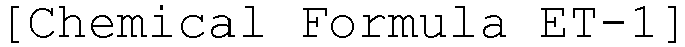 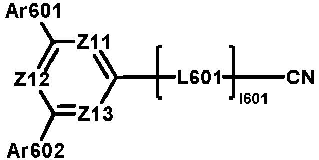
in Chemical Formula ET-1,
at least one of Z11 to Z13 is N, and the others are CH;
L601 is a direct bond, a substituted or unsubstituted arylene group, or a substituted or unsubstituted heteroarylene group;
Ar601 and Ar602 are the same as or different from each other, and are each independently a substituted or unsubstituted aryl group, or a substituted or unsubstituted heteroaryl group; and
l601 is an integer from 1 to 5, and when l601 is 2 or higher, the two or more L601s are the same as or different from each other.

### Advantageous Effects

The compound according to the present specification can be used for an organic light emitting device to lower the driving voltage of the organic light emitting device and improve the light efficiency. Further, the service life characteristics of the device can be improved by the thermal stability of the compound.

### Brief Description of Drawings

FIGS. 1 and 2 illustrate an example of an organic light emitting device according to an exemplary embodiment of the present specification.
FIG. 3 is an MS graph of Compound A.

### [Explanation of Reference Numerals and Symbols]

1: Substrate
2: First electrode
3: Second electrode
4: Light Emitting Layer
5: Hole injection layer
6: Hole Transport layer
7: Hole adjusting layer
8: Electron adjusting layer
9: Electron transport layer
10: Electron injection layer
11: Capping layer

### Best Mode

Hereinafter, the present specification will be described in more detail.

The compounds of the invention have excellent electron transfer and hole transfer ability and high quantum efficiency. Therefore, by applying the compounds the organic material layer of an organic light emitting device, it is possible to obtain the effects of reducing the driving voltage, improving the efficiency, and prolonging the service life.

Throughout the specification of the present application, the term "combination thereof" included in the Markush type expression means a mixture or combination of one or more selected from the group consisting of constituent elements described in the Markush type expression, and means including one or more selected from the group consisting of the above-described constituent elements.

Examples of the substituents in the present specification will be described below, but are not limited thereto.

In the present specification, 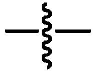 means a moiety to be linked.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, the term "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of an aromatic hydrocarbon ring and an aliphatic hydrocarbon ring and a heterocyclic group, being substituted with a substituent to which two or more substituents among the exemplified substituents are linked, or having no substituent.

In the present specification, the fact that two or more substituents are linked indicates that hydrogen of any one substituent is linked to another substituent. For example, when two substituents are linked to each other, a phenyl group and a naphthyl group may be linked to each other to become a substituent of 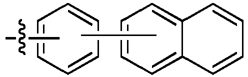 or 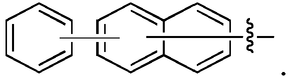 Further, the case where three substituents are linked to one another includes not only a case where (Substituent 1)-(Substituent 2)-(Substituent 3) are consecutively linked to one another, but also a case where (Substituent 2) and (Substituent 3) are linked to (Substituent 1). For example, a phenyl group, a naphthyl group, and an isopropyl group may be linked to one another to become a substituent of 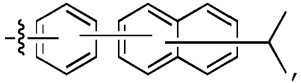 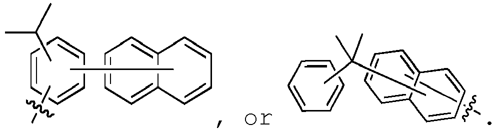 The above-described definition also applies equally to the case where four or more substituents are linked to one another.

In the present specification, examples of a halogen group include fluorine, chlorine, bromine or iodine.

In the present specification, the alkyl group may be straight-chained or branched, and the number of carbon atoms thereof is not particularly limited, but is preferably 1 to 30. Specific examples thereof include methyl, ethyl, propyl, n-propyl, isopropyl, butyl, n-butyl, isobutyl, tert-butyl, sec-butyl, 1-methyl-butyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present specification, a cycloalkyl group is not particularly limited, but has preferably 3 to 30 carbon atoms, and specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, an adamantyl group, a bicyclo[2.2.1]heptyl group, a bicyclo[2.2.1]octyl group, a norbornyl group, and the like, but are not limited thereto.

In the present specification, the alkoxy group may be straight-chained, branched, or cyclic. The number of carbon atoms of the alkoxy group is not particularly limited, but is preferably 1 to 30. Specific examples thereof include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the alkenyl group may be straight-chained or branched, and the number of carbon atoms thereof is not particularly limited, but is preferably 2 to 30. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present specification, the haloalkyl group means that at least one halogen group is substituted instead of hydrogen in an alkyl group in the definition of the alkyl group.

In the present specification, the haloalkoxy group means that at least one halogen group is substituted instead of hydrogen in an alkoxy group in the definition of the alkoxy group.

In the present specification, an aryl group is not particularly limited, but has preferably 6 to 30 carbon atoms, and the aryl group may be monocyclic or polycyclic.

When the aryl group is a monocyclic aryl group, the number of carbon atoms is not particularly limited, but is preferably 6 to 30. Specific examples of the monocyclic aryl group include a phenyl group, a biphenyl group, a terphenyl group, and the like, but are not limited thereto.

When the aryl group is a polycyclic aryl group, the number of carbon atoms thereof is not particularly limited, but is preferably 10 to 30. Specific examples of the polycyclic aryl group include a naphthyl group, an anthracene group, a phenanthrene group, a triphenylene group, a pyrene group, a phenalene group, a perylene group, a chrysene group, a fluorene group, and the like, but are not limited thereto.

In the present specification, the fluorene group may be substituted, and adjacent groups may be bonded to each other to form a ring.

When the fluorene group is substituted or forms a ring, the fluorene group is 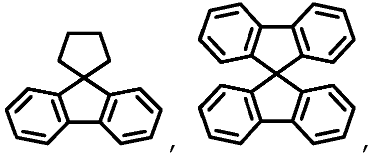 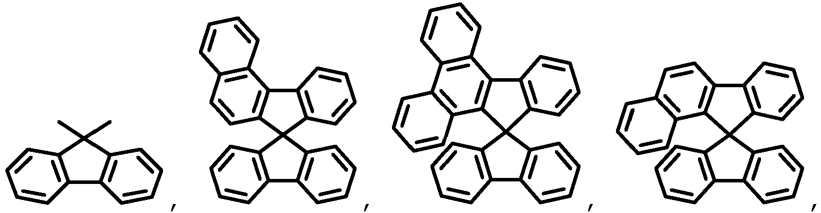 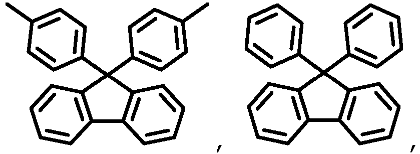 and the like, but is not limited thereto.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In the present specification, an arylalkyl group means that the alkyl group is substituted with an aryl group, and examples of the aryl group and the alkyl group described above may be applied to the aryl group and the alkyl group of the arylalkyl group.

In the present specification, an aryloxy group means that in the definition of the alkoxy group, the alkoxy group is substituted with an aryl group instead of an alkyl group, and examples of the aryloxy group include a phenoxy group, a p-tolyloxy group, an m-tolyloxy group, a 3,5-dimethyl-phenoxy group, a 2,4,6-trimethylphenoxy group, a p-tert-butylphenoxy group, a 3-biphenyloxy group, a 4-biphenyloxy group, a 1-naphthyloxy group, a 2-naphthyloxy group, a 4-methyl-1-naphthyloxy group, a 5-methyl-2-naphthyloxy group, a 1-anthryloxy group, a 2-anthryloxy group, a 9-anthryloxy group, a 1-phenanthryloxy group, a 3-phenanthryloxy group, a 9-phenanthryloxy group, and the like, but are not limited thereto.

In the present specification, the alkyl group in the alkylthioxy group is the same as the above-described examples of the alkyl group. Specific examples of the alkylthioxy group include a methylthioxy group, an ethylthioxy group, a tert-butylthioxy group, a hexylthioxy group, an octylthioxy group, and the like, but are not limited thereto.

In the present specification, the aryl group in the arylthioxy group is the same as the above-described examples of the aryl group. Specific examples of the arylthioxy group include a phenylthioxy group, a 2-methylphenylthioxy group, a 4-tert-butylphenylthioxy group, and the like, but are not limited thereto.

In the present specification, a heterocyclic group includes one or more atoms other than carbon, that is, one or more heteroatoms, and specifically, the heteroatom may include one or more atoms selected from the group consisting of O, N, Se, S, and the like, and includes an aromatic heterocyclic group, or an aliphatic heterocyclic group. The aromatic heterocyclic group may be represented by a heteroaryl group. The number of carbon atoms of the heterocyclic group is not particularly limited, but is preferably 2 to 30, and the heterocyclic group may be monocyclic or polycyclic. Examples of the heterocyclic group include a thiophene group, a furan group, a pyrrole group, an imidazole group, a thiazole group, an oxazole group, an oxadiazole group, a pyridine group, a bipyridine group, a pyrimidine group, a triazine group, a triazole group, an acridine group, a pyridazine group, a pyrazine group, a quinoline group, a quinazoline group, a quinoxaline group, a phthalazine group, a pyridopyrimidine group, a pyridopyrazine group, a pyrazinopyrazine group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthridine group, a phenanthroline group, an isoxazole group, a thiadiazole group, a dibenzofuran group, a dibenzosilole group, a phenoxathiin group, a phenoxazine group, a phenothiazine group, a decahydrobenzocarbazole group, a hexahydrocarbazole group, a dihydrobenzoazasiline group, a dihydroindenocarbazole group, a spirofluorenexanthene group, a spirofluorenethioxanthene group, a tetrahydronaphthothiophene group, a tetrahydronaphthofuran group, a tetrahydrobenzothiophene group, a tetrahydrobenzofuran group, and the like, but are not limited thereto.

In the present specification, the silyl group may be an alkylsilyl group, an arylsilyl group, an alkylarylsilyl group, a heteroarylsilyl group, and the like. The above-described examples of the alkyl group may be applied to the alkyl group in the alkylsilyl group, the above-described examples of the aryl group may be applied to the aryl group in the arylsilyl group, the examples of the alkyl group and the aryl group may be applied to the alkyl group and the aryl group in the alkylarylsilyl group, and the examples of the heterocyclic group may be applied to the heteroaryl group in the heteroarylsilyl group.

In the present specification, a boron group may be - BY₁₀₀Y₁₀₁, and Y₁₀₀ and Y₁₀₁ are the same as or different from each other, and may be each independently selected from the group consisting of hydrogen; deuterium; halogen; a nitrile group; a substituted or unsubstituted monocyclic or polycyclic cycloalkyl group having 3 to 30 carbon atoms; a substituted or unsubstituted straight-chained or branched alkyl group having 1 to 30 carbon atoms; a substituted or unsubstituted monocyclic or polycyclic aryl group having 6 to 30 carbon atoms; and a substituted or unsubstituted monocyclic or polycyclic heterocyclic group having 2 to 30 carbon atoms. Specific examples of the boron group include a dimethylboron group, a diethylboron group, a t-butylmethylboron group, a diphenylboron group, and the like, but are not limited thereto.

In the present specification, an amine group may be selected from the group consisting of -NH₂, an alkylamine group, an N-alkylarylamine group, an arylamine group, an N-arylheteroarylamine group, an N-alkylheteroarylamine group, and a heteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, an anthracenylamine group, a 9-methyl-anthracenylamine group, a diphenylamine group, a ditolylamine group, an N-phenyltolylamine group, an N-phenylbiphenylamine group, an N-phenylnaphthylamine group, an N-biphenylnaphthylamine group, an N-naphthylfluorenylamine group, an N-phenylphenanthrenylamine group, an N-biphenylphenanthrenylamine group, an N-phenylfluorenylamine group, an N-phenyl terphenylamine group, an N-phenanthrenylfluorenylamine group, an N-biphenylfluorenylamine group, and the like, but are not limited thereto.

In the present specification, an N-alkylarylamine group means an amine group in which an alkyl group and an aryl group are substituted with N of the amine group. The alkyl group and the aryl group in the N-alkylarylamine group are the same as the above-described examples of the alkyl group and the aryl group.

In the present specification, an N-arylheteroarylamine group means an amine group in which an aryl group and a heteroaryl group are substituted with N of the amine group. The aryl group and heteroaryl group in the N-arylheteroarylamine group are the same as the above-described examples of the aryl group and the heterocyclic group.

In the present specification, an N-alkylheteroarylamine group means an amine group in which an alkyl group and a heteroaryl group are substituted with N of the amine group. The alkyl group and the heteroaryl group in the N-alkylheteroarylamine group are the same as the above-described examples of the alkyl group and the heterocyclic group.

In the present specification, examples of an alkylamine group include a substituted or unsubstituted monoalkylamine group or a substituted or unsubstituted dialkylamine group. The alkyl group in the alkylamine group may be a straight-chained or branched alkyl group. The alkylamine group including two or more alkyl groups may include a straight-chained alkyl group, a branched alkyl group, or both a straight-chained alkyl group and a branched alkyl group. For example, the alkyl group in the alkylamine group may be selected from the above-described examples of the alkyl group.

In the present specification, examples of a heteroarylamine group include a substituted or unsubstituted monoheteroarylamine group or a substituted or unsubstituted diheteroarylamine group. The heteroarylamine group including two or more heteroaryl groups may include a monocyclic heteroaryl group, a polycyclic heteroaryl group, or both a monocyclic heteroaryl group and a polycyclic heteroaryl group. For example, the heteroaryl group in the heteroarylamine group may be selected from the above-described examples of the heterocyclic group.

In the present specification, the alkyl group in the N-alkylarylamine group, the alkylthioxy group, and the N-alkylheteroarylamine group is the same as the above-described examples of the alkyl group. Specific examples of the alkylthioxy group include a methylthioxy group, an ethylthioxy group, a tert-butylthioxy group, a hexylthioxy group, an octylthioxy group, and the like, but are not limited thereto.

In the present specification, the aryl group in the aryloxy group, the arylthioxy group, the N-arylalkylamine group, and the N-arylheteroarylamine group is the same as the above-described examples of the aryl group. Specifically, examples of the aryloxy group include a phenoxy group, a p-tolyloxy group, an m-tolyloxy group, a 3,5-dimethyl-phenoxy group, a 2,4,6-trimethylphenoxy group, a p-tert-butylphenoxy group, a 3-biphenyloxy group, a 4-biphenyloxy group, a 1-naphthyloxy group, a 2-naphthyloxy group, a 4-methyl-1-naphthyloxy group, a 5-methyl-2-naphthyloxy group, a 1-anthryloxy group, a 2-anthryloxy group, a 9-anthryloxy group, a 1-phenanthryloxy group, a 3-phenanthryloxy group, a 9-phenanthryloxy group, and the like, and examples of the arylthioxy group include a phenylthioxy group, a 2-methylphenylthioxy group, a 4-tert-butylphenylthioxy group, and the like, but the examples are not limited thereto.

In the present specification, a hydrocarbon ring group may be an aromatic hydrocarbon ring group, an aliphatic hydrocarbon ring group, or a condensed group of an aromatic hydrocarbon ring and an aliphatic hydrocarbon ring and may be selected among examples of the cycloalkyl group, the aryl group, and a combination thereof, and examples of the hydrocarbon ring group include a phenyl group, a cyclohexyl group, an adamantyl group, a bicylo[2.2.1]heptyl group, a bicyclo[2.2.1]octyl group, a tetrahydronaphthalene group, a tetrahydroanthracene group, a 1,2,3,4-tetrahydro-1,4-methanonaphthalene group, a 1,2,3,4-tetrahydro-1,4-ethanonaphthalene group, a spirocyclopentanefluorene group, a spiroadamantanefluorene group, a spirocyclohexanefluorene group, and the like, but are not limited thereto.

In the present specification, the meaning of "adjacent" in "bonded to an adjacent group to form a ring" is as described above, and the "ring" means a substituted or unsubstituted hydrocarbon ring; or a substituted or unsubstituted hetero ring.

In the present specification, the hydrocarbon ring may be an aromatic hydrocarbon ring, an aliphatic hydrocarbon ring, or a fused ring of the aromatic hydrocarbon ring and the aliphatic hydrocarbon ring and may be selected among examples of the cycloalkyl group, the aryl group, and a combination thereof except for those which are not monovalent, and examples of the hydrocarbon ring include benzene, cyclohexane, adamantane, bicyclo[2.2.1]heptane, bicyclo[2.2.1]octane, tetrahydronaphthalene, tetrahydroanthracene, 1,2,3,4-tetrahydro-1,4-methanonaphthalene, 1,2,3,4-tetrahydro-1,4-ethanonaphthalene, spiro cyclopentane fluorene, spiro adamantane fluorene, spiro cyclohexane fluorene, and the like, but are not limited thereto.

In the present specification, a hetero ring includes one or more atoms other than carbon, that is, one or more heteroatoms, and specifically, the heteroatom may include one or more atoms selected from the group consisting of O, N, Se, S, and the like. The hetero ring may be monocyclic or polycyclic and may be an aromatic ring, an aliphatic ring, or a fused ring of the aromatic ring and the aliphatic ring, and the aromatic hetero ring may be selected from the examples of the heteroaryl group in the heterocyclic group, except for the aromatic hetero ring which is not monovalent.

In the present specification, an aliphatic hetero ring means an aliphatic ring including one or more of hetero atoms. Examples of the aliphatic hetero ring include oxirane, tetrahydrofuran, 1,4-dioxane, pyrrolidine, piperidine, morpholine, oxepane, azocane, thiocane, tetrahydronaphthothiophene, tetrahydronaphthofuran, tetrahydrobenzothiophene, tetrahydrobenzofuran, and the like, but are not limited thereto.

In the present specification, examples of an aliphatic ring, or a fused hetero ring of the aromatic ring and the aliphatic ring include tetrahydrobenzonaphthothiophene, tetrahydrobenzonaphthofuran, and the like, but are not limited thereto.

In the present specification, an arylene group means a group having two bonding positions in an aryl group, that is, a divalent group. The above-described description on the aryl group may be applied to the arylene group, except for a divalent group.

In the present specification, a divalent heterocyclic group means that there are two bonding positions in a heterocyclic group, that is, a divalent group. The above-described description on the heterocyclic group may be applied to the heterocyclic group, except that the heterocyclic groups are each a divalent group.

Unless otherwise defined in the present specification, all technical and scientific terms used in the present specification have the same meaning as commonly understood by one with ordinary skill in the art to which the present invention pertains. Although methods and materials similar to or equivalent to those described in the present specification may be used in the practice or in the test of exemplary embodiments of the present invention, suitable methods and materials will be described below. In addition, the materials, methods, and examples are illustrative only and are not intended to be limiting.

According to an exemplary embodiment of the present specification, any one of R1 to R5 is a moiety bonded to L1, the others and R6 are the same as or different from each other, and are each independently hydrogen; deuterium; a straight-chained or branched alkyl group having 1 to 30 carbon atoms, which is unsubstituted or substituted with deuterium or a monocyclic or polycyclic aryl group having 6 to 30 carbon atoms; a monocyclic or polycyclic cycloalkyl group having 3 to 30 carbon atoms, which is unsubstituted or substituted with deuterium; a straight-chained or branched alkylsilyl group having 1 to 30 carbon atoms; a monocyclic or polycyclic aryl group having 6 to 30 carbon atoms, which is unsubstituted or substituted with deuterium, a cyano group, a straight-chained or branched alkyl group having 1 to 30 carbon atoms, or a monocyclic or polycyclic heterocyclic group having 2 to 30 carbon atoms, which is unsubstituted or substituted with a monocyclic or polycyclic aryl group having 6 to 30 carbon atoms; or a monocyclic or polycyclic heteroaryl group having 2 to 30 carbon atoms, which is unsubstituted or substituted with deuterium, L1 and L2 are the same as or different from each other, and are each independently a direct bond; or a monocyclic or polycyclic arylene group having 6 to 30 carbon atoms, which is unsubstituted or substituted with deuterium, G1 is hydrogen; deuterium; or a monocyclic or polycyclic aryl group having 6 to 30 carbon atoms, which is unsubstituted or substituted with deuterium, Ar1 is a monocyclic or polycyclic aryl group having 6 to 30 carbon atoms, which is unsubstituted or substituted with deuterium, and when R3 or R4 is a moiety bonded to L1 of, R2 is a straight-chained or branched alkyl group having 1 to 30 carbon atoms, which is unsubstituted or substituted with deuterium or a monocyclic or polycyclic aryl group having 6 to 30 carbon atoms; a monocyclic or polycyclic cycloalkyl group having 3 to 30 carbon atoms, which is unsubstituted or substituted with deuterium; a straight-chained or branched alkylsilyl group having 1 to 30 carbon atoms; a monocyclic or polycyclic aryl group having 6 to 30 carbon atoms, which is unsubstituted or substituted with deuterium, a cyano group, or a straight-chained or branched alkyl group having 1 to 30 carbon atoms; or a monocyclic or polycyclic heteroaryl group having 2 to 30 carbon atoms, which is unsubstituted or substituted with deuterium.

According to an exemplary embodiment of the present specification, any one of R1 to R5 is a moiety bonded to L1, and the others and R6 are the same as or different from each other, and are each independently hydrogen; deuterium; a substituted or unsubstituted straight-chained or branched alkyl group having 1 to 30 carbon atoms; a substituted or unsubstituted monocyclic or polycyclic cycloalkyl group having 3 to 30 carbon atoms; a substituted or unsubstituted monocyclic or polycyclic aryl group having 6 to 30 carbon atoms; or a substituted or unsubstituted monocyclic or polycyclic heteroaryl group having 2 to 30 carbon atoms.

According to an exemplary embodiment of the present specification, any one of R1 to R5 is a moiety bonded to L1, and the others and R6 are the same as or different from each other, and are each independently hydrogen; deuterium; a substituted or unsubstituted straight-chained or branched alkyl group having 1 to 20 carbon atoms; a substituted or unsubstituted monocyclic or polycyclic cycloalkyl group having 3 to 20 carbon atoms; a substituted or unsubstituted monocyclic or polycyclic aryl group having 6 to 20 carbon atoms; or a substituted or unsubstituted monocyclic or polycyclic heteroaryl group having 2 to 20 carbon atoms.

According to an exemplary embodiment of the present specification, any one of R1 to R5 is a moiety bonded to L1, and the others and R6 are the same as or different from each other, and are each independently hydrogen; deuterium; a straight-chained or branched alkyl group having 1 to 30 carbon atoms, which is unsubstituted or substituted with deuterium or a monocyclic or polycyclic aryl group having 6 to 30 carbon atoms; a monocyclic or polycyclic cycloalkyl group having 3 to 30 carbon atoms, which is unsubstituted or substituted with deuterium; a straight-chained or branched alkylsilyl group having 1 to 30 carbon atoms; a monocyclic or polycyclic aryl group having 6 to 30 carbon atoms, which is unsubstituted or substituted with a monocyclic or polycyclic heterocyclic group having 2 to 30 carbon atoms, which is unsubstituted or substituted with deuterium, a cyano group, a straight-chained or branched alkyl group having 1 to 30 carbon atoms, or a monocyclic or polycyclic aryl group having 6 to 30 carbon atoms; or a monocyclic or polycyclic heteroaryl group having 2 to 30 carbon atoms, which is unsubstituted or substituted with deuterium.

According to an exemplary embodiment of the present specification, any one of R1 to R5 is a moiety bonded to L1, and the others and R6 are the same as or different from each other, and are each independently hydrogen; deuterium; a straight-chained or branched alkyl group having 1 to 20 carbon atoms, which is unsubstituted or substituted with deuterium or a monocyclic or polycyclic aryl group having 6 to 20 carbon atoms; a monocyclic or polycyclic cycloalkyl group having 3 to 20 carbon atoms, which is unsubstituted or substituted with deuterium; a straight-chained or branched alkylsilyl group having 1 to 20 carbon atoms; a monocyclic or polycyclic aryl group having 6 to 20 carbon atoms, which is unsubstituted or substituted with a monocyclic or polycyclic heterocyclic group having 2 to 20 carbon atoms, which is unsubstituted or substituted with deuterium, a cyano group, a straight-chained or branched alkyl group having 1 to 20 carbon atoms, or a monocyclic or polycyclic aryl group having 6 to 20 carbon atoms; or a monocyclic or polycyclic heteroaryl group having 2 to 20 carbon atoms, which is unsubstituted or substituted with deuterium.

According to an exemplary embodiment of the present specification, any one of R1 to R5 is a moiety bonded to L1, and the others and R6 are the same as or different from each other, and are each independently hydrogen; deuterium; a methyl group unsubstituted or substituted with deuterium or a monocyclic or polycyclic aryl group having 6 to 30 carbon atoms; an isopropyl group unsubstituted or substituted with deuterium; an n-butyl group unsubstituted or substituted with deuterium; a tert-butyl group unsubstituted or substituted with deuterium; a cyclopentyl group unsubstituted or substituted with deuterium; a cyclohexyl group unsubstituted or substituted with deuterium; a trimethylsilyl group unsubstituted or substituted with deuterium; a phenyl group unsubstituted or substituted with deuterium, a cyano group, a straight-chained or branched alkyl group having 1 to 30 carbon atoms, or a monocyclic or polycyclic heterocyclic group having 2 to 30 carbon atoms, which is unsubstituted or substituted with a monocyclic or polycyclic aryl group having 6 to 30 carbon atoms; a biphenyl group unsubstituted or substituted with deuterium; a naphthyl group unsubstituted or substituted with deuterium; a phenanthrerne group unsubstituted or substituted with deuterium; a thiophene group unsubstituted or substituted with deuterium; a furan group unsubstituted or substituted with deuterium; a pyridine group unsubstituted or substituted with deuterium; a benzofuran group unsubstituted or substituted with deuterium; a benzothiophene group unsubstituted or substituted with deuterium; a carbazole group unsubstituted or substituted with deuterium; a dibenzofuran group unsubstituted or substituted with deuterium; or a dibenzothiophene group unsubstituted or substituted with deuterium.

According to an exemplary embodiment of the present specification, any one of R1 to R5 is a moiety bonded to L1, and the others and R6 are the same as or different from each other, and are each independently hydrogen; deuterium; a methyl group unsubstituted or substituted with deuterium or a phenyl group; an isopropyl group unsubstituted or substituted with deuterium; an n-butyl group unsubstituted or substituted with deuterium; a tert-butyl group unsubstituted or substituted with deuterium; a cyclopentyl group unsubstituted or substituted with deuterium; a cyclohexyl group unsubstituted or substituted with deuterium; a trimethylsilyl group unsubstituted or substituted with deuterium; a phenyl group unsubstituted or substituted with deuterium, a cyano group, a tert-butyl group, or a thiophene group unsubstituted or substituted with a phenyl group; a biphenyl group unsubstituted or substituted with deuterium; a naphthyl group unsubstituted or substituted with deuterium; a phenanthrene group unsubstituted or substituted with deuterium; a thiophene group unsubstituted or substituted with deuterium; a furan group unsubstituted or substituted with deuterium; a pyridine group unsubstituted or substituted with deuterium; a benzofuran group unsubstituted or substituted with deuterium; a benzothiophene group unsubstituted or substituted with deuterium; a carbazole group unsubstituted or substituted with deuterium; a dibenzofuran group unsubstituted or substituted with deuterium; or a dibenzothiophene group unsubstituted or substituted with deuterium.

According to an exemplary embodiment of the present specification, L1 and L2 are the same as or different from each other, and are each independently a direct bond; or a substituted or unsubstituted monocyclic or polycyclic arylene group having 6 to 30 carbon atoms.

According to an exemplary embodiment of the present specification, L1 and L2 are the same as or different from each other, and are each independently a direct bond; or a substituted or unsubstituted monocyclic or polycyclic arylene group having 6 to 20 carbon atoms.

According to an exemplary embodiment of the present specification, L1 and L2 are the same as or different from each other, and are each independently a direct bond; or a monocyclic or polycyclic arylene group having 6 to 30 carbon atoms, which is unsubstituted or substituted with deuterium.

According to an exemplary embodiment of the present specification, L1 and L2 are the same as or different from each other, and are each independently a direct bond; or a monocyclic or polycyclic arylene group having 6 to 20 carbon atoms, which is unsubstituted or substituted with deuterium.

According to an exemplary embodiment of the present specification, L1 and L2 are the same as or different from each other, and are each independently a direct bond; a phenylene group unsubstituted or substituted with deuterium; a biphenylylene group unsubstituted or substituted with deuterium; or a naphthylene group unsubstituted or substituted with deuterium.

According to an exemplary embodiment of the present specification, G1 is hydrogen; deuterium; or a substituted or unsubstituted monocyclic or polycyclic aryl group having 6 to 30 carbon atoms.

According to an exemplary embodiment of the present specification, G1 is hydrogen; deuterium; or a substituted or unsubstituted monocyclic or polycyclic aryl group having 6 to 20 carbon atoms.

According to an exemplary embodiment of the present specification, G1 is hydrogen; deuterium; or a monocyclic or polycyclic aryl group having 6 to 30 carbon atoms, which is unsubstituted or substituted with deuterium.

According to an exemplary embodiment of the present specification, G1 is hydrogen; deuterium; or a monocyclic or polycyclic aryl group having 6 to 20 carbon atoms, which is unsubstituted or substituted with deuterium.

According to an exemplary embodiment of the present specification, G1 is hydrogen; deuterium; a phenyl group unsubstituted or substituted with deuterium; or a naphthyl group unsubstituted or substituted with deuterium.

According to an exemplary embodiment of the present specification, Ar1 is a substituted or unsubstituted monocyclic or polycyclic aryl group having 6 to 30 carbon atoms.

According to an exemplary embodiment of the present specification, Ar1 is a substituted or unsubstituted monocyclic or polycyclic aryl group having 6 to 20 carbon atoms.

According to an exemplary embodiment of the present specification, Ar1 is a monocyclic or polycyclic aryl group having 6 to 30 carbon atoms, which is unsubstituted or substituted with deuterium.

According to an exemplary embodiment of the present specification, Ar1 is a monocyclic or polycyclic aryl group having 6 to 20 carbon atoms, which is unsubstituted or substituted with deuterium.

According to an exemplary embodiment of the present specification, Ar1 is a phenyl group unsubstituted or substituted with deuterium; a biphenyl group unsubstituted or substituted with deuterium; a terphenyl group unsubstituted or substituted with deuterium; a naphthyl group unsubstituted or substituted with deuterium; a phenanthrene group unsubstituted or substituted with deuterium; a triphenylene group unsubstituted or substituted with deuterium; or a pyrene group unsubstituted or substituted with deuterium.

According to an exemplary embodiment of the present specification, when R3 or R4 is a moiety bonded to L1 of Chemical Formula A, R2 is a substituted or unsubstituted straight-chained or branched alkyl group having 1 to 30 carbon atoms; a substituted or unsubstituted monocyclic or polycyclic cycloalkyl group having 3 to 30 carbon atoms; a substituted or unsubstituted monocyclic or polycyclic aryl group having 6 to 30 carbon atoms; or a substituted or unsubstituted monocyclic or polycyclic heteroaryl group having 2 to 30 carbon atoms.

According to an exemplary embodiment of the present specification, when R3 or R4 is a moiety bonded to L1, R2 is a substituted or unsubstituted straight-chained or branched alkyl group having 1 to 20 carbon atoms; a substituted or unsubstituted monocyclic or polycyclic cycloalkyl group having 3 to 20 carbon atoms; a substituted or unsubstituted monocyclic or polycyclic aryl group having 6 to 20 carbon atoms; or a substituted or unsubstituted monocyclic or polycyclic heteroaryl group having 2 to 20 carbon atoms.

According to an exemplary embodiment of the present specification, when R3 or R4 is a moiety bonded to L1, R2 is a straight-chained or branched alkyl group having 1 to 30 carbon atoms, which is unsubstituted or substituted with deuterium or a monocyclic or polycyclic aryl group having 6 to 30 carbon atoms; a monocyclic or polycyclic cycloalkyl group having 3 to 30 carbon atoms, which is unsubstituted or substituted with deuterium; a straight-chained or branched alkylsilyl group having 1 to 30 carbon atoms; a monocyclic or polycyclic aryl group having 6 to 30 carbon atoms, which is unsubstituted or substituted with deuterium, a cyano group, or a straight-chained or branched alkyl group having 1 to 30 carbon atoms; or a monocyclic or polycyclic heteroaryl group having 2 to 30 carbon atoms, which is unsubstituted or substituted with deuterium.

According to an exemplary embodiment of the present specification, when R3 or R4 is a moiety bonded to L1, R2 is a straight-chained or branched alkyl group having 1 to 20 carbon atoms, which is unsubstituted or substituted with deuterium or a monocyclic or polycyclic aryl group having 6 to 20 carbon atoms; a monocyclic or polycyclic cycloalkyl group having 3 to 20 carbon atoms, which is unsubstituted or substituted with deuterium; a straight-chained or branched alkylsilyl group having 1 to 20 carbon atoms; a monocyclic or polycyclic aryl group having 6 to 20 carbon atoms, which is unsubstituted or substituted with deuterium, a cyano group, or a straight-chained or branched alkyl group having 1 to 20 carbon atoms; or a monocyclic or polycyclic heteroaryl group having 2 to 20 carbon atoms, which is unsubstituted or substituted with deuterium.

According to an exemplary embodiment of the present specification, when R3 or R4 is a moiety bonded to L1, R2 is a methyl group unsubstituted or substituted with deuterium or a monocyclic or polycyclic aryl group having 6 to 30 carbon atoms; an isopropyl group unsubstituted or substituted with deuterium; an n-butyl group unsubstituted or substituted with deuterium; a tert-butyl group unsubstituted or substituted with deuterium; a cyclopentyl group unsubstituted or substituted with deuterium; a cyclohexyl group unsubstituted or substituted with deuterium; a trimethylsilyl group unsubstituted or substituted with deuterium; a phenyl group unsubstituted or substituted with deuterium, a cyano group, or a straight-chained or branched alkyl group having 1 to 30 carbon atoms; a biphenyl group unsubstituted or substituted with deuterium; a naphthyl group unsubstituted or substituted with deuterium; a phenanthrene group unsubstituted or substituted with deuterium; a thiophene group unsubstituted or substituted with deuterium; a furan group unsubstituted or substituted with deuterium; a pyridine group unsubstituted or substituted with deuterium; a benzofuran group unsubstituted or substituted with deuterium; a benzothiophene group unsubstituted or substituted with deuterium; a carbazole group unsubstituted or substituted with deuterium; a dibenzofuran group unsubstituted or substituted with deuterium; or a dibenzothiophene group unsubstituted or substituted with deuterium.

According to an exemplary embodiment of the present specification, when R3 or R4 is a moiety bonded to L1, R2 is a methyl group unsubstituted or substituted with deuterium or a phenyl group; an isopropyl group unsubstituted or substituted with deuterium; an n-butyl group unsubstituted or substituted with deuterium; a tert-butyl group unsubstituted or substituted with deuterium; a cyclopentyl group unsubstituted or substituted with deuterium; a cyclohexyl group unsubstituted or substituted with deuterium; a trimethylsilyl group unsubstituted or substituted with deuterium; a phenyl group unsubstituted or substituted with deuterium, a cyano group, or a tert-butyl group; a biphenyl group unsubstituted or substituted with deuterium; a naphthyl group unsubstituted or substituted with deuterium; a phenanthrene group unsubstituted or substituted with deuterium; a thiophene group unsubstituted or substituted with deuterium; a furan group unsubstituted or substituted with deuterium; a pyridine group unsubstituted or substituted with deuterium; a benzofuran group unsubstituted or substituted with deuterium; a benzothiophene group unsubstituted or substituted with deuterium; a carbazole group unsubstituted or substituted with deuterium; a dibenzofuran group unsubstituted or substituted with deuterium; or a dibenzothiophene group unsubstituted or substituted with deuterium.

In the present specification, "including at least one deuterium" means that one or more hydrogens at substitutable positions are substituted with deuterium or with a substituent substituted with deuterium. The substituent includes the substituent defined in "substituted or unsubstituted" above.

The physicochemical properties related to deuterium, such as the chemical bond length, are different from those of hydrogen, the van der Waals radius of deuterium is smaller than that of hydrogen because the stretching amplitude of the C-D bond is smaller than that of the C-H bond, and in general, it can be shown that the C-D bond is shorter and stronger than the C-H bond. Therefore, when hydrogen at a substitutable position is substituted with deuterium, the energy of the ground state is reduced and the bond length between deuterium and carbon is shortened, resulting in a reduction in molecular center volume, and accordingly, the electrical polarizability may be reduced, and the thin film volume may be increased by weakening the intermolecular interaction. In addition, these characteristics may have an effect of reducing the crystallinity of the thin film, that is, create an amorphous state, and may be generally effective for increasing the service life and driving characteristics of an organic light emitting device, and the heat resistance may be improved compared to organic light emitting devices in the related art.

In the present specification, "including deuterium, "deuteration" or "deuterated" means that hydrogen at a substitutable position of a compound is substituted with deuterium.

In the present specification, "overdeuterated" means a compound or group in which all hydrogens in the molecule are substituted with deuterium, and has the same meaning as "100% deuterated".

In the present specification, "X% deuterated", "X% degree of deuteration", or "X% deuterium substitution rate" means that X% of the hydrogens at substitutable positions in the corresponding structure are substituted with deuterium. For example, when the corresponding structure is dibenzofuran, the dibenzofuran being "25% deuterated", "25% degree of deuteration" of the dibenzofuran, or "25% deuterium substitution rate" of the dibenzofuran means that 2 of the 8 hydrogens at the substitutable positions of the dibenzofuran are substituted with deuterium.

In the present specification, the "degree of deuteration" or "deuterium substitution rate" may be confirmed by a known method such as nuclear magnetic resonance spectroscopy (¹H NMR), thin-layer chromatography mass spectrometry (TLS/MS), or gas chromatography mass spectrometry (GC/MS).

Specifically, when the "degree of deuteration" or "deuterium substitution rate" is analyzed using nuclear magnetic resonance spectroscopy (¹H NMR), the degree of deuteration or deuterium substitution rate may be calculated from the integral amount of the total peak through the integration ratio on ¹H NMR by adding dimethylformamide (DMF) as an internal standard.

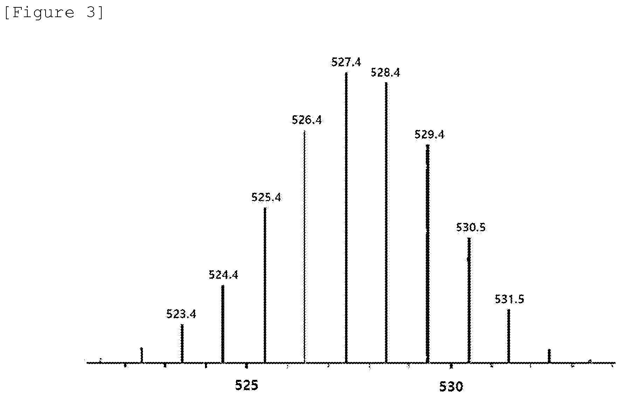

In addition, when the "degree of deuteration" or "deuterium substitution rate" is analyzed by thin-Layer chromatography/mass spectrometry (TLS/MS), the substitution rate may be calculated based on the maximum value (median value) of the distribution of molecular weights at the end of the reaction. For example, when the degree of deuteration of the following Compound A is analyzed, the molecular weight of the following starting material is set to 506 and the maximum molecular weight (median value) of the following Compound A is set to 527 in the MS graph of FIG. 3, then it may be calculated that about 81% of the hydrogen has been deuterated because 21 hydrogens of the hydrogens (26 ea) at the substitutable positions in the following starting material have been substituted with deuterium.

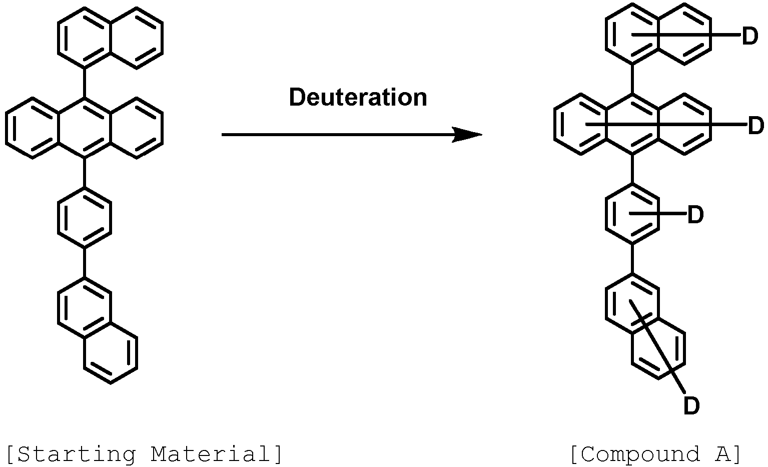

In the present specification, D means deuterium.

The compound of the invention is any one selected from the following compounds.

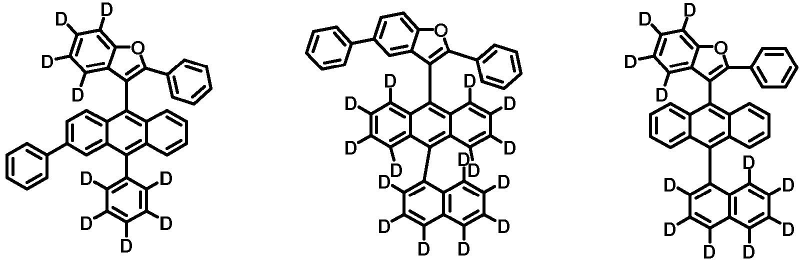

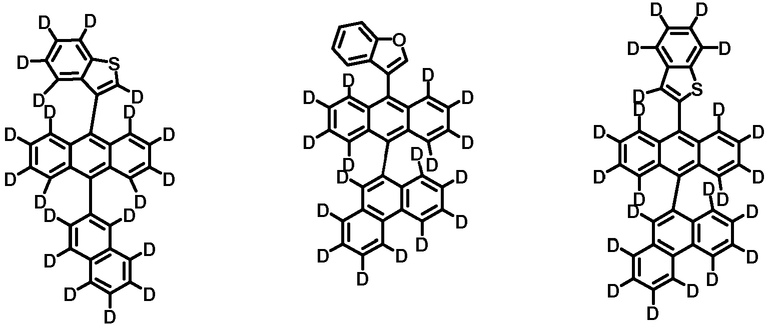

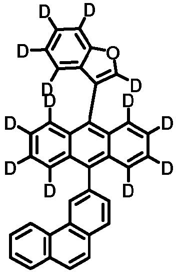

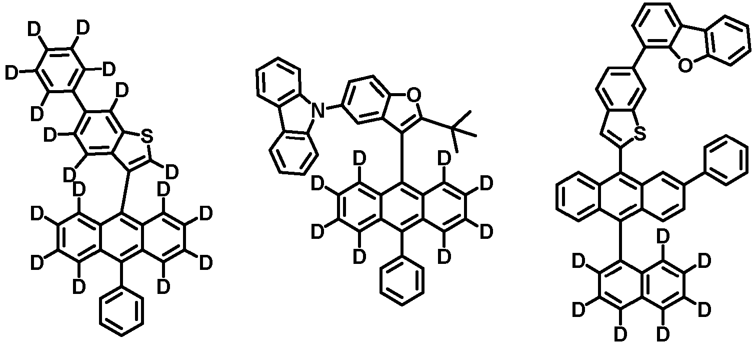

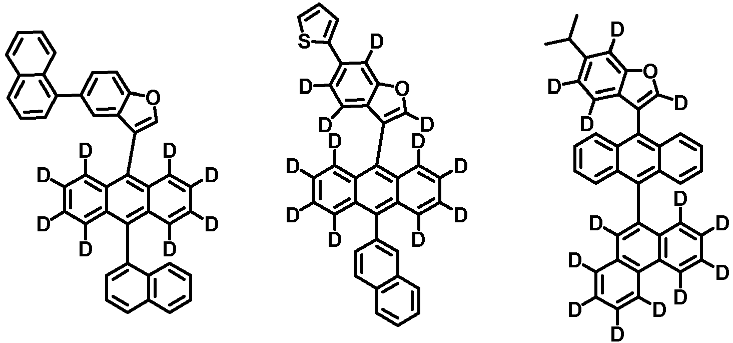

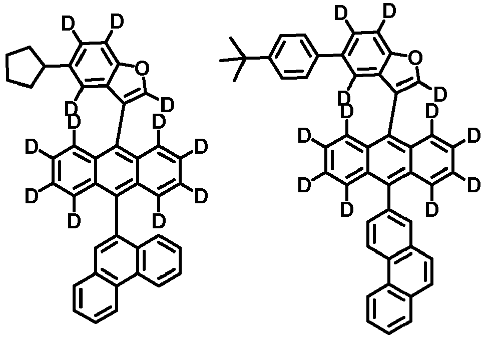

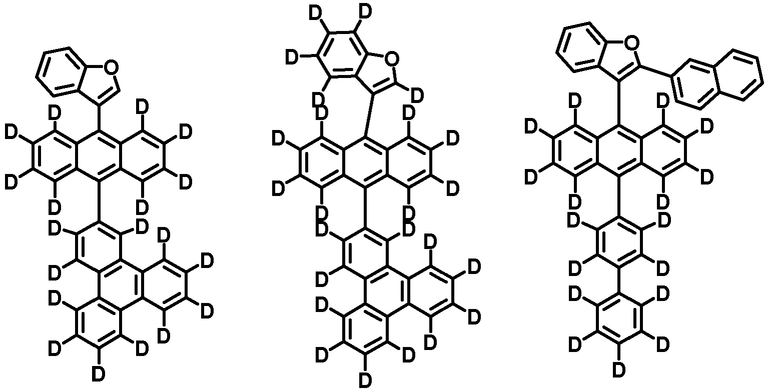

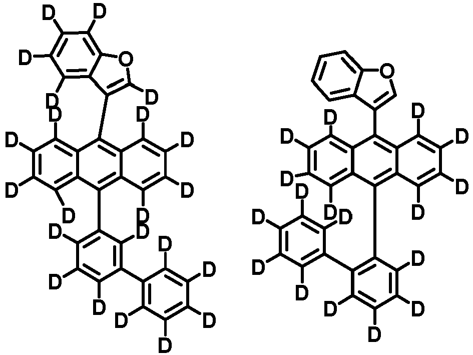

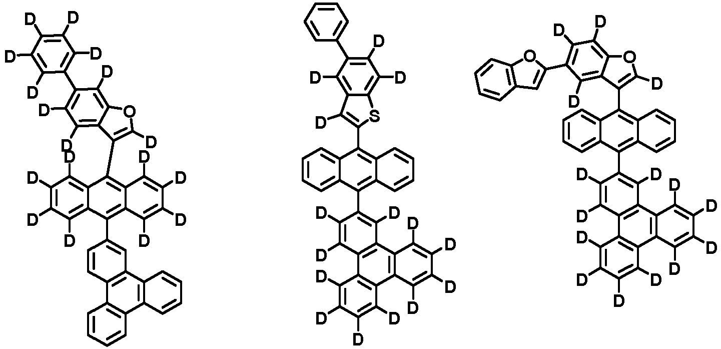

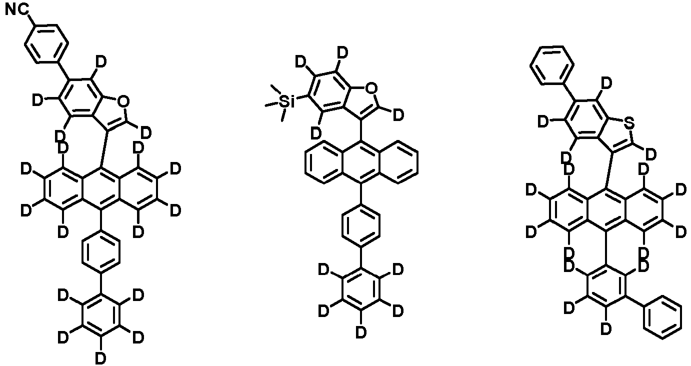

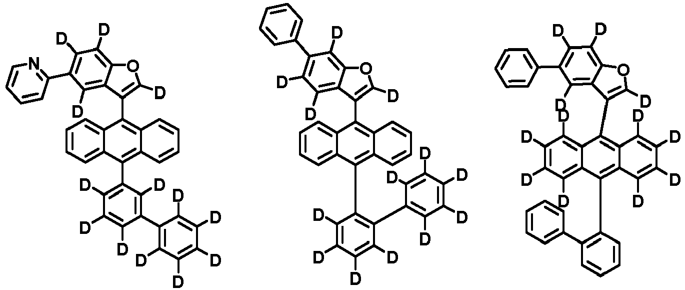

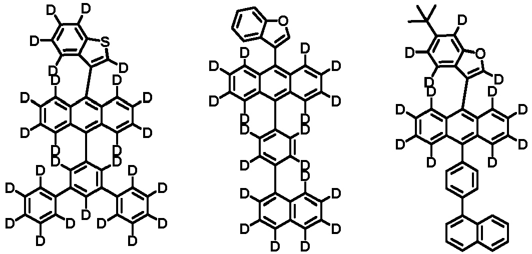

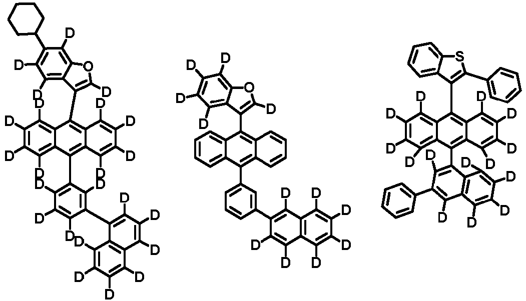

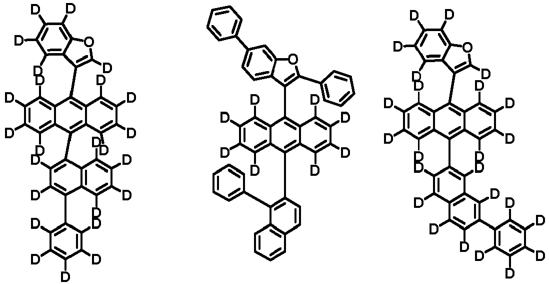

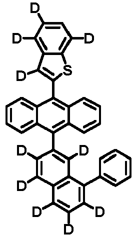

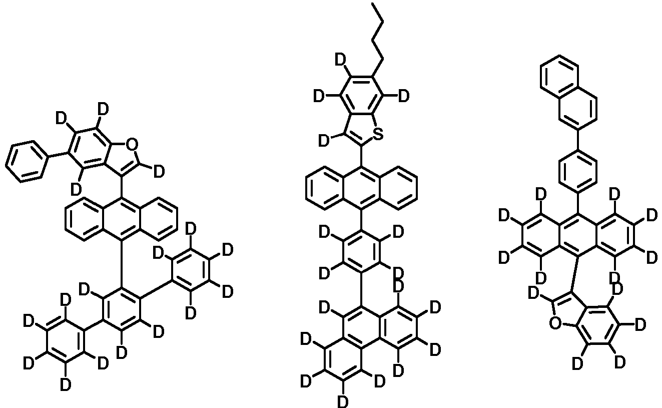

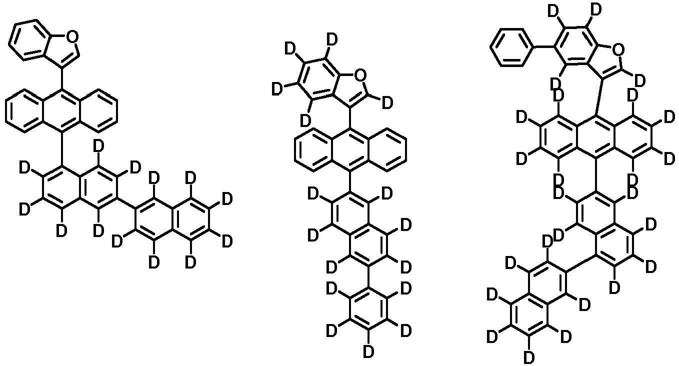

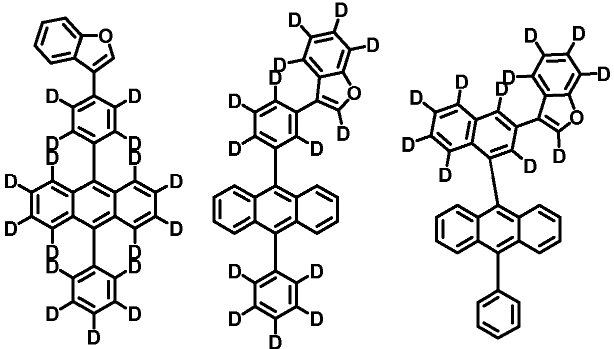

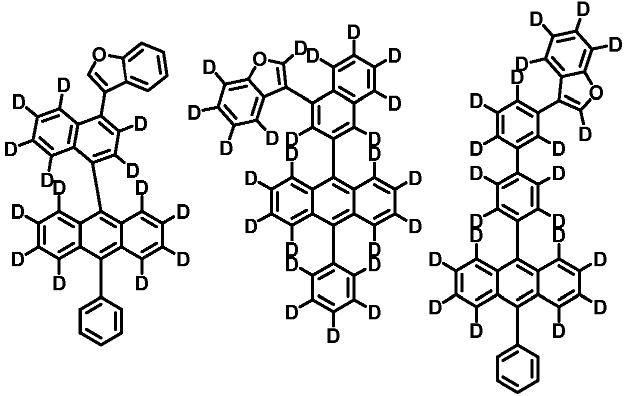

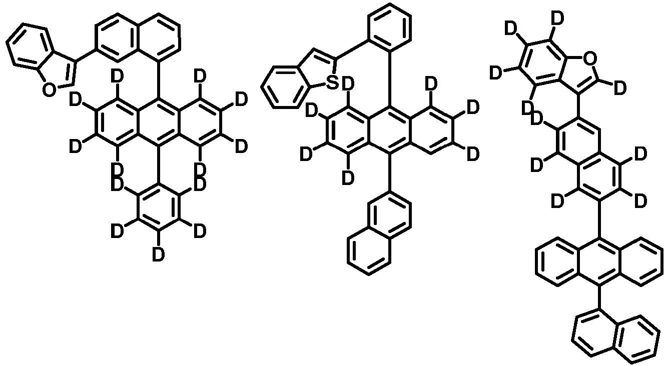

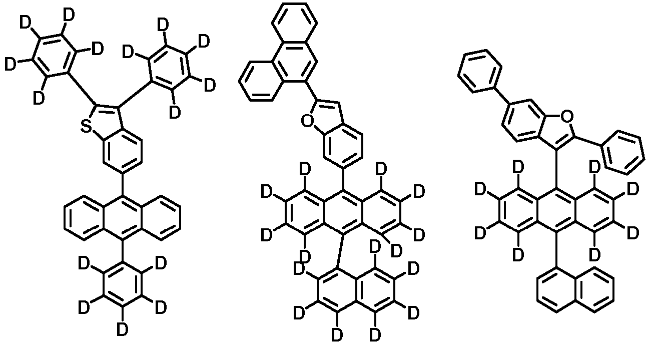

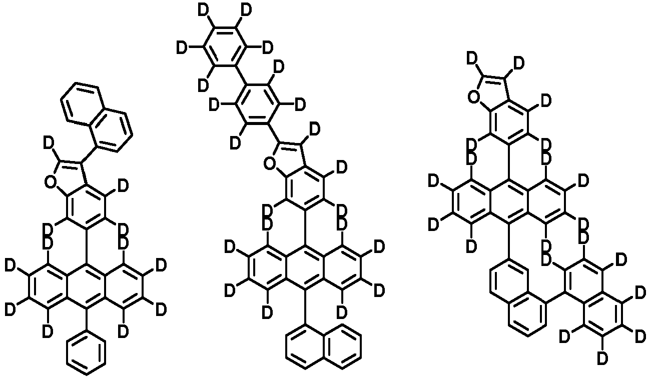

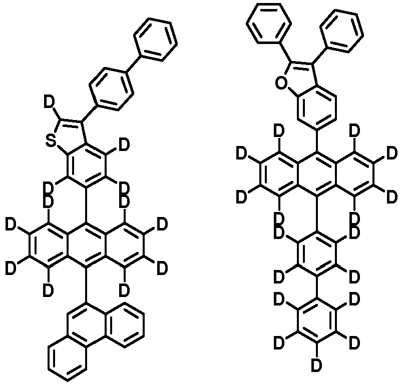

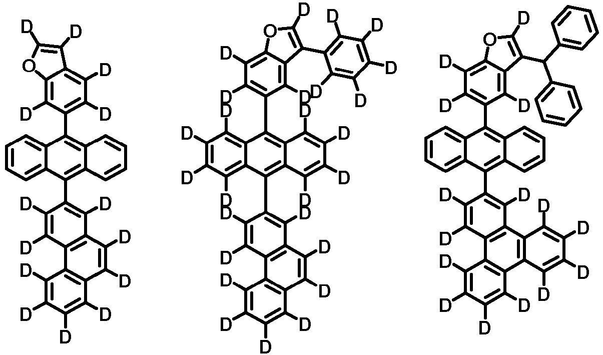

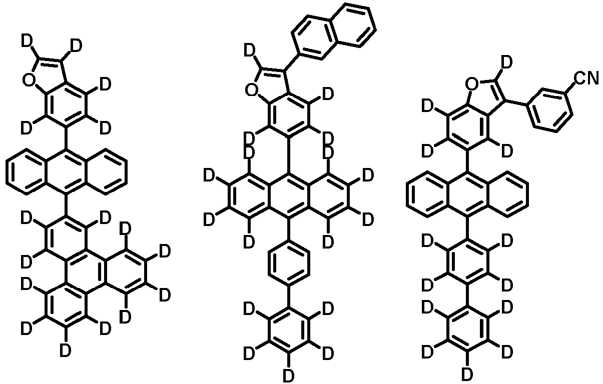

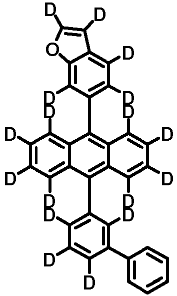

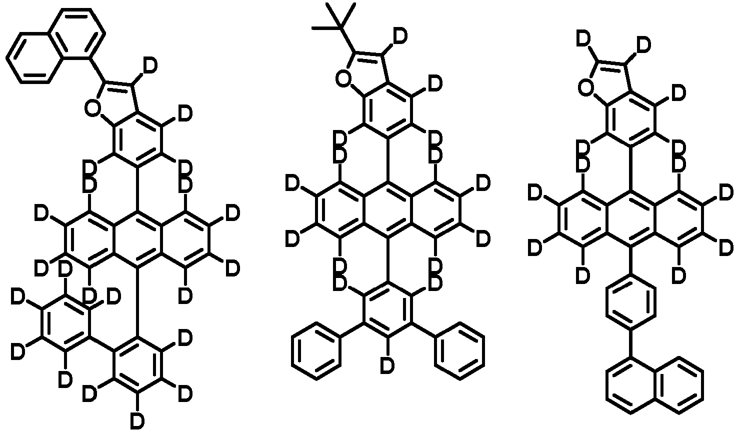

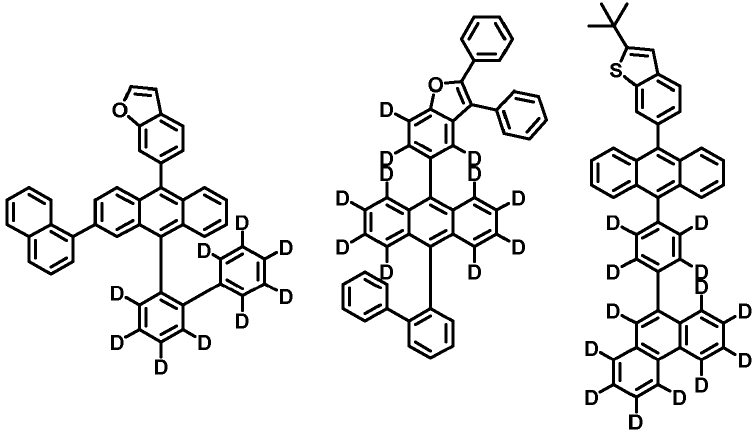

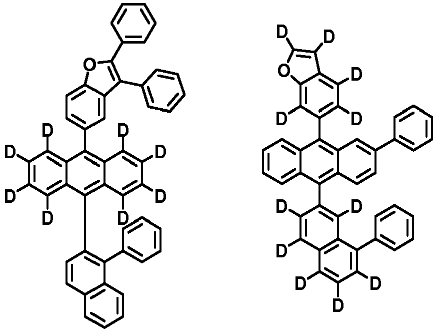

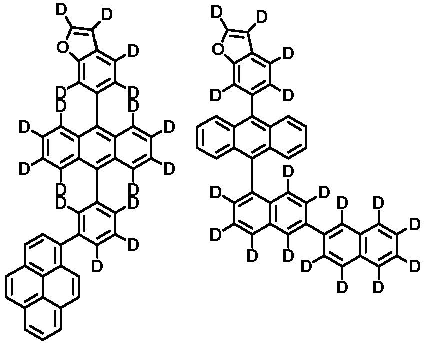

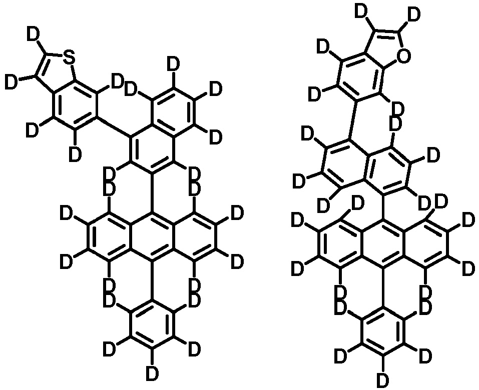

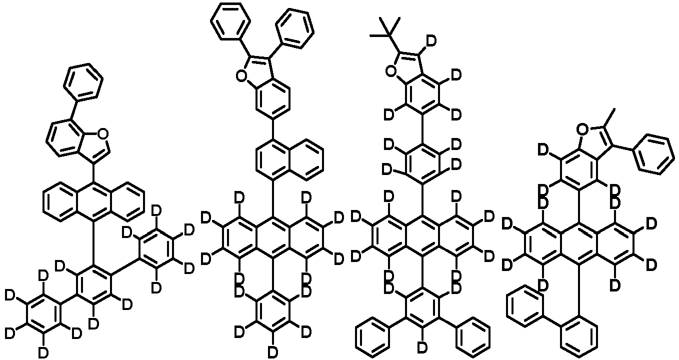

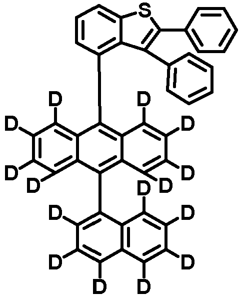

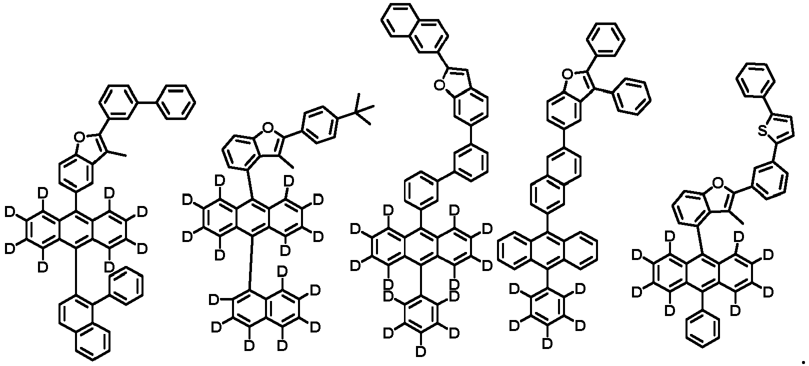

According to an exemplary embodiment of the present specification, the band gap of the compound represented by Chemical Formula 1 is 2.9 eV or more.

According to an exemplary embodiment of the present specification, the band gap of the compound represented by Chemical Formula 1 is 2.9 eV to 4.0 eV.

Specifically, the organic compound used in the organic light emitting devices may serve as an organic semiconductor only when the organic compound needs to have a band gap of 0.5 eV to 4.0 eV, and in order to exhibit blue color, the organic compound needs to have a band gap energy of at least 2.9 eV. According to the principle of fluorescent blue light emission in the host-dopant system, energy transfer occurs smoothly when the band gap of the blue fluorescent host is larger than the band gap of the blue fluorescent dopant. Therefore, it is preferred that the blue fluorescent host has an energy band gap of 2.9 eV to 4.0 eV.

In the present specification, "energy level" means a size of energy. Therefore, the energy level is interpreted to mean the absolute value of the corresponding energy value. For example, a low or deep energy level means that the absolute value increases in the negative direction from the vacuum level.

In the present specification, the highest occupied molecular orbital (HOMO) means a molecular orbital (highest occupied molecular orbital) in the highest energy region in regions in which electrons can participate in bonding, the lowest unoccupied molecular orbital (LUMO) means the molecular orbital (lowest unoccupied molecular orbital) in which electrons are present in the lowest energy region among the semi-bonded regions, and the HOMO energy level means the distance from the vacuum level to the HOMO. Furthermore, the LUMO energy level means the distance from the vacuum level to the LUMO.

In the present specification, the HOMO energy level may be measured using a photoelectron spectrometer under the atmosphere (manufactured by RIKEN KEIKI Co., Ltd.: AC3), and the LUMO energy level may be calculated from wavelength values measured through photoluminescence (PL).

In the present specification, a band gap means the difference between the HOMO energy level and the LUMO energy level.

The present specification provides an organic light emitting device as defined above.

When one member is disposed "on" another member in the present specification, this includes not only a case where the one member is brought into contact with another member, but also a case where still another member is present between the two members.

When one part "includes" one constituent element in the present specification, unless otherwise specifically described, this does not mean that another constituent element is excluded, but means that another constituent element may be further included.

In the present specification, the 'layer' has a meaning compatible with a 'film' usually used in the art, and means a coating covering a target region. The size of the 'layer' is not limited, and the sizes of the respective 'layers' may be the same as or different from one another. According to an exemplary embodiment, the size of the 'layer' may be the same as that of the entire device, may correspond to the size of a specific functional region, and may also be as small as a single sub-pixel.

In the present specification, when a specific A material is included in a B layer, this means both i) the fact that one or more A materials are included in one B layer and ii) the fact that the B layer is composed of one or more layers, and the A material is included in one or more layers of the multi-layered B layer.

In the present specification, when a specific A material is included in a C layer or a D layer, this means all of i) the fact that the A material is included in one or more layers of the C layer having one or more layers, ii) the fact that the A material is included in one or more layers of the D layer having one or more layers, and iii) the fact that the A material is included in each of the C layer having one or more layers and the D layer having one or more layers.

The present specification provides an organic light emitting device including: a first electrode; a second electrode provided to face the first electrode; and an organic material layer having one or more layers provided between the first electrode and the second electrode, in which one or more layers of the organic material layer include the compound represented by Chemical Formula 1.

For example, the organic material layer may have a structure including a hole injection layer, a hole transport layer, a light emitting layer, an electron transport layer, an electron injection layer, an electron blocking layer, a hole blocking layer, and the like. However, the structure of the organic light emitting device is not limited thereto, and may include a fewer number of organic layers.

The organic material layer includes a light emitting layer, and the light emitting layer includes the compound as a host of the light emitting layer.

According to an exemplary embodiment of the present specification, the light emitting layer includes a dopant, and the dopant includes a fluorescent dopant.

According to an exemplary embodiment of the present specification, the fluorescent dopant is a pyrene-based compound or a non-pyrene-based compound.

According to an exemplary embodiment of the present specification, the non-pyrene-based compound includes a boron-based compound.

According to an exemplary embodiment of the present specification, the light emitting layer further includes one or more hosts different from the compound of Chemical Formula 1.

Any anthracene-based host used in the art can be used without limitation as long as the host different from the compound of Chemical Formula 1 is different from Chemical Formula 1, and the host is not limited thereto.

According to an exemplary embodiment of the present specification, the light emitting layer includes a host and a dopant.

According to an exemplary embodiment of the present specification, the light emitting layer includes a host and a dopant, and the host includes the compound represented by Chemical Formula 1.

According to an exemplary embodiment of the present specification, the dopant is a blue dopant.

According to an exemplary embodiment of the present specification, the organic light emitting device is a blue organic light emitting device.

According to an exemplary embodiment of the present specification, the light emitting layer includes two or more mixed hosts, and one or more of the two or more mixed hosts include the compound represented by Chemical Formula 1.

According to an exemplary embodiment of the present specification, the light emitting layer includes two or more mixed hosts, at least one of the two or more mixed hosts includes the compound represented by Chemical Formula 1, and the others include an anthracene-based compound different from Chemical Formula 1.

At least one of the two or more mixed hosts includes the compound represented by Chemical Formula 1, and an anthracene-based host used in the art can be used without limitation as long as the others are different from Chemical Formula 1, and the mixed hosts are not limited thereto.

An organic light emitting device using two or more mixed hosts according to an exemplary embodiment of the present specification is intended to improve the performance of the device by combining the advantages of each host, and for example, when two types of hosts are mixed, one type of host with high efficiency and low voltage effects and one type of host with long service life effects may be mixed to manufacture an organic light emitting device having the effects of high efficiency, low voltage and long service life.

According to an exemplary embodiment of the present specification, the light emitting layer has two or more layers, and one or more of the two or more light emitting layers include the compound of Chemical Formula 1.

According to an exemplary embodiment of the present specification, the light emitting layer has two or more layers, one or more layers of the light emitting layers having two or more layers includes two or more types of mixed hosts, and one or more of the two or more types of mixed hosts include the compound of Chemical Formula 1.

According to an exemplary embodiment of the present specification, the light emitting layer has two layers, and one layer of the light emitting layer having two layers includes the compound of Chemical Formula 1.

According to an exemplary embodiment of the present specification, the light emitting layer has two layers, one layer of the light emitting layer having two layers includes two or more types of mixed hosts, and one or more of the two or more types of mixed hosts include the compound of Chemical Formula 1.

The two or more types of mixed hosts are as described above.

According to an exemplary embodiment of the present specification, the organic light emitting device has a maximum emission wavelength (λₘₐₓ) of 400 nm to 470 nm in an emission spectrum.

According to an exemplary embodiment of the present specification, the light emitting layer includes a host and a dopant, and the dopant is a fluorescent dopant.

According to an exemplary embodiment of the present specification, the light emitting layer includes a host and a dopant, and the dopant includes one or more selected from a pyrene-based compound and a non-pyrene-based compound.

The pyrene-based compound and the non-pyrene-based compounds can be used without limitation as long as they are compounds used in the art, and are not limited thereto.

According to an exemplary embodiment of the present specification, the non-pyrene-based compound includes a boron-based compound.

According to an exemplary embodiment of the present specification, the light emitting layer includes a host and a dopant, the host includes the compound represented by Chemical Formula 1, and the dopant includes one or more selected from a pyrene-based compound and a non-pyrene-based compound.

According to an exemplary embodiment of the present specification, the light emitting layer includes the host and the dopant at a weight ratio of 0.1:99.9 to 20:80.

According to another exemplary embodiment of the present specification, the light emitting layer further includes a capping layer provided on a surface of at least one of the first electrode and the second electrode opposite to the surface facing the organic material layer.

The capping layer is formed to prevent a considerable amount of light from being lost through total reflection of light in the organic light emitting device, and the capping layer has the performance capable of sufficiently protecting the underlying negative electrode and light emitting layer from external moisture penetration or contamination, and has a high refractive index, and thus may prevent light loss caused by total reflection.

According to still another exemplary embodiment of the present specification, the capping layer may be provided on each of a surface of the first electrode opposite to the surface facing the organic layer and a surface of the second electrode opposite to the surface facing the organic layer.

According to yet another exemplary embodiment of the present specification, the capping layer may be provided on a surface of the first electrode opposite to the surface facing the organic layer.

According to still yet another exemplary embodiment of the present specification, the capping layer may be provided on a surface of the second electrode opposite to the surface facing the organic layer.

According to an exemplary embodiment of the present specification, the organic light emitting device further includes one or two or more layers selected from the group consisting of a hole injection layer, a hole transport layer, a light emitting layer, an electron transport layer, an electron injection layer, a hole blocking layer, and an electron blocking layer.

According to an exemplary embodiment of the present specification, the organic light emitting device includes: a first electrode; a second electrode provided to face the first electrode; a light emitting layer provided between the first electrode and the second electrode; and an organic material layer having two or more layers provided between the light emitting layer and the first electrode, or between the light emitting layer and the second electrode.

According to an exemplary embodiment of the present specification, as the organic material layer having two or more layers between the light emitting layer and the first electrode, or between the light emitting layer and the second electrode, two or more may be selected from the group consisting of a light emitting layer, a hole transport layer, a hole injection layer, a hole injection and transport layer, an electron blocking layer, a hole blocking layer, an electron injection layer, an electron transport layer, and an electron injection and transport layer.

According to an exemplary embodiment of the present specification, a hole transport layer having two or more layers is included between the light emitting layer and the first electrode. The hole transport layer having two or more layers may include materials which are the same as or different from each other.

According to an exemplary embodiment of the present specification, the first electrode is an anode or a cathode.

According to an exemplary embodiment of the present specification, the second electrode is a cathode or an anode.

According to an exemplary embodiment of the present specification, the organic light emitting device may be a normal type organic light emitting device in which an anode, an organic material layer having one or more layers, and a cathode are sequentially stacked on a substrate.

According to an exemplary embodiment of the present specification, the organic light emitting device may be an inverted type organic light emitting device in which a cathode, an organic material layer having one or more layers, and an anode are sequentially stacked on a substrate.

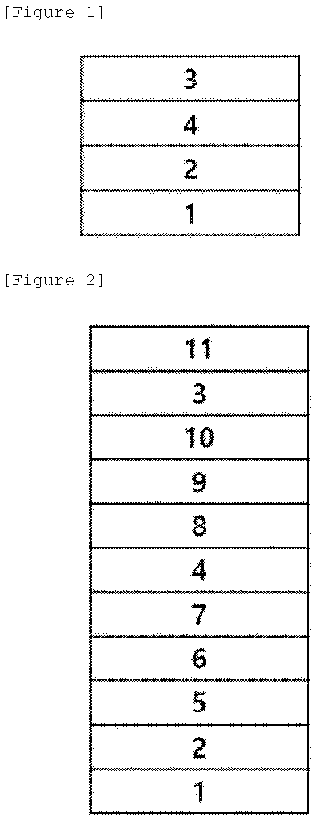

For example, the structure of the organic light emitting device according to an exemplary embodiment of the present specification is exemplified in FIGS. 1 and 2. FIGS. 1 and 2 exemplify an organic light emitting device, and the organic light emitting device is not limited thereto.

FIG. 1 exemplifies a structure of an organic light emitting device in which a first electrode 2, a light emitting layer 4, and a second electrode 3 are sequentially stacked on a substrate 1. The compound is included in the light emitting layer.

FIG. 2 exemplifies the structure of an organic light emitting device in which a first electrode 2, a hole injection layer 5, a hole transport layer 6, a hole adjusting layer 7, a light emitting layer 4, an electron adjusting layer 8, an electron transport layer 9, an electron injection layer 10, a second electrode 3, and a capping layer 11 are sequentially stacked on a substrate 1. The compound is included in the light emitting layer.

The organic light emitting device of the present specification may be manufactured by the materials and methods known in the art, except that the light emitting layer includes the compound, that is, the compound of Chemical Formula 1.

When the organic light emitting device includes a plurality of organic material layers, the organic material layers may be formed of the same material or different materials.

For example, the organic light emitting device of the present specification may be manufactured by sequentially stacking a first electrode, an organic material layer, and a second electrode on a substrate. In this case, the organic light emitting device may be manufactured by depositing a metal or a metal oxide having conductivity, or an alloy thereof on a substrate to form an anode, forming an organic material layer including a hole injection layer, a hole transport layer, a light emitting layer, and an electron transport layer thereon, and then depositing a material, which may be used as a cathode, thereon, by using a physical vapor deposition (PVD) method such as sputtering or e-beam evaporation. In addition to the method described above, an organic light emitting device may be manufactured by sequentially depositing a second electrode material, an organic material layer, and a first electrode material on a substrate.

Further, the compound represented by Chemical Formula 1 may be formed as an organic material layer by not only a vacuum deposition method, but also a solution application method when an organic light emitting device is manufactured. Here, the solution application method means spin coating, dip coating, doctor blading, inkjet printing, screen printing, a spray method, roll coating, and the like, but is not limited thereto.

In addition to the method described above, an organic light emitting device may be made by sequentially depositing a second electrode material, an organic material layer, and a first electrode material on a substrate. However, the manufacturing method is not limited thereto.

As the first electrode material, materials having a high work function are usually preferred so as to facilitate the injection of holes into an organic material layer. Examples thereof include: a metal, such as vanadium, chromium, copper, zinc, and gold, or an alloy thereof; a metal oxide, such as zinc oxide, indium oxide, indium tin oxide (ITO), and indium zinc oxide (IZO); a combination of a metal and an oxide, such as ZnO:Al or SnO₂:Sb; a conductive polymer, such as poly(3-methylthiophene), poly[3,4-(ethylene-1,2-dioxy)thiophene] (PEDOT), polypyrrole, and polyaniline; and the like, but are not limited thereto.

As the second electrode material, materials having a low work function are usually preferred so as to facilitate the injection of electrons into an organic material layer. Examples thereof include: a metal, such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

The light emitting layer may include a host material and a dopant material. When an additional light emitting layer is included in addition to a light emitting layer including the compound of Chemical Formula 1 according to an exemplary embodiment of the present specification, examples of a host material include a fused and/or non-fused aromatic ring derivative, a hetero ring-containing compound, or the like. Specific examples of the fused aromatic ring derivative include anthracene derivatives, pyrene derivatives, naphthalene derivatives, pentacene derivatives, phenanthrene compounds, fluoranthene compounds, and the like, and specific examples of the hetero ring-containing compound include dibenzofuran derivatives, ladder-type furan compounds, pyrimidine derivatives, and the like, but the examples are not limited thereto.

According to an exemplary embodiment of the present specification, the host includes a compound represented by the following Chemical Formula H-1, but is not limited thereto. 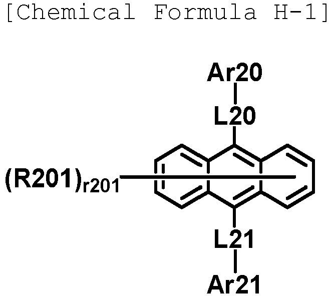 In Chemical Formula H-1,
L20 and L21 are the same as or different from each other, and are each independently a direct bond; a substituted or unsubstituted arylene group; or a substituted or unsubstituted divalent heterocyclic group,
Ar20 and Ar21 are the same as or different from each other, and are each independently hydrogen; deuterium; a substituted or unsubstituted aryl group; or a substituted or unsubstituted heterocyclic group,
R201 is hydrogen; deuterium; a halogen group; a substituted or unsubstituted alkyl group; a substituted or unsubstituted cycloalkyl group; a substituted or unsubstituted aryl group; or a substituted or unsubstituted heterocyclic group, and
r201 is an integer from 1 to 8, and when r201 is 2 or greater, two or more R201's are the same as or different from each other.

In an exemplary embodiment of the present specification, L20 and L21 are the same as or different from each other, and are each independently a direct bond; a monocyclic or polycyclic arylene group having 6 to 30 carbon atoms; or a monocyclic or polycyclic divalent heterocyclic group having 2 to 30 carbon atoms.

In an exemplary embodiment of the present specification, L20 and L21 are the same as or different from each other, and are each independently a direct bond; a phenylene group which is unsubstituted or substituted with deuterium; a biphenylylene group which is unsubstituted or substituted with deuterium; a naphthylene group which is unsubstituted or substituted with deuterium; a divalent dibenzofuran group; or a divalent dibenzothiophene group.

In an exemplary embodiment of the present specification, Ar20 and Ar21 are the same as or different from each other, and are each independently a substituted or unsubstituted monocyclic or polycyclic aryl group having 6 to 30 carbon atoms; or a substituted or unsubstituted monocyclic or polycyclic heterocyclic group having 2 to 30 carbon atoms.

In an exemplary embodiment of the present specification, Ar20 and Ar21 are the same as or different from each other, and are each independently a substituted or unsubstituted monocyclic to tetracyclic aryl group having 6 to 20 carbon atoms; or a substituted or unsubstituted monocyclic to tetracyclic heterocyclic group having 6 to 20 carbon atoms.

In an exemplary embodiment of the present specification, Ar20 and Ar21 are the same as or different from each other, and are each independently a phenyl group which is unsubstituted or substituted with deuterium or a monocyclic or polycyclic aryl group having 6 to 20 carbon atoms; a biphenyl group which is unsubstituted or substituted with deuterium or a monocyclic or polycyclic aryl group having 6 to 20 carbon atoms; a naphthyl group which is unsubstituted or substituted with a monocyclic or polycyclic aryl group having 6 to 20 carbon atoms; a thiophene group which is unsubstituted or substituted with a monocyclic or polycyclic aryl group having 6 to 30 carbon atoms; a dibenzofuran group which is unsubstituted or substituted with a monocyclic or polycyclic aryl group having 6 to 20 carbon atoms; a naphthobenzofuran group which is unsubstituted or substituted with a monocyclic or polycyclic aryl group having 6 to 20 carbon atoms; a dibenzothiophene group which is unsubstituted or substituted with a monocyclic or polycyclic aryl group having 6 to 20 carbon atoms; or a naphthobenzothiophene group which is unsubstituted or substituted with a monocyclic or polycyclic aryl group having 6 to 20 carbon atoms.

In an exemplary embodiment of the present specification, Ar20 and Ar21 are the same as or different from each other, and are each independently a phenyl group which is unsubstituted or substituted with deuterium; a biphenyl group which is unsubstituted or substituted with deuterium; a terphenyl group; a naphthyl group which is unsubstituted or substituted with deuterium; a thiophene group which is unsubstituted or substituted with a phenyl group; a phenanthrene group; a dibenzofuran group; a naphthobenzofuran group; a dibenzothiophene group; or a naphthobenzothiophene group.

In an exemplary embodiment of the present specification, Ar20 and Ar21 are the same as or different from each other, and each independently a substituted or unsubstituted aryl group.

In an exemplary embodiment of the present specification, Ar20 and Ar21 are the same as or different from each other, and are each independently a phenyl group which is unsubstituted or substituted with deuterium; a biphenyl group which is unsubstituted or substituted with deuterium; a terphenyl group; or a naphthyl group which is unsubstituted or substituted with deuterium.

According to an exemplary embodiment of the present specification, R201 is hydrogen.

According to an exemplary embodiment of the present specification, Chemical Formula H-1 is represented by the following compound.

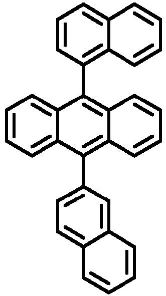

Examples of the dopant material include an aromatic amine derivative, a styrylamine compound, a boron complex, a fluoranthene compound, a metal complex, and the like. Specifically, the aromatic amine derivative is a fused aromatic ring derivative having a substituted or unsubstituted arylamine group, and examples thereof include pyrene, anthracene, chrysene, periflanthene, and the like having an arylamine group. Further, the styrylamine compound is a compound in which a substituted or unsubstituted arylamine is substituted with at least one arylvinyl group, and one or two or more substituents selected from the group consisting of an aryl group, a silyl group, an alkyl group, a cycloalkyl group, and an arylamine group are substituted or unsubstituted. Specific examples thereof include styrylamine, styryldiamine, styryltriamine, styryltetramine, and the like, but are not limited thereto. Further, examples of the metal complex include an iridium complex, a platinum complex, and the like, but are not limited thereto.

According to an exemplary embodiment of the present specification, the dopant material includes a compound of the following Chemical Formula D-1 or D-2, but is not limited thereto. 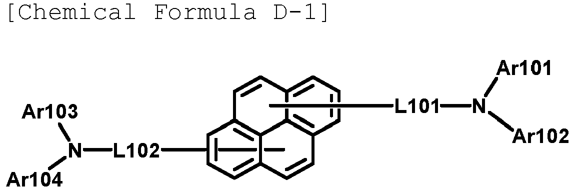 In Chemical Formula D-1,
L101 and L102 are the same as or different from each other, and are each independently a direct bond; or a substituted or unsubstituted arylene group, and
Ar101 to Ar104 are the same as or different from each other, and are each independently a substituted or unsubstituted aryl group; or a substituted or unsubstituted heteroaryl group, 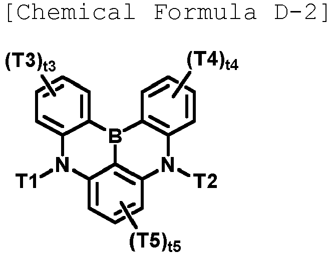
In Chemical Formula D-2,
T1 to T5 are the same as or different from each other, and are each independently hydrogen; a substituted or unsubstituted alkyl group; a substituted or unsubstituted amine group; or a substituted or unsubstituted aryl group,
t3 and t4 are each an integer from 1 to 4,
t5 is an integer from 1 to 3,
when t3 is 2 or greater, two or more T3's are the same as or different from each other,
when t4 is 2 or greater, two or more T4's are the same as or different from each other, and
when t5 is 2 or greater, two or more T5's are the same as or different from each other.

According to an exemplary embodiment of the present specification, L101 and L102 are a direct bond.

According to an exemplary embodiment of the present specification, Ar101 to Ar104 are the same as or different from each other, and are each independently a substituted or unsubstituted monocyclic or polycyclic aryl group having 6 to 30 carbon atoms; or a substituted or unsubstituted monocyclic or polycyclic heteroaryl group having 2 to 30 carbon atoms.

According to an exemplary embodiment of the present specification, Ar101 to Ar104 are the same as or different from each other, and are each independently a monocyclic or polycyclic aryl group having 6 to 30 carbon atoms, which is unsubstituted or substituted with a straight-chained or branched alkyl group having 1 to 30 carbon atoms; or a monocyclic or polycyclic heteroaryl group having 2 to 30 carbon atoms.

According to an exemplary embodiment of the present specification, Ar101 to Ar104 are the same as or different from each other, and are each independently a phenyl group substituted with a methyl group; or a dibenzofuran group. According to an exemplary embodiment of the present specification, Chemical Formula D-1 is represented by the following compound.

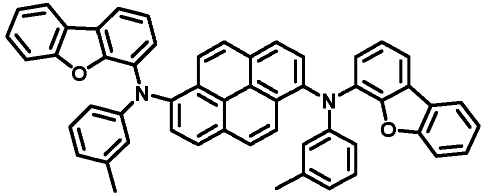

According to an exemplary embodiment of the present specification, T1 to T5 are the same as or different from each other, and are each independently hydrogen; a substituted or unsubstituted straight-chained or branched alkyl group having 1 to 30 carbon atoms; a substituted or unsubstituted monocyclic or polycyclic arylamine group having 6 to 30 carbon atoms; or a substituted or unsubstituted monocyclic or polycyclic aryl group having 6 to 30 carbon atoms.

According to an exemplary embodiment of the present specification, T1 to T5 are the same as or different from each other, and are each independently hydrogen; a straight-chained or branched alkyl group having 1 to 30 carbon atoms; a monocyclic or polycyclic arylamine group having 6 to 30 carbon atoms; or a monocyclic or polycyclic aryl group having 6 to 30 carbon atoms, which is unsubstituted or substituted with a straight-chained or branched alkyl group having 1 to 30 carbon atoms.

According to an exemplary embodiment of the present specification, T1 to T5 are the same as or different from each other, and are each independently hydrogen; a methyl group; a tert-butyl group; a diphenylamine group; or a phenyl group which is unsubstituted or substituted with a methyl group or tert-butyl group.

According to an exemplary embodiment of the present specification, Chemical Formula D-2 is represented by the following compound.

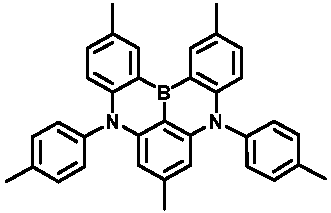

The hole injection layer is a layer which accepts holes from an electrode. It is preferred that hole injection material has an ability to transport holes, and has an effect of accepting holes from an anode and an excellent hole injection effect for a light emitting layer or a light emitting material. Further, the hole injection material is preferably a material which is excellent in ability to prevent excitons produced from a light emitting layer from moving to an electron injection layer or an electron injection material. In addition, the hole injection material is preferably a material which is excellent in ability to form a thin film. In addition, the highest occupied molecular orbital (HOMO) of the hole injection material is preferably a value between the work function of the anode material and the HOMO of the neighboring organic material layer. Specific examples of the hole injection material include: metal porphyrin, oligothiophene, and arylamine-based organic materials; hexanitrile hexaazatriphenylene-based organic materials; quinacridone-based organic materials; perylene-based organic materials; polythiophene-based conductive polymers such as anthraquinone and polyaniline; and the like, but are not limited thereto.

According to an exemplary embodiment of the present specification, the hole injection layer includes a compound of the following Chemical Formula HI-1, but is not limited thereto. 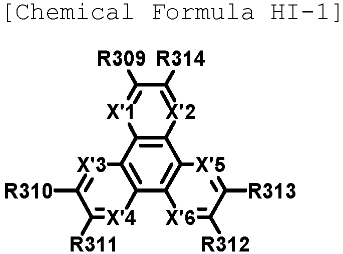 In Chemical Formula HI-1,
at least one of X'1 to X'6 is N, and the others are CH, and
R309 to R314 are the same as or different from each other, and are each independently hydrogen; deuterium; a cyano group; a substituted or unsubstituted alkyl group; a substituted or unsubstituted amine group; a substituted or unsubstituted aryl group; or a substituted or unsubstituted heteroaryl group, or are bonded to an adjacent group to form a substituted or unsubstituted ring.

According to an exemplary embodiment of the present specification, X'1 to X'6 are N.

According to an exemplary embodiment of the present specification, R309 to R314 are a cyano group.

According to an exemplary embodiment of the present specification, Chemical Formula HI-1 is represented by the following compound.

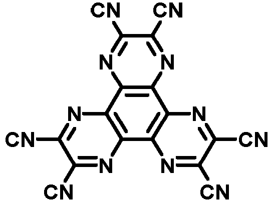

The hole transport layer is a layer which accepts holes from a hole injection layer and transports the holes to a light emitting layer. A hole transport material is preferably a material having high hole mobility which may accept holes from an anode or a hole injection layer and transfer the holes to a light emitting layer. Specific examples thereof include arylamine-based organic materials, conductive polymers, block copolymers having both conjugated portions and non-conjugated portions, and the like, but are not limited thereto.

The hole adjusting layer is a layer which may improve the service life and efficiency of a device by adjusting holes transported from a hole transport layer such that the holes are smoothly injected into a light emitting layer, and preventing electrons injected from an electron injection layer from passing through a light emitting layer and entering a hole injection layer. As the electron blocking layer, the publicly-known material can be used without limitation, and the electron blocking layer may be formed between a light emitting layer and a hole injection layer, between a light emitting layer and a hole transport layer, or between a light emitting layer and a layer which simultaneously injects and transports holes.

According to an exemplary embodiment of the present specification, the hole transport layer or hole adjusting layer includes a compound of following Chemical Formula HT-1, but is not limited thereto. 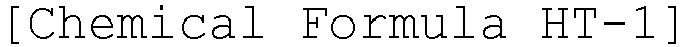 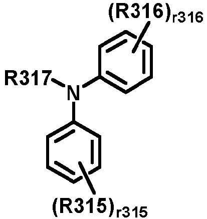 In Chemical Formula HT-1,
R315 to R317 are the same as or different from each other, and are each independently any one selected from the group consisting of hydrogen; deuterium; a substituted or unsubstituted alkyl group; a substituted or unsubstituted aryl group; a substituted or unsubstituted heteroaryl group; and a combination thereof, or are bonded to an adjacent group to form a substituted or unsubstituted ring,
r315 is an integer from 1 to 5, and when r315 is 2 or higher, two or more R315's are the same as or different from each other, and
r316 is an integer from 1 to 5, and when r316 is 2 or higher, two or more R316's are the same as or different from each other.

According to an exemplary embodiment of the present specification, R317 is any one selected from the group consisting of a substituted or unsubstituted aryl group; a substituted or unsubstituted heteroaryl group; and a combination thereof.

According to an exemplary embodiment of the present specification, R317 is any one selected from the group consisting of a carbazole group; a phenyl group; a biphenyl group; and a combination thereof.

According to an exemplary embodiment of the present specification, R315 and R316 are the same as or different from each other, and are each independently a substituted or unsubstituted aryl group, or are bonded to an adjacent group to form an aromatic hydrocarbon ring substituted with an alkyl group.

According to an exemplary embodiment of the present specification, R315 and R316 are the same as or different from each other, and are each independently a phenyl group or a phenanthrene group, or are bonded to an adjacent group to form indene substituted with a methyl group.

According to an exemplary embodiment of the present specification, Chemical Formula HT-1 is represented by any one of the following compounds.

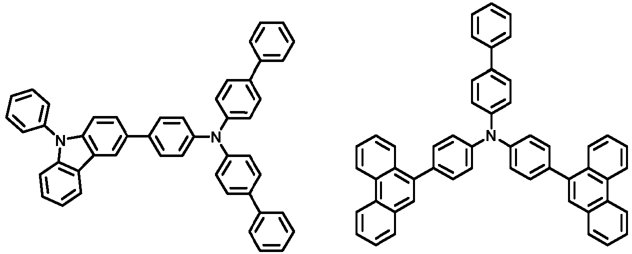

The electron adjusting layer is a layer that adjusts electrons transferred from the electron transport layer to be smoothly injected into the light emitting layer, and known materials can be used without limitation.

According to an exemplary embodiment of the present specification, the electron adjusting layer includes a compound of the following Chemical Formula EG-1, but is not limited thereto. 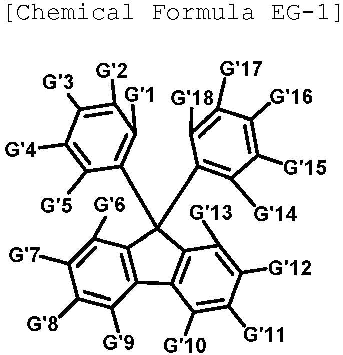 In Chemical Formula EG-1,
at least one of G'1 to G'18 is -L5-Ar5, the others are hydrogen, or G'1 and G'18 are linked through -L51- to form a substituted or unsubstituted ring,
L5 is a direct bond; or a substituted or unsubstituted arylene group,
Ar5 is a substituted or unsubstituted aryl group; or a substituted or unsubstituted heteroaryl group, and L51 is O; or S.

According to an exemplary embodiment of the present specification, L51 is O.

According to an exemplary embodiment of the present specification, L51 is S.

According to an exemplary embodiment of the present specification, G'1 and G'18 are linked through -L51- to form a substituted or unsubstituted hetero ring.

According to an exemplary embodiment of the present specification, G'1 and G'18 are linked through -L51- to form a substituted or unsubstituted xanthene ring; or a substituted or unsubstituted thioxanthene ring.

According to an exemplary embodiment of the present specification, G'1 and G'18 are linked through -O- to form a substituted or unsubstituted xanthene ring.

According to an exemplary embodiment of the present specification, G'1 and G'18 are linked through -S- to form a substituted or unsubstituted thioxanthene ring.

According to an exemplary embodiment of the present specification, G'1 and G'18 are linked through -O- to form a xanthene ring.

According to an exemplary embodiment of the present specification, G'1 and G'18 are linked through -S- to form a thioxanthene ring.

According to an exemplary embodiment of the present specification, L5 is a direct bond; or a substituted or unsubstituted monocyclic or polycyclic arylene group having 6 to 30 carbon atoms.

According to an exemplary embodiment of the present specification, L5 is a direct bond; or a substituted or unsubstituted monocyclic or polycyclic arylene group having 6 to 20 carbon atoms.

According to an exemplary embodiment of the present specification, L5 is a direct bond; or a monocyclic or polycyclic arylene group having 6 to 30 carbon atoms.

According to an exemplary embodiment of the present specification, L5 is a direct bond; or a monocyclic or polycyclic arylene group having 6 to 20 carbon atoms.

According to an exemplary embodiment of the present specification, L5 is a direct bond; or a phenylene group.

According to an exemplary embodiment of the present specification, Ar5 is a substituted or unsubstituted triazine group.

According to an exemplary embodiment of the present specification, Ar5 is a triazaine group which is unsubstituted or substituted with a monocyclic or polycyclic aryl group having 6 to 30 carbon atoms.

According to an exemplary embodiment of the present specification, Ar5 is a triazine group substituted with a phenyl group.

According to an exemplary embodiment of the present specification, EG-1 is represented by the following compound.

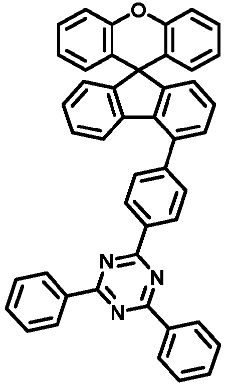

The electron transport layer is a layer which accepts electrons from an electron injection layer and transports the electrons to a light emitting layer. An electron transport material is preferably a material having high electron mobility which may proficiently accept electrons from a cathode and transfer the electrons to a light emitting layer. Specific examples thereof include: Al complexes of 8-hydroxyquinoline; complexes including Alq₃; organic radical compounds; hydroxyflavone-metal complexes; and the like, but are not limited thereto. The electron transport layer may be used with any desired cathode material, as used according to the related art. In particular, an appropriate cathode material is a typical material which has a low work function, followed by an aluminum layer or a silver layer. Specific examples thereof include cesium, barium, calcium, ytterbium, and samarium, in each case followed by an aluminum layer or a silver layer.

According to the present specification, the electron transport layer includes a compound of the following Chemical Formula ET-1, but is not limited thereto. 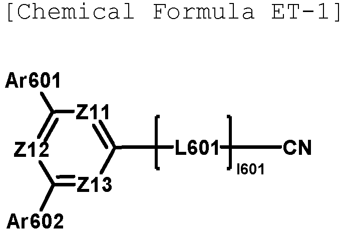 In Chemical Formula ET-1,
at least one of Z11 to Z13 is N, and the others are CH,
L601 is a direct bond; a substituted or unsubstituted arylene group; or a substituted or unsubstituted heteroarylene group, and
Ar601 and Ar602 are the same as or different from each other, and are each independently a substituted or unsubstituted aryl group; or a substituted or unsubstituted heteroaryl group, and
l601 is an integer from 1 to 5, and when l601 is 2 or higher, two or more L601's are the same as or different from each other.

According to an exemplary embodiment of the present specification, L601 is a substituted or unsubstituted monocyclic or polycyclic arylene group having 6 to 30 carbon atoms.

According to an exemplary embodiment of the present specification, L601 is a phenylene group; a biphenylylene group; or a naphthylene group.

According to an exemplary embodiment of the present specification, Ar601 and Ar602 are the same as or different from each other, and are each independently a substituted or unsubstituted monocyclic or polycyclic aryl group having 6 to 30 carbon atoms.

According to an exemplary embodiment of the present specification, Ar601 and Ar602 are a phenyl group.

According to an exemplary embodiment of the present specification, Chemical Formula ET-1 is represented by the following compound.

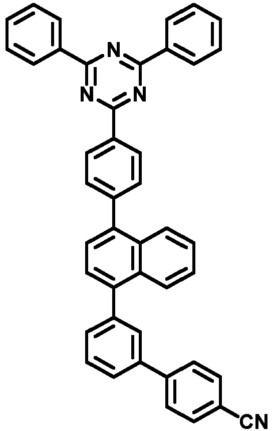

The electron injection layer is a layer which accepts electrons from an electrode. It is preferred that an electron injection material is excellent in ability to transport electrons and has an effect of accepting electrons from the second electrode and an excellent electron injection effect for a light emitting layer or a light emitting material. Further, the electron injection material is preferably a material which prevents excitons produced from a light emitting layer from moving to a hole injection layer and is excellent in ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, metal complex compounds, nitrogen-containing 5-membered ring derivatives, and the like, but are not limited thereto.

Examples of the metal complex compounds include 8-hydroxyquinolinato lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato) (o-cresolato) gallium, bis(2-methyl-8-quinolinato) (1-naphtholato) aluminum, bis(2-methyl-8-quinolinato) (2-naphtholato) gallium, and the like, but are not limited thereto.

The electron blocking layer is a layer which may improve the service life and efficiency of a device by preventing electrons injected from an electron injection layer from passing through a light emitting layer and entering a hole injection layer. As the electron blocking layer, the publicly-known material can be used without limitation, and the electron blocking layer may be formed between a light emitting layer and a hole injection layer, between a light emitting layer and a hole transport layer, or between a light emitting layer and a layer which simultaneously injects and transports holes.

The hole blocking layer is a layer which blocks holes from reaching a cathode, and may be generally formed under the same conditions as those of the electron injection layer. Specific examples thereof include oxadiazole derivatives or triazole derivatives, phenanthroline derivatives, aluminum complexes, and the like, but are not limited thereto.

The capping layer is formed to prevent a considerable amount of light from being lost through total reflection of light in the organic light emitting device, and the capping layer has the performance capable of sufficiently protecting the underlying negative electrode and light emitting layer from external moisture penetration or contamination, and has a high refractive index, and thus may prevent light loss caused by total reflection, and materials in the related art can be used without limitation.

According to an exemplary embodiment of the present specification, the capping layer includes a compound represented by the following Chemical Formula CP-1, but is not limited thereto. 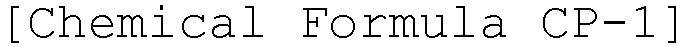 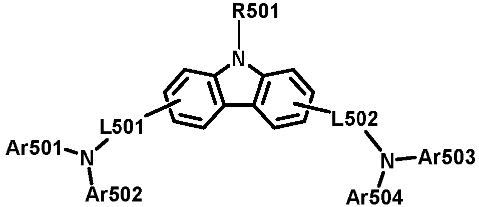 In Chemical Formula CP-1,
L501 and L502 are the same as or different from each other, and are each independently a direct bond; a substituted or unsubstituted arylene group; or a substituted or unsubstituted heteroarylene group, and
R501 and Ar501 to Ar504 are the same as or different from each other, and are each independently a substituted or unsubstituted aryl group; or a substituted or unsubstituted heteroaryl group, or adjacent groups are bonded to each other to form a substituted or unsubstituted ring.

According to an exemplary embodiment of the present specification, L501 and L502 are the same as or different from each other, and are each independently a substituted or unsubstituted monocyclic or polycyclic arylene group having 6 to 30 carbon atoms.

According to an exemplary embodiment of the present specification, L501 and L502 are a phenylene group.

According to an exemplary embodiment of the present specification, R501 and Ar501 to Ar504 are the same as or different from each other, and are each independently a substituted or unsubstituted monocyclic or polycyclic aryl group having 6 to 30 carbon atoms, or are bonded to an adjacent group to form a substituted or unsubstituted monocyclic or polycyclic hetero ring having 2 to 30 carbon atoms.

According to an exemplary embodiment of the present specification, R501 and Ar501 to Ar504 are a phenyl group, or are bonded to an adjacent group to form a carbazole group which is unsubstituted or substituted with a phenyl group.

According to an exemplary embodiment of the present specification, Ar501 and L501 are bonded to form a carbazole substituted with a phenyl group.

According to an exemplary embodiment of the present specification, Ar503 is bonded to L503 to form a carbazole substituted with a phenyl group.

According to an exemplary embodiment of the present specification, Chemical Formula CP-1 is represented by the following compound.

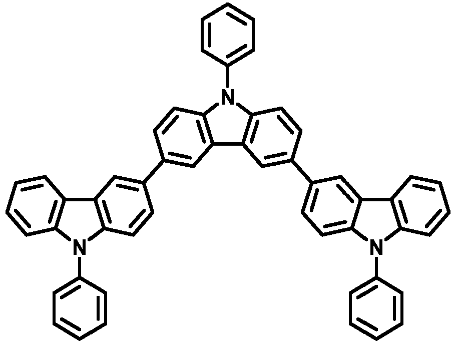

The organic light emitting device according to the present specification may be a top-emission type, a bottom-emission type or a dual-emission type depending on the materials used.

The organic light emitting device according to the present specification may be included and used in various electronic devices. For example, the electronic device may be a display panel, a touch panel, a solar module, a lighting device, and the like, and is not limited thereto.

### [Mode for Invention]

Hereinafter, the present specification will be described in detail with reference to Examples, Comparative Examples, and the like for specifically describing the present specification. However, the Examples and the Comparative Examples according to the present specification may be modified in various forms, and it is not interpreted that the scope of the present specification is limited to the Examples and the Comparative Examples described below in detail. The Examples and the Comparative Examples of the present specification are provided to more completely explain the present specification to a person with ordinary skill in the art.

Preparation Example 1. Synthesis of Chemical Formulae A1, A2 and A3 (Synthesis of Chemical Formulae A1-1 to A1-5, A2-1 and A3-1 to A3-12)

### 1) Synthesis of Chemical Formula A1

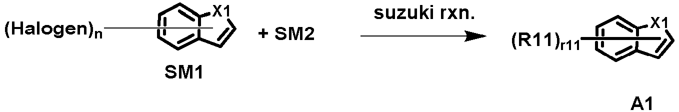

After SM1 (1 eq) and SM2 (1.1 eq) were added to tetrahydrofuran (THF) (excess), a 2 M aqueous potassium carbonate solution (30 volume ratio compared to THF) was added thereto, and tetrakis triphenyl-phosphino palladium (2 mol%) was added thereto, and then the resulting mixture was heated and stirred at 85°C for 10 hours. After the temperature was lowered to room temperature and the reaction was terminated, layers were separated by removing the aqueous potassium carbonate solution, and the residue was purified by column chromatography with hexane and ethyl acetate to prepare Chemical Formula A1 (Chemical Formulae A1-1 to A1-5).

In the reaction scheme, n is an integer from 1 to 6, R11 has the same definitions as R1 to R6 in Chemical Formula 1, and r11 is an integer from 1 to 6.

Chemical Formulae A1-1 to A1-5 in Table 1 were synthesized in the same manner as in the synthesis of Chemical Formula A1, except that SM1 and SM2 were changed to SM1 and SM2 in the following Table 1.

**[Table 1]**

| Chemical Formula | SM1 | SM2 | Product | Yield | MS [M+H]+ |
|---|---|---|---|---|---|
| A1-1 | 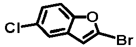 | 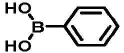 | 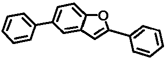 | 82% | 271.33 |
| A1-2 | 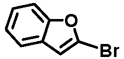 | 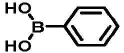 | 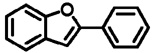 | 84% | 195.23 |
| A1-3 | 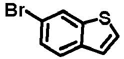 | 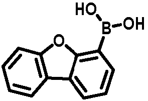 | 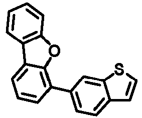 | 80% | 380.27 |
| A1-4 | 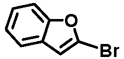 | 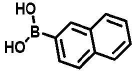 | 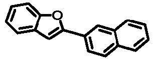 | 60% | 245.29 |
| A1-5 | 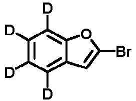 | 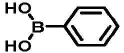 | 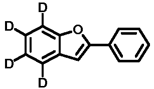 | 75% | 199.26 |

### 2) Synthesis of Chemical Formula A2

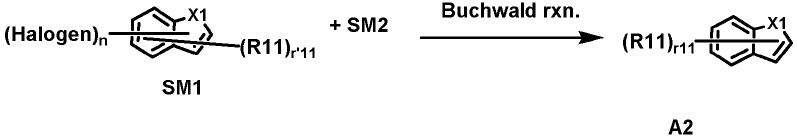

SM1 (1 eq), SM2 (1.02 eq), and sodium-t-butoxide (1.4 eq) were put into xylene, the resulting mixture was heated and stirred, then refluxed, and [bis(tri-t-butylphosphine)]palladium (1 mol%) was added thereto. After the temperature was lowered to room temperature and the reaction was terminated, the resulting product was purified by column chromatography with hexane and ethyl acetate to prepare Chemical Formula A2 (Chemical Formula A2-1).

In the reaction scheme, R11 is the same as the definitions of R1 to R6 in Chemical Formula 1, r11 is an integer from 1 to 6, and r'11 is an integer from 1 to 5.

In the reaction scheme, n is an integer from 1 to 6, and 2≤n+r'11≤6.

**[Table 2]**

| Chemical Formula | SM1 | SM2 | Product | Yield | MS [M+H]+ |
|---|---|---|---|---|---|
| A2-1 | 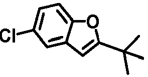 | 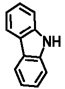 | 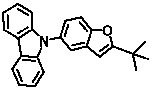 | 78% | 340.44 |

### 3) Synthesis of Chemical Formula A3

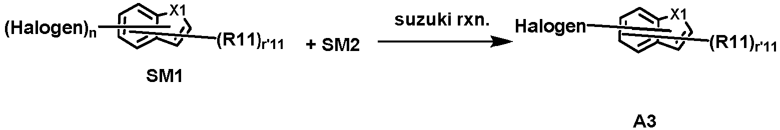

Chemical Formulae A3-1 to A3-12 in Table 3 were synthesized in the same manner as in the method of synthesizing Chemical Formula A1, except that SM1 and SM2 were changed to those in the following Table 3.

In the reaction scheme, R11 has the same definition as R1 to R6 in Chemical Formula 1, and r'll is an integer from 1 to 6.

In the reaction scheme, n is an integer from 1 to 6, and 2≤n+r'11≤6.

**[Table 3]**

| Chemica l Formula | SM1 | SM2 | Product | Yiel d | MS [M+H] + |
|---|---|---|---|---|---|
| A3-1 | 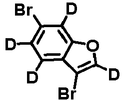 | 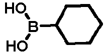 | 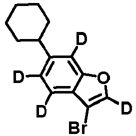 | 30% | 284.2 0 |
| A3-2 | 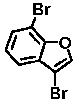 | 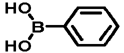 | 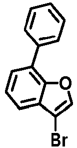 | 28% | 274.1 3 |
| A3-3 | 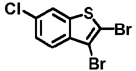 | 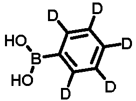 | 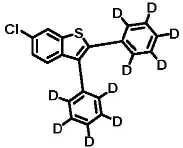 | 77% | 331.9 1 |
| A3-4 | 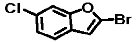 | 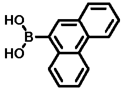 | 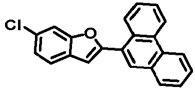 | 79% | 329.8 0 |
| A3-5 | 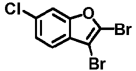 | 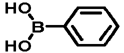 | 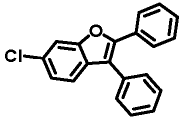 | 62% | 305.7 7 |
| A3-6 | 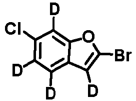 | 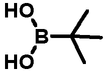 | 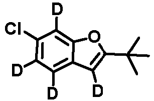 | 70% | 213.7 1 |
| A3-7 | 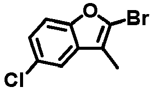 | 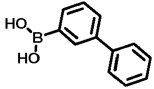 | 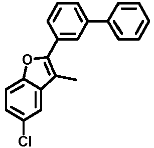 | 76% | 305.7 7 |
| A3-8 | 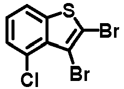 | 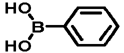 | 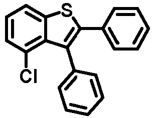 | 77% | 321.8 3 |
| A3-9 | 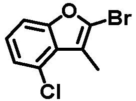 | 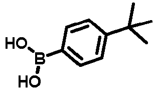 | 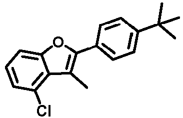 | 72% | 285.7 8 |
| A3-10 | 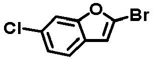 | 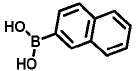 | 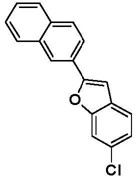 | 80% | 279.7 4 |
| A3-11 | 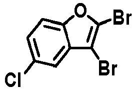 | 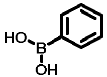 | 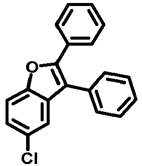 | 74% | 195.6 6 |
| A3-12 | 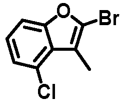 | 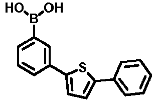 | 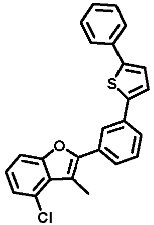 | 78% | 387.8 9 |

Preparation Example 2. Synthesis of Chemical Formulae B1 and B2 (Synthesis of Chemical Formulae B1-1 to B1-6 and B2-1)

### 1) Synthesis of Chemical Formula B1

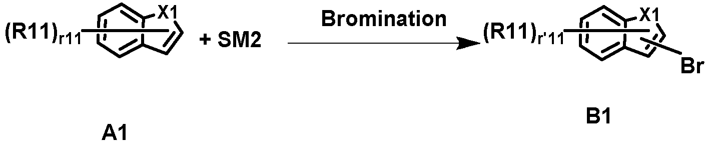

After SM1 (1 eq) was dissolved in DMF (excess), the temperature was lowered to 0°C, the temperature was stabilized, and then, N-bromosuccinimide (1 eq), which is SM2, was added thereto. Thereafter, the reactant was warmed to room temperature and stirred for 1 hours, and 1 N HCl (excess) was added thereto to terminate the reaction. After the reaction was completed, the solvent was removed by separating layers, and then the residue was subjected to silica column chromatography (ethyl acetate/hexane 1:15) to prepare Chemical Formulae B1 (Chemical Formulae B1-1 to B1-6).

The definitions of R11, r11, and r'll are the same as those described above.

Chemical Formulae B1-1 to B1-6 in Table 4 were synthesized in the same manner as in the method of synthesizing Chemical Formula B1, except that SM1 and SM2 were changed to SM1 and SM2 in the following Table 4.

**[Table 4]**

| Chemica l Formula | SM1 | SM2 | Product | Yiel d | MS [M+H] + |
|---|---|---|---|---|---|
| B1-1 | A1-1 | N-bromosuccimid e | 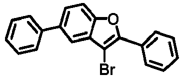 | 72% | 350.23 |
| B1-2 | A1-2 | N-bromosuccimid e | 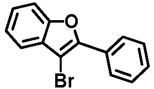 | 70% | 274.13 |
| B1-3 | A2-1 | N-bromosuccimid e | 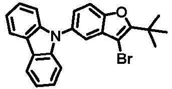 | 68% | 419.33 |
| B1-4 | A1-3 | N-bromosuccimid e | 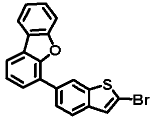 | 62% | 380.27 |
| B1-5 | A1-4 | N-bromosuccimid e | 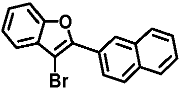 | 70% | 324.19 |
| B1-6 | A1-5 | N-bromosuccimid e | 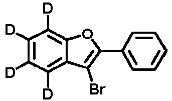 | 65% | 278.15 |

### 2) Synthesis of Chemical Formula B2

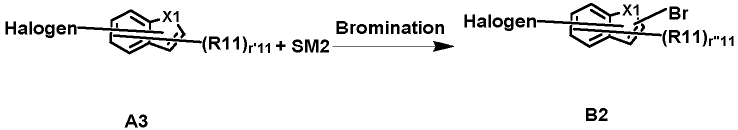

Chemical Formula B2 was prepared in the same manner as in the synthesis of Chemical Formula B1, except that SM1 and SM2 were changed to SM1 and SM2 in the following Table 5.

In the reaction scheme, the definitions of R11 and r'll are the same as those described above, and r"11 is an integer from 1 to 4.

**[Table 5]**

| Chemica l Formula | SM1 | SM2 | Product | Yiel d | MS [M+H] + |
|---|---|---|---|---|---|
| B2-1 | 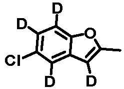 | N-Bromosuccinimid e | 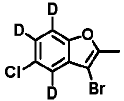 | 71% | 249.5 2 |

Preparation Example 3. Synthesis of Chemical Formulae C1 and C2 (Synthesis of Chemical Formulae C1-1 to C1-6, C2-1 and C2-12)

### 1) Synthesis of Chemical Formula C1

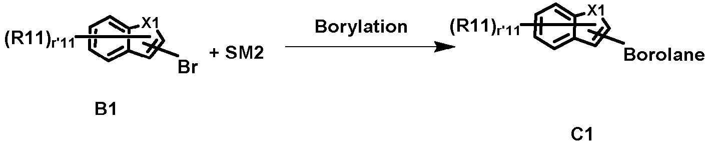

SM1 (1 eq) and SM2 (1.3 eq) were put into 1,4-dioxane (12-fold <mass ratio> compared to SM1), potassium acetate (3 eq) was added thereto, and the resulting mixture was stirred and refluxed. After palladium acetate (0.02 eq) and tricyclohexylphosphine (0.04 eq) are stirred in 1,4-dioxane for 5 minutes, the resulting mixture was added thereto, and 2 hours later, the mixture was cooled to room temperature after confirming that the reaction was terminated. After ethanol and water were added thereto, the resulting mixture was filtered and purified by recrystallization with ethyl acetate and ethanol to prepare Chemical Formulae C1 (Chemical Formulae C1-1 and C1-6). In the reaction scheme, the definitions of R11 and r'll are the same as those described above.

Chemical Formulae C1-1 to C1-6 in Table 6 were synthesized in the same manner as in the synthesis of Chemical Formula C1, except that SM1 and SM2 were changed to SM1 and SM2 in the following Table 6.

**[Table 6]**

| Chemical Formula | SM1 | SM2 | Product | Yield | MS [M+H]+ |
|---|---|---|---|---|---|
| C1-1 | B1-1 | 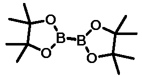 | 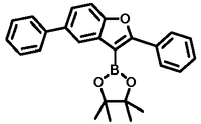 | 83% | 397.29 |
| C1-2 | B1-2 | 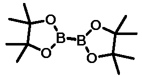 | 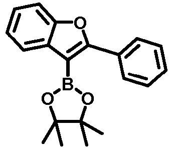 | 78% | 321.20 |
| C1-3 | B1-3 | 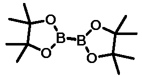 | 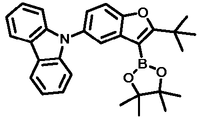 | 80% | 466.40 |
| C1-4 | B1-4 | 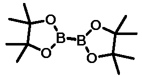 | 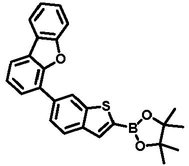 | 79% | 427.34 |
| C1-5 | B1-5 | 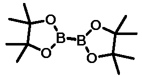 | 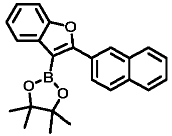 | 75% | 371.26 |
| C1-6 | B1-6 | 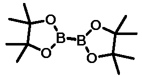 | 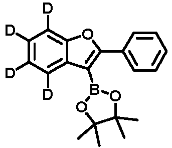 | 68% | 352.55 |

### 2) Synthesis of Chemical Formula C2

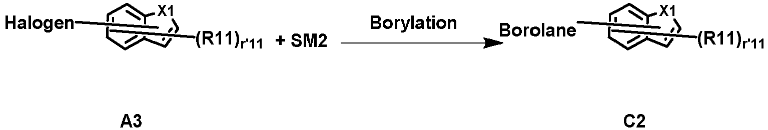

Chemical Formulae C2-1 to C2-12 in Table 7 were synthesized in the same manner as in the synthesis of Chemical Formula C1, except that SM1 and SM2 were changed to SM1 and SM2 in Table 7.

In the reaction scheme, the definitions of R11 and r'll are the same as those described above.

**[Table 7]**

| Chemical Formula | SM1 | SM2 | Product | Yield | MS [M+H]+ |
|---|---|---|---|---|---|
| C2-1 | A3-1 | 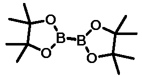 | 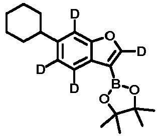 | 74% | 331.27 |
| C2-2 | A3-2 | 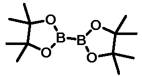 | 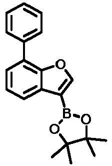 | 70% | 321.20 |
| C2-3 | A3-3 | 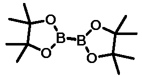 | 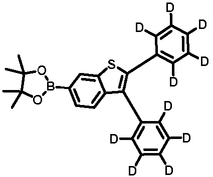 | 79% | 423.42 |
| C2-4 | A3-4 | 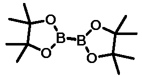 | 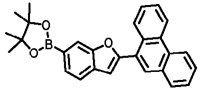 | 80% | 421.32 |
| C2-5 | A3-5 | 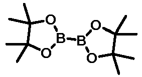 | 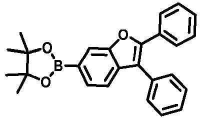 | 81% | 397.29 |
| C2-6 | A3-6 | 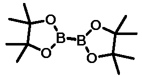 | 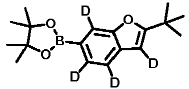 | 75% | 305.23 |
| C2-7 | A3-7 | 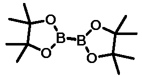 | 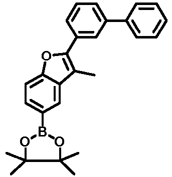 | 79% | 397.29 |
| C2-8 | A3-8 | 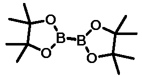 | 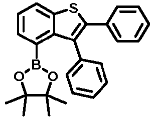 | 77% | 413.35 |
| C2-9 | A3-9 | 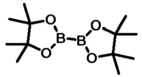 | 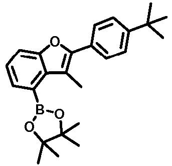 | 73% | 377.30 |
| C2-10 | A3-10 | 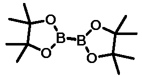 | 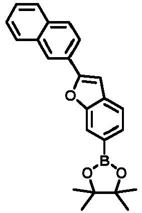 | 75% | 371.26 |
| C2-11 | A3-11 | 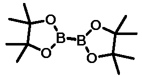 | 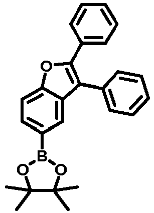 | 70% | 287.18 |
| C2-12 | A3-12 | 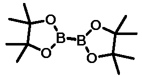 | 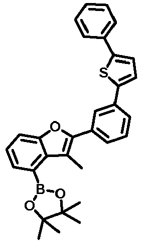 | 76% | 479.41 |

### Preparation Example 4. Synthesis of Chemical Formula D1 (Synthesis of Chemical Formula D1-1)

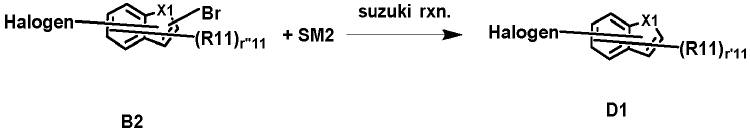

Chemical Formula D1-1 in Table 8 was synthesized in the same manner as in the method of synthesizing Chemical Formula A1, except that SM1 and SM2 were changed to SM1 and SM2 in the following Table 8.

In the reaction scheme, the definitions of R11, r'11, and r"11 are the same as those described above.

**[Table 8]**

| Chemical Formula | SM1 | SM2 | Product | Yield | MS [M+H]+ |
|---|---|---|---|---|---|
| D1-1 | B2-1 | 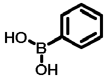 | 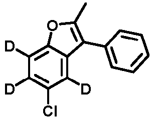 | 83% | 246.72 |

### Preparation Example 5. Synthesis of Chemical Formula E1 (Synthesis of Chemical Formula E1-1)

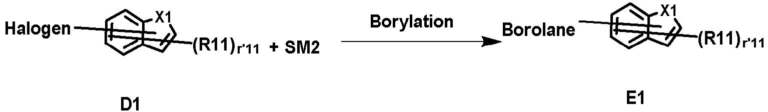

Chemical Formula E1-1 in Table 9 was synthesized in the same manner as in the synthesis of Chemical Formula C1, except that SM1 and SM2 were changed to SM1 and SM2 in the following Table 9.

**[Table 9]**

| Chemical Formula | SM1 | SM2 | Product | Yield | MS [M+H]+ |
|---|---|---|---|---|---|
| E1-1 | D1-1 | 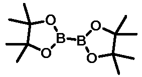 | 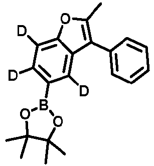 | 80% | 338.24 |

### Preparation Example 6. Synthesis of Chemical Formula F1 (Synthesis of Chemical Formulae F1-1 to F1-6)

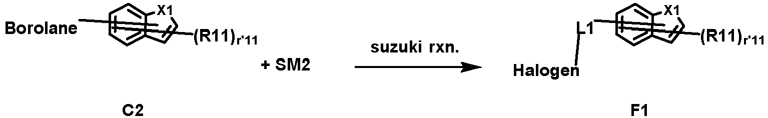

Chemical Formulae F1-1 to F1-6 in Table 10 were synthesized in the same manner as in the synthesis of Chemical Formula A1, except that SM1 and SM2 were changed to SM1 and SM2 in the following Table 10.

In the reaction scheme, the definitions of R11 and r'll are the same as those described above.

**[Table 10]**

| Chemical Formula | SM1 | SM2 | Product | Yield | MS [M+H]+ |
|---|---|---|---|---|---|
| F1-1 | 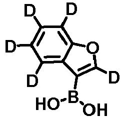 | 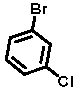 | 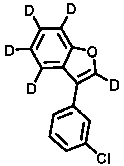 | 80% | 234.71 |
| F1-2 | C2-5 | 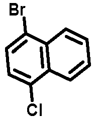 | 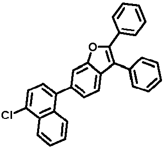 | 82% | 431.93 |
| F1-3 | C2-6 | 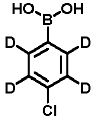 | 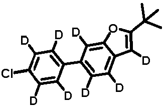 | 81% | 293.83 |
| F1-4 | 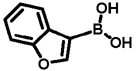 | 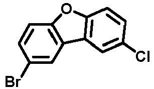 | 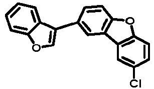 | 80% | 319.76 |
| F1-5 | C2-10 | 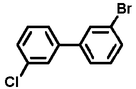 | 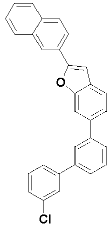 | 80% | 431.93 |
| F1-6 | C2-11 | 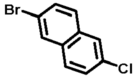 | 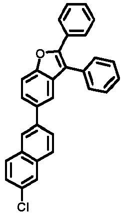 | 76% | 321.82 |

### Preparation Example 7. Synthesis of Chemical Formula G1 (Synthesis of Chemical Formulae G1-1 to G1-6)

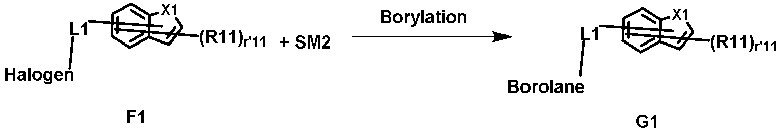

Chemical Formulae G1-1 to G1-6 in Table 11 were synthesized in the same manner as in the synthesis of Chemical Formula C1, except that SM1 and SM2 were changed to SM1 and SM2 in the following Table 11.

In the reaction scheme, the definitions of R11 and r'll are the same as those described above.

**[Table 11]**

| Chemical Formula | SM1 | SM2 | Product | Yield | MS [M+H] + |
|---|---|---|---|---|---|
| G1-1 | F1-1 | 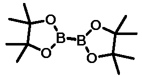 | 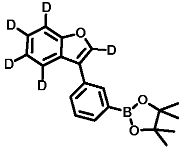 | 78% | 326.23 |
| G1-2 | F1-2 | 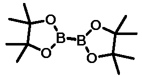 | 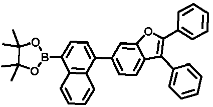 | 79% | 523.45 |
| G1-3 | F1-3 | 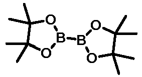 | 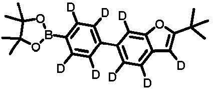 | 70% | 385.35 |
| G1-4 | F1-4 | 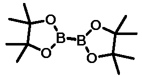 | 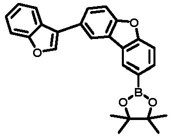 | 75% | 411.28 |
| G1-5 | F1-5 | 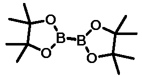 | 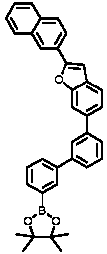 | 76% | 523.45 |
| G1-6 | F1-6 | 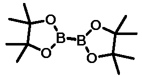 | 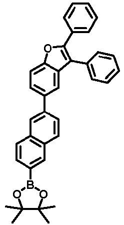 | 69% | 413.34 |

### Preparation Example 8. Synthesis of Compounds 1 to 23, 28 and 29

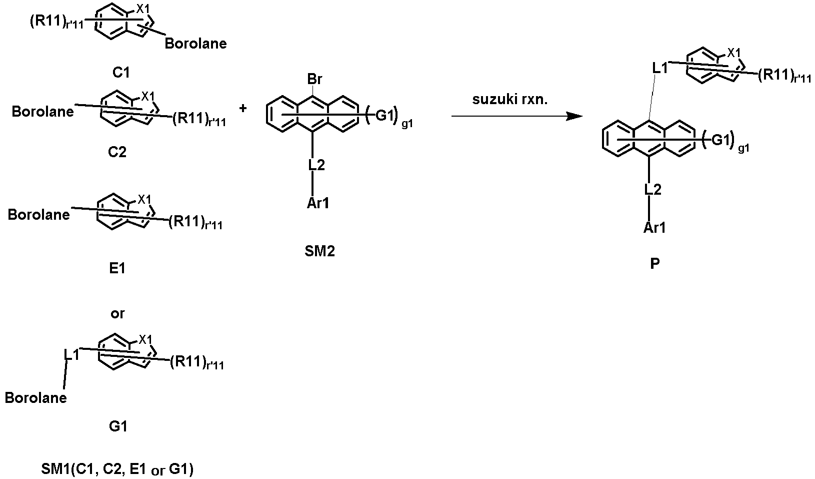

After SM1 (1 eq) and SM2 (1.05 eq) were added to tetrahydrofuran (excess), a 2 M aqueous potassium carbonate solution (30 volume ratio compared to THF) was added thereto, and tetrakis triphenyl-phosphino palladium (2 mol%) was added thereto, and then the resulting mixture was heated and stirred for 10 hours. After the temperature was lowered to room temperature and the reaction was terminated, the aqueous potassium carbonate solution was removed to separate the layers. After it was confirmed that the reaction was terminated, the temperature was lowered to room temperature to remove the solvent, and the residue was purified by recrystallization using toluene to prepare Chemical Formula P (the following Compounds 1 to 23, 28, and 29).

In the reaction scheme, the definitions of R11 and r'll are the same as those described above.

Compounds 1 to 23, 28, and 29 of the following Table 12 were synthesized in the same manner as described above, except that SM1 and SM2 in Preparation Example 8 were changed to SM1 and SM2 of the following Table 12, respectively.

SM2 of the following Table 12 was prepared in the following scheme.

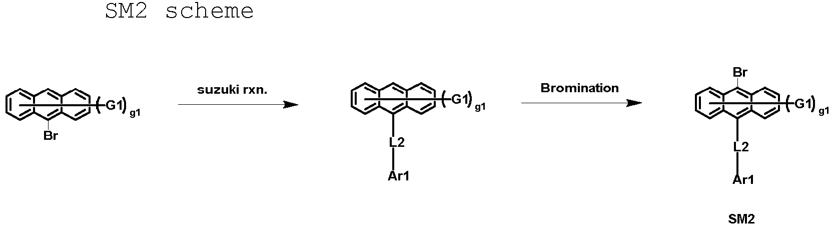

### SM2 Example 1>

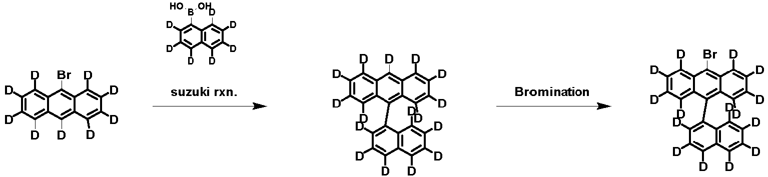

### SM2 Example 2>

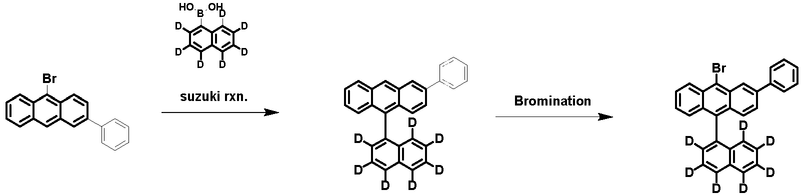

SM2 of Compounds 1 to 28, 28, and 29 was prepared as the synthesis examples as described above.

Furthermore, the Suzuki reaction is the same method as the synthesis of Chemical Formula A1, and the bromination is the same method as the synthesis of Chemical Formula B1, except that SM1 and SM2 are changed.

**[Table 12]**

| Compound | SM1 | SM2 | Product | Yield | MS [M+H] + |
|---|---|---|---|---|---|
| 1 | C1-1 | 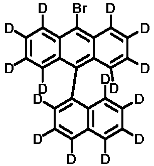 | 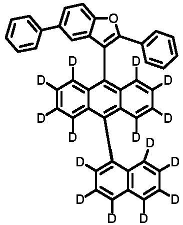 | 62% | 588.80 |
| 2 | C1-2 | 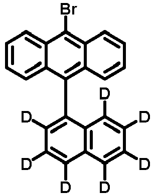 | 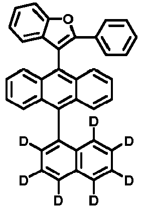 | 66% | 504.65 |
| 3 | C1-3 | 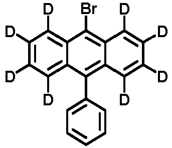 | 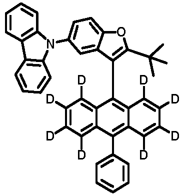 | 63% | 600.80 |
| 4 | C1-4 | 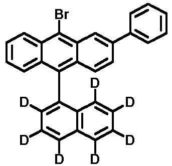 | 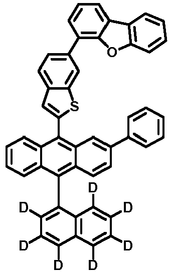 | 65% | 686.89 |
| 5 | C1-5 | 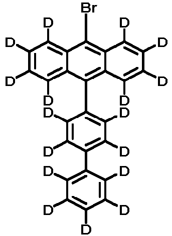 | 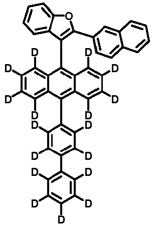 | 60% | 590.81 |
| 6 | G1-1 | 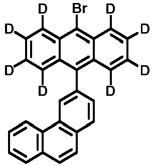 | 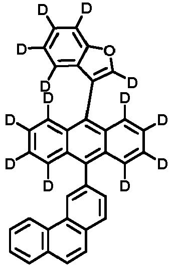 | 67% | 484.65 |
| 7 | 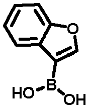 | 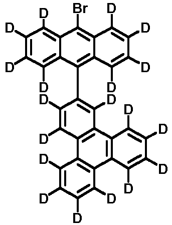 | 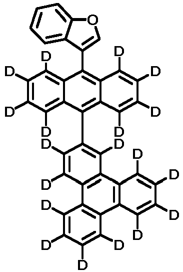 | 58% | 540.75 |
| 8 | C2-1 | 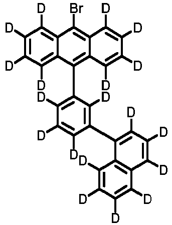 | 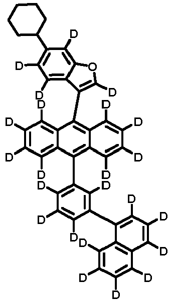 | 63% | 602.90 |
| 9 | C2-2 | 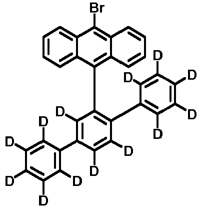 | 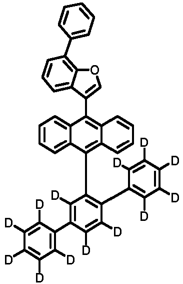 | 66% | 612.82 |
| 10 | 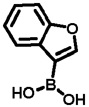 | 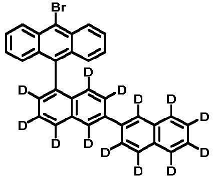 | 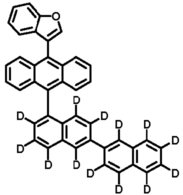 | 60% | 560.75 |
| 11 | C2-3 | 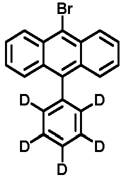 | 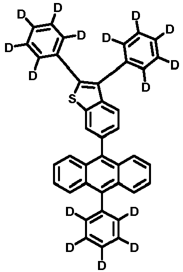 | 67% | 554.80 |
| 12 | C2-4 | 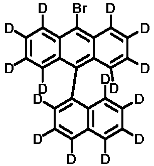 | 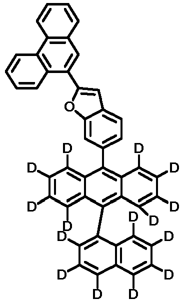 | 68% | 612.82 |
| 13 | G1-2 | 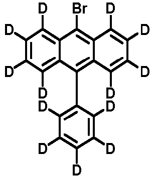 | 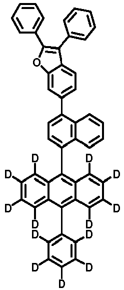 | 60% | 662.88 |
| 14 | 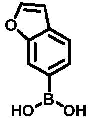 | 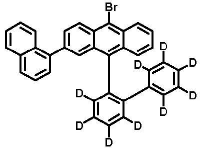 | 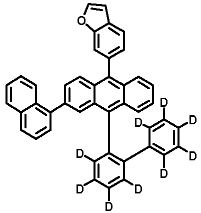 | 61% | 582.76 |
| 15 | G1-3 | 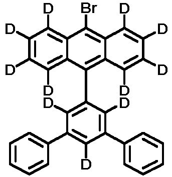 | 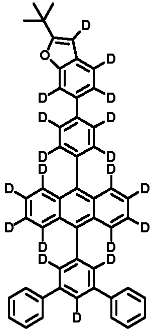 | 63% | 674.97 |
| 16 | C2-7 | 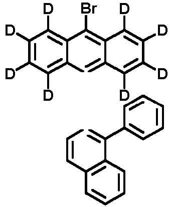 | 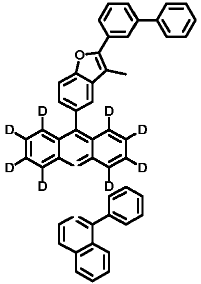 | 66% | 671.88 |
| 17 | E1-1 | 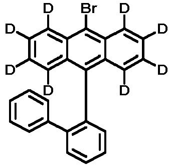 | 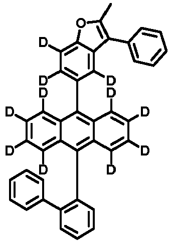 | 68% | 548.74 |
| 18 | G1-4 | 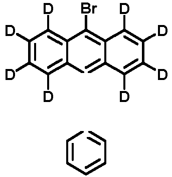 | 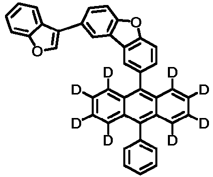 | 66% | 545.68 |
| 19 | C2-8 | 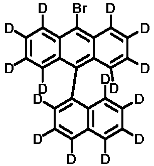 | 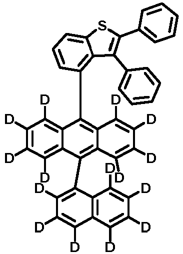 | 69% | 604.86 |
| 20 | C2-9 | 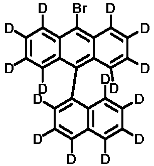 | 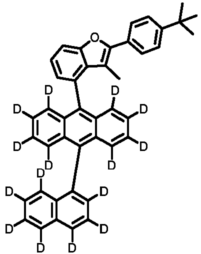 | 65% | 582.84 |
| 21 | G1-5 | 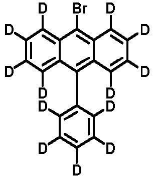 | 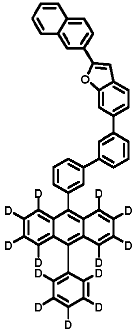 | 63% | 662.88 |
| 22 | G1-6 | 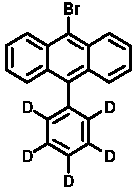 | 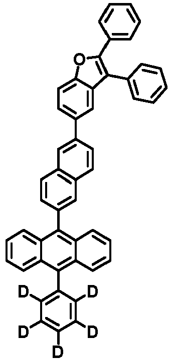 | 60% | 654.84 |
| 23 | C2-12 | 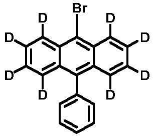 | 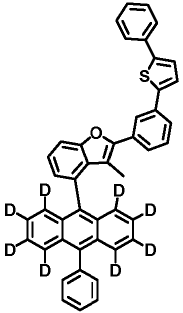 | 66% | 627.84 |
| 28 | C1-1 | 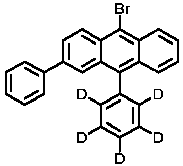 | 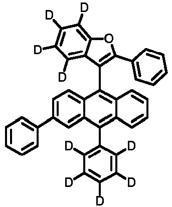 | 65% | 532.70 |
| 29 | C1-6 | 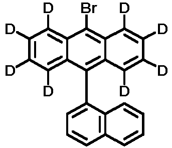 | 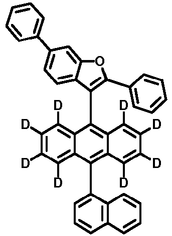 | 72% | 581.76 |

### Preparation Example 9. Synthesis of Compounds 24 to 27

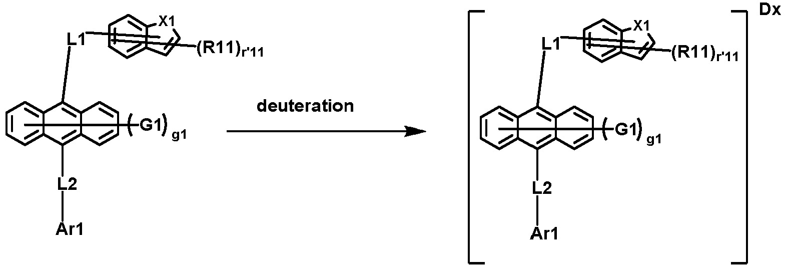

The reactant (1 eq) and trifluoromethanesulfonic acid (cat.) were put into C₆D₆ (10- to 50-fold mass ratio compared to the reactant), and the resulting mixture was stirred at 70°C for 10 minutes to 100 minutes. After the reaction was completed, D₂O (excess) was added thereto, the resulting solution was stirred for 30 minutes, and then trimethylamine (excess) was added dropwise thereto. The reaction solution was transferred to a separatory funnel, and an extraction with water and chloroform was performed. The extract was dried over MgSO₄, and then recrystallized by heating with toluene to obtain Compounds 24 to 27 of the following Table 13.

In the reaction scheme, the substituents are defined as described above, and Dx means the number of deuterium atoms substituted in the compound.

Compounds 24 to 27 of the following Table 13 were synthesized in the same manner as described above, except that SM1 and SM2 in Preparation Examples 1 to 8 were changed to SM1 and SM2 of the following Table 13, respectively. (non-deuterated compound)

**[Table 13]**

| Comp ound | Reactant | Product | React ant m/z | Product theory max m/z | Reaction time (min) |
|---|---|---|---|---|---|
| 24 | 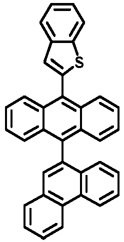 | 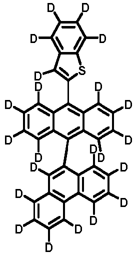 | 486 | 508 | 50 |
| 25 | 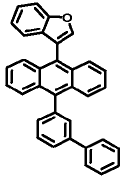 | 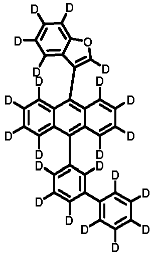 | 446 | 468 | 50 |
| 26 | 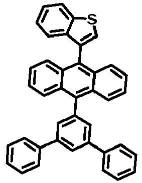 | 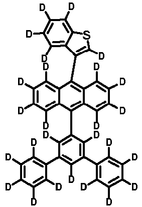 | 538 | 564 | 50 |
| 27 | 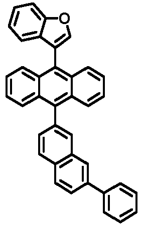 | 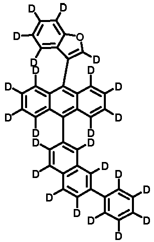 | 496 | 520 | 50 |

| Comp ound | Product actual max m/z | number of total hydrogens | Numbe r of D subst ituti ons | D substit ution ratio (%) | Yield |
|---|---|---|---|---|---|
| 24 | 505 | 22 | 19 | 86% | 69% |
| 25 | 466 | 22 | 20 | 91% | 68% |
| 26 | 560 | 26 | 23 | 88% | 70% |
| 27 | 516 | 24 | 20 | 83% | 74% |

The compounds in Table 13 differ in the deuteration method from the compounds in Table 12, the deuteration method is a method of first synthesizing a compound substituted only with hydrogen and then substituting the compound with deuterium, and reference was made to the prior document of KR1538534 [the degree of deuterium substitution for each product varies depending on the reaction time, and the substitution rate is determined depending on the maximum m/z (M+) value] for the deuterium substitution for the above product.

### <Example 1> Manufacture of OLED

As a positive electrode, a substrate on which ITO/Ag/ITO were deposited to have a thickness of 70/1,000/70 Å was cut into a size of 50 mm x 50 mm x 0.5 mm, put into distilled water in which a dispersant was dissolved, and washed with ultrasonic waves. A product manufactured by Fischer Co., was used as the detergent, and distilled water twice filtered using a filter manufactured by Millipore Co., was used as the distilled water. After the ITO was washed for 30 minutes, ultrasonic washing was conducted twice repeatedly using distilled water for 10 minutes. After the washing using distilled water was completed, ultrasonic washing was conducted using isopropyl alcohol, acetone, and methanol solvents in this order, and drying was then conducted.

HI-1 was thermally vacuum-deposited to have a thickness of 50 Å on the positive electrode thus prepared, thereby forming a hole injection layer, and HT1 as a material which transports holes was vacuum-deposited to have a thickness of 1,150 Å thereon, thereby forming a hole transport layer, Next, a hole adjusting layer was formed using EB1 (150 Å), and then Compound 1 (host) and dopant BD1 (2 wt%) were vacuum-deposited to have a thickness of 360 Å, thereby forming a light emitting layer. Thereafter, ET1 was deposited to have a thickness of 50 Å, thereby forming an electron adjusting layer, and Compounds ET2 and Liq were mixed at a ratio of 7:3, thereby forming an electron transport layer having a thickness of 250 Å. Sequentially, after magnesium and lithium fluoride (LiF) were deposited to have a thickness of 50 Å to form a film as an electron injection layer <EIL>, magnesium and silver (1:4) were used to form a film having a thickness of 200 Å as a negative electrode, and then CP1 was deposited to have a thickness of 600 Å, thereby completing a device. In the aforementioned procedure, the deposition rates of the organic materials were each maintained at 1 Å/sec.

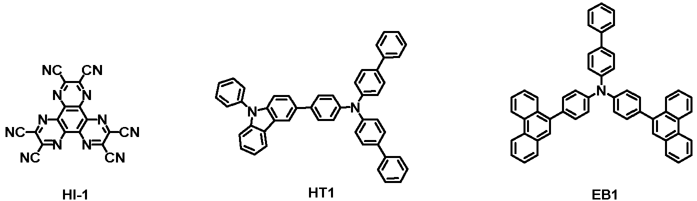

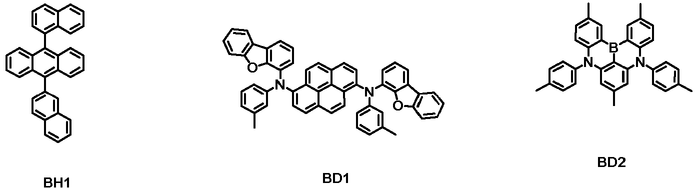

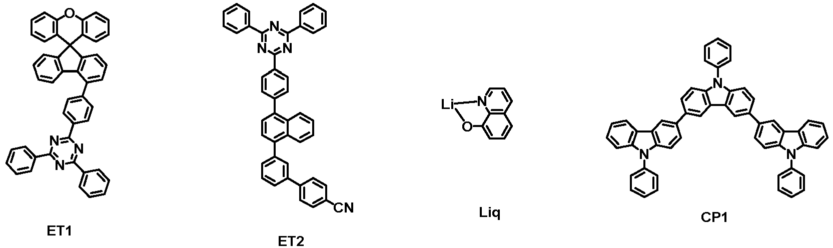

Examples 2 to 34 and Comparative Examples 1 to 11 Organic light emitting devices of Examples 2 to 34 and Comparative Examples 1 to 11 were manufactured in the same manner as in Example 1, except that compounds described in the following Table 14 were used, respectively, instead of Compound 1 and BD1 in Example 1.

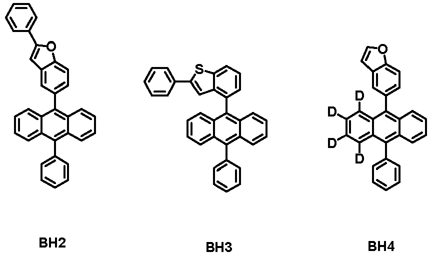

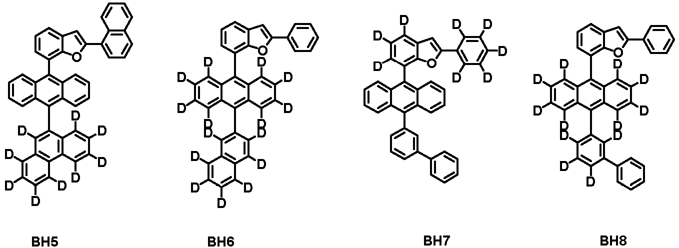

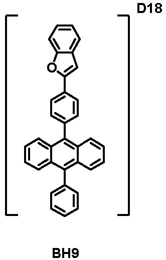

A current of 20 mA/cm² was applied to the organic light emitting devices manufactured in Examples 1 to 34 and Comparative Examples 1 to 11 to measure the voltage, efficiency, color coordinates, and service life (T95), and the results are shown in the following Table 14. In this case, T95 means the time taken for the initial luminance to drop to 95% at a current density of 20 mA/cm².

**[Table 14]**

| Experimental Example 20 mA/cm² | Host | Dopant | Voltage (V) (@20 mA/ cm² ) | Cd/A (@20 mA/ cm² ) | Color coordinate (x,y) | Service life (T95, h) (@20 mA/ cm² ) |
|---|---|---|---|---|---|---|
| Example 1 | Compound 1 | BD1 | 3.32 | 7.12 | (0.135, 0.138) | 72.8 |
| Example 2 | Compound 2 | BD1 | 3.40 | 7.08 | (0.134, 0.137) | 63.5 |
| Example 3 | Compound 3 | BD1 | 3.58 | 6.84 | (0.134, 0.137) | 66.4 |
| Example 4 | Compound 4 | BD1 | 3.31 | 6.80 | (0.134, 0.137) | 63.4 |
| Example 5 | Compound 5 | BD1 | 3.53 | 7.10 | (0.134, 0.137) | 71.2 |
| Example 6 | Compound 6 | BD1 | 3.50 | 6.79 | (0.135, 0.138) | 60.8 |
| Example 7 | Compound 7 | BD1 | 3.51 | 6.80 | (0.134, 0.137) | 70.9 |
| Example 8 | Compound 8 | BD1 | 3.59 | 6.89 | (0.134, 0.137) | 72.3 |
| Example 9 | Compound 9 | BD1 | 3.56 | 7.13 | (0.135, 0.138) | 62.5 |
| Example 10 | Compound 10 | BD1 | 3.60 | 6.85 | (0.134, 0.137) | 65.3 |
| Example 11 | Compound 11 | BD1 | 3.32 | 6.87 | (0.134, 0.137) | 66.0 |
| Example 12 | Compound 12 | BD1 | 3.50 | 6.84 | (0.134, 0.138) | 70.1 |
| Example 13 | Compound 13 | BD1 | 3.33 | 6.88 | (0.134, 0.137) | 71.1 |
| Example 14 | Compound 14 | BD1 | 3.54 | 6.82 | (0.133, 0.142) | 61.6 |
| Example 15 | Compound 15 | BD1 | 3.55 | 6.85 | (0.135, 0.138) | 70.4 |
| Example 16 | Compound 16 | BD1 | 3.57 | 6.88 | (0.134, 0.137) | 65.4 |
| Example 17 | Compound 17 | BD1 | 3.58 | 6.84 | (0.134, 0.137) | 66.2 |
| Example 18 | Compound 18 | BD1 | 3.31 | 6.89 | (0.135, 0.144) | 63.9 |
| Example 19 | Compound 19 | BD1 | 3.58 | 6.90 | (0.134, 0.137) | 71.0 |
| Example 20 | Compound 20 | BD1 | 3.57 | 6.75 | (0.135, 0.143) | 70.2 |
| Example 21 | Compound 21 | BD1 | 3.33 | 6.80 | (0.134, 0.139) | 71.5 |
| Example 22 | Compound 22 | BD1 | 3.59 | 6.82 | (0.135, 0.144) | 66.0 |
| Example 23 | Compound 23 | BD1 | 3.60 | 6.88 | (0.134, 0.139) | 64.8 |
| Example 24 | Compound 24 | BD1 | 3.55 | 6.85 | (0.134, 0.138) | 70.8 |
| Example 25 | Compound 25 | BD1 | 3.51 | 6.84 | (0.134, 0.137) | 71.2 |
| Example 26 | Compound 26 | BD1 | 3.53 | 6.80 | (0.134, 0.137) | 72.6 |
| Example 27 | Compound 27 | BD1 | 3.51 | 6.82 | (0.134, 0.137) | 70.4 |
| Example 28 | Compound 1 | BD2 | 3.40 | 7.35 | (0.122, 0.121) | 60.4 |
| Example 29 | Compound 2 | BD2 | 3.51 | 7.40 | (0.122, 0.122) | 53.4 |
| Example 30 | Compound 13 | BD2 | 3.42 | 7.10 | (0.122, 0.121) | 62.1 |
| Example 31 | Compound 19 | BD2 | 3.67 | 7.21 | (0.122, 0.122) | 63.0 |
| Example 32 | Compound 24 | BD2 | 3.64 | 7.12 | (0.122, 0.122) | 62.5 |
| Example 33 | Compound 28 | BD1 | 3.33 | 7.13 | (0.135, 0.138) | 75.2 |
| Example 34 | Compound 29 | BD1 | 3.35 | 7.08 | (0.135, 0.144) | 70.1 |
| Comparative Example 1 | BH1 | BD1 | 3.90 | 5.88 | (0.134, 0.139) | 40.8 |
| Comparative Example 2 | BH2 | BD1 | 3.80 | 6.02 | (0.134, 0.139) | 31.2 |
| Comparative Example 3 | BH3 | BD1 | 3.80 | 6.11 | (0.134, 0.137) | 20.1 |
| Comparative Example 4 | BH4 | BD1 | 3.80 | 5.68 | (0.134, 0.138) | 23.4 |
| Comparative Example 5 | BH5 | BD1 | 3.76 | 6.08 | (0.134, 0.138) | 30.2 |
| Comparative Example 6 | BH6 | BD1 | 3.81 | 6.11 | (0.134, 0.137) | 41.5 |
| Comparative Example 7 | BH7 | BD1 | 3.77 | 5.89 | (0.134, 0.137) | 28.9 |
| Comparative Example 8 | BH8 | BD1 | 3.78 | 5.90 | (0.134, 0.137) | 34.8 |
| Comparative Example 9 | BH9 | BD1 | 3.91 | 5.98 | (0.134, 0.138) | 42.3 |
| Comparative Example 10 | BH1 | BD2 | 4.01 | 6.11 | (0.122, 0.122) | 35.0 |
| Comparative Example 11 | BH2 | BD2 | 4.00 | 6.20 | (0.122, 0.123) | 20.5 |

According to Table 14 above, Chemical Formula 1 of the present specification elicits the improvement of the blue light emitting device through the improvement of the characteristics of the anthracene blue fluorescent host into which benzofuran or benzothiophene units are introduced.

In the Examples of the present specification, it was possible to observe a clear improvement in device performance of the blue device compared to Comparative Examples 1 to 11 in which various derivatives known in the related art were used.

Comparative Examples 2, 3, 10, and 11 are not deuterated and have structures that differ from those of Chemical Formula 1 in the present specification in the substitution position of the benzofuran or benzothiophene unit, and the compound of Chemical Formula 1 of the present specification showed a significant improvement in service life compared to anthracene derivatives in the related art, and it could be observed that the combination of the substitution position of benzofuran or benzothiophene bonded to the anthracene core and additional substituents showed the improvement in characteristics as a blue fluorescent host.

Although Comparative Examples 4 to 9 show the device results obtained through various deuterium substitutions of the compounds in the related art, the comparative examples also have various substituents and cores of the same series, but it could be observed in Table 14 that the combination of the substitution position of benzofuran or benzothiophene bonded to the anthracene core and additional substituents showed the improvement in characteristics as a blue fluorescent host.

The compound of Chemical Formula 1 of the present specification may adjust the role of smoothly injecting holes into the light emitting layer by using the compounds of Preparation Examples 8 and 9 (Tables 12 and 13 above) as a host for a blue organic light emitting device, and it could be seen through the balance between holes and electrons of the organic light emitting device according to the chemical structure that the organic light emitting device according to the present specification exhibits excellent characteristics in terms of efficiency, driving voltage, and stability.

## Claims

1. A compound selected from the following:

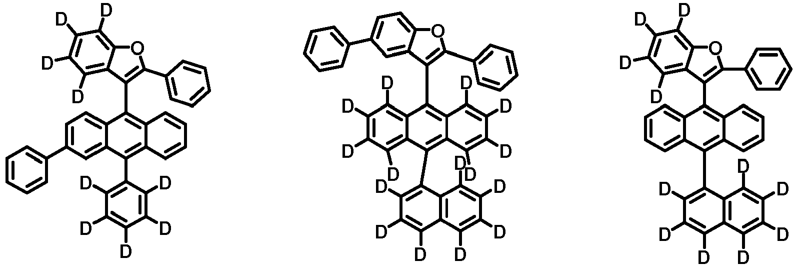

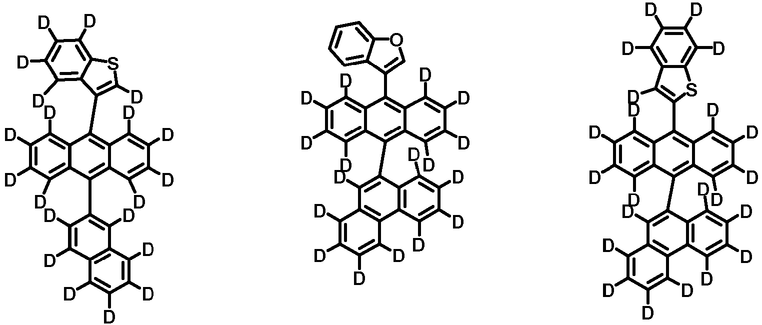

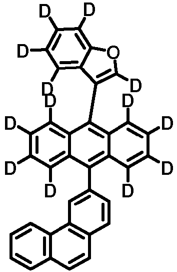

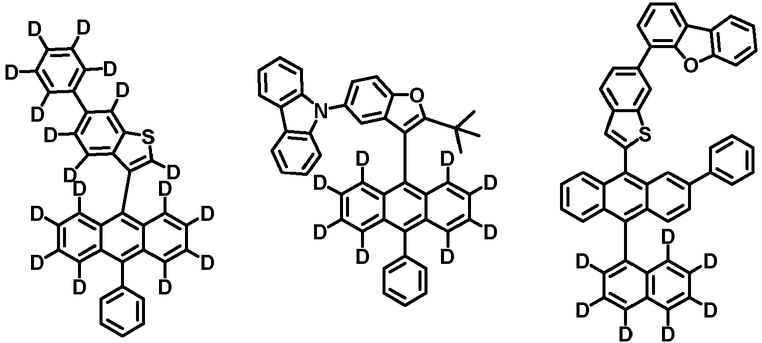

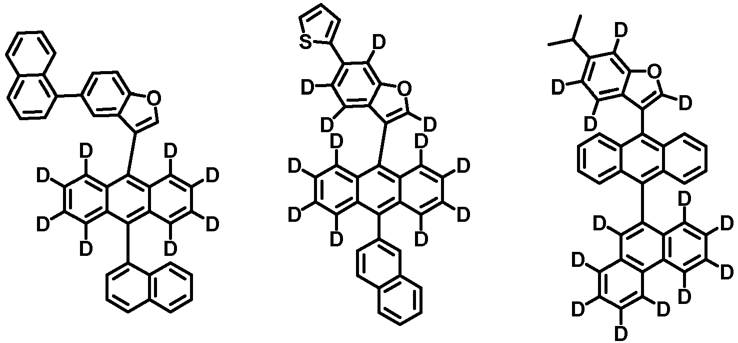

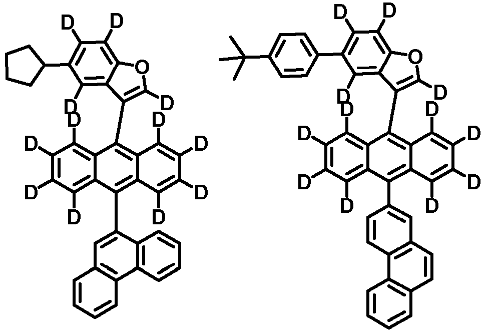

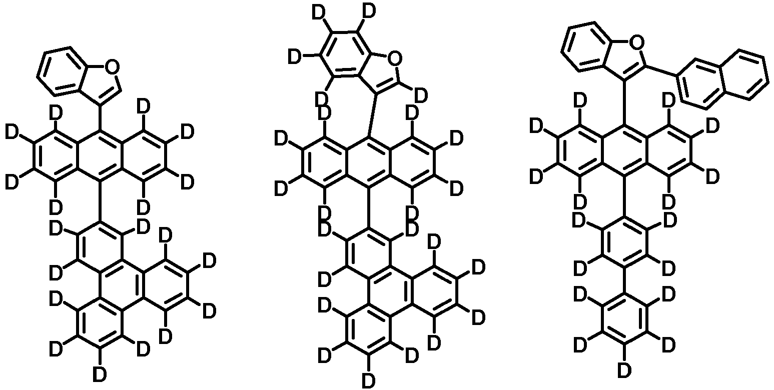

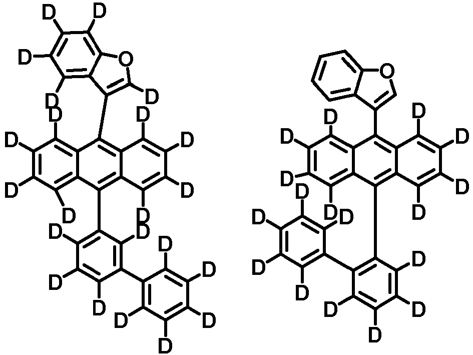

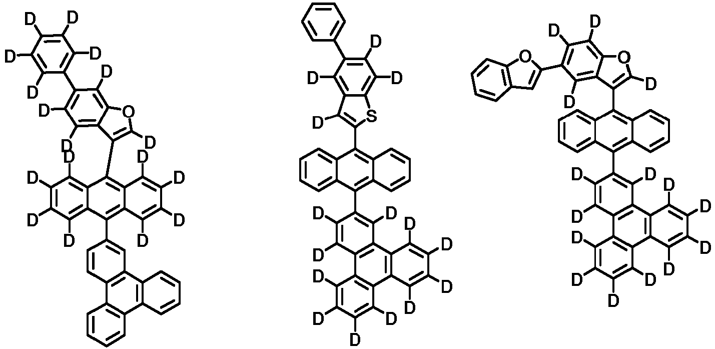

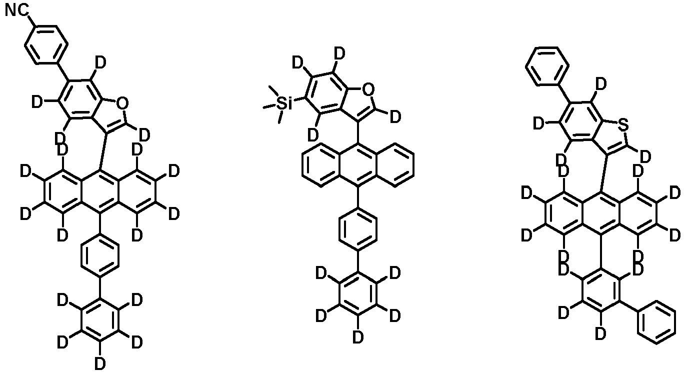

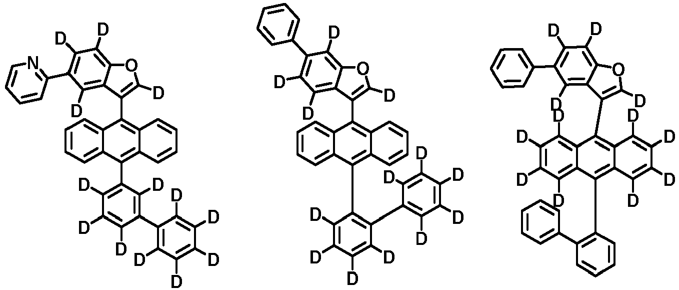

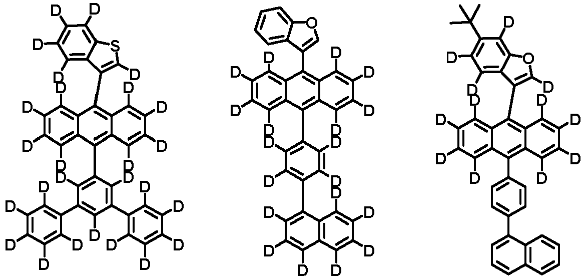

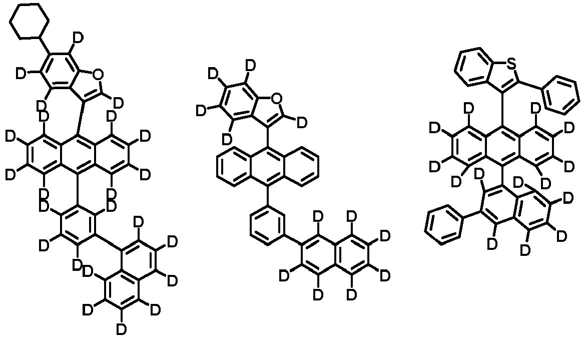

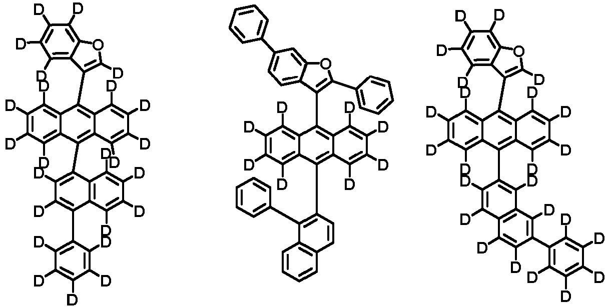

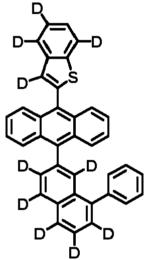

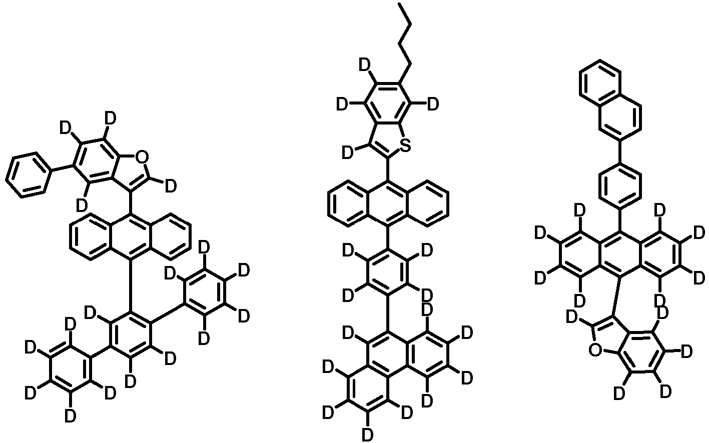

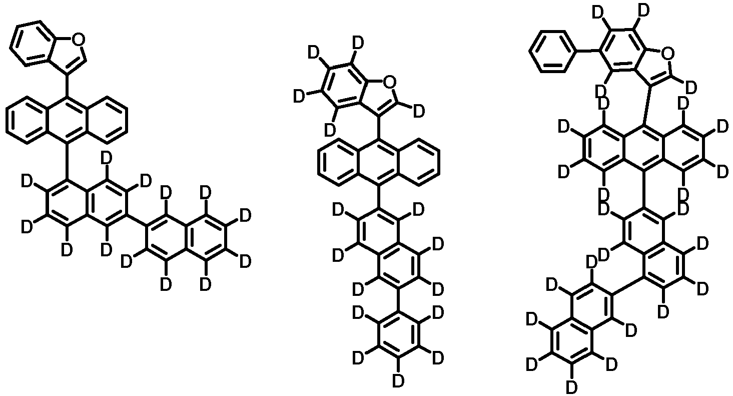

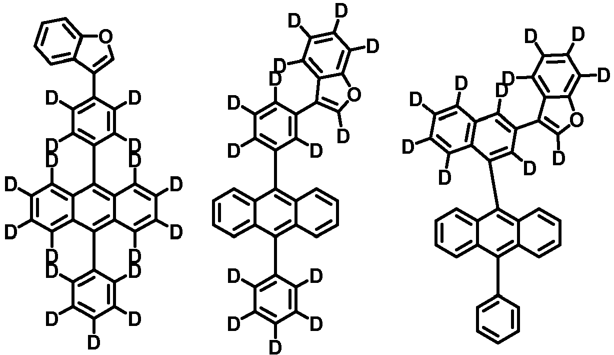

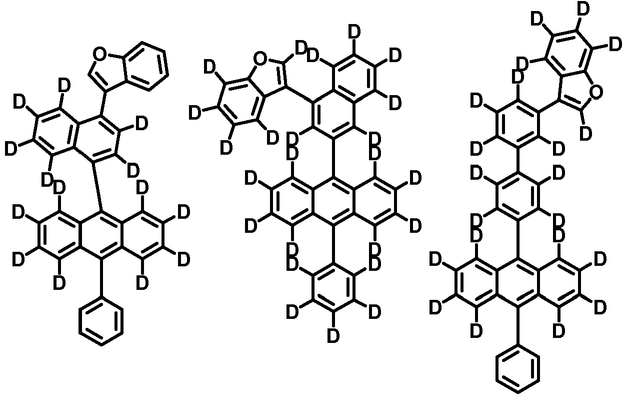

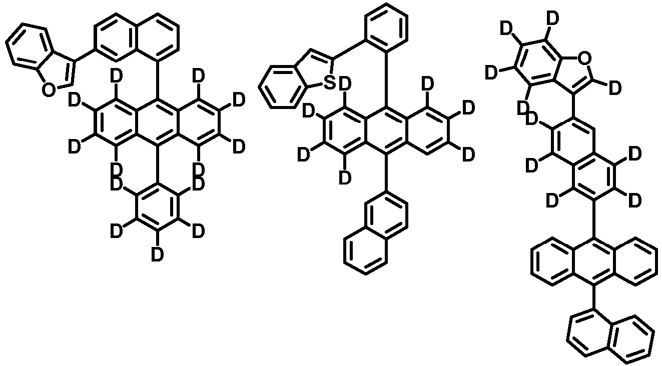

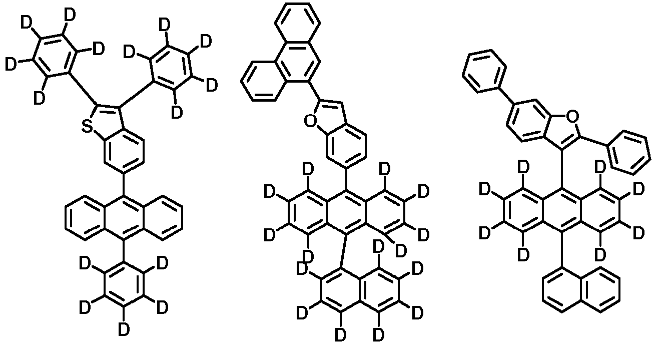

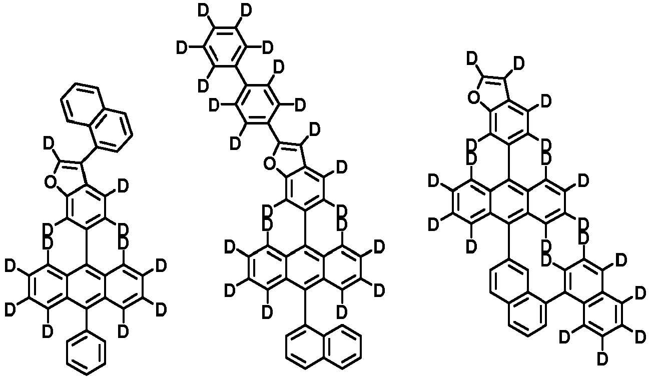

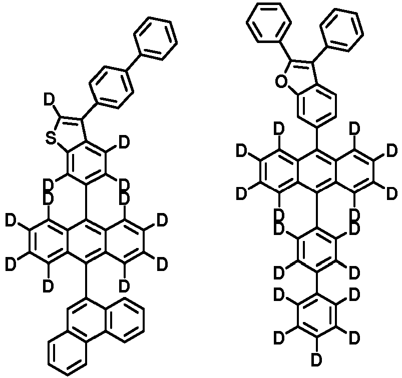

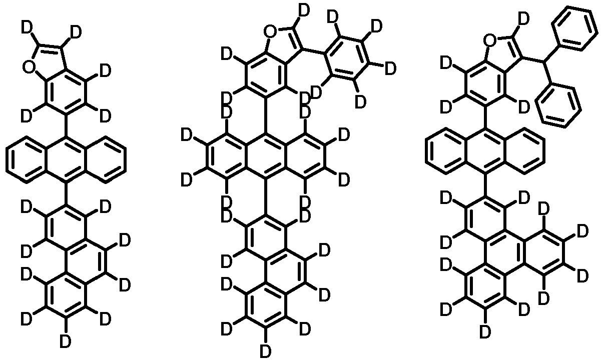

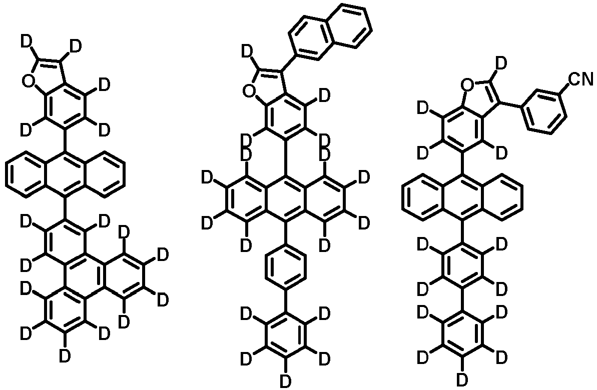

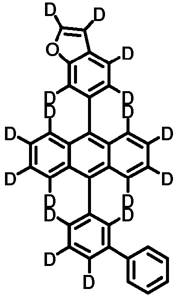

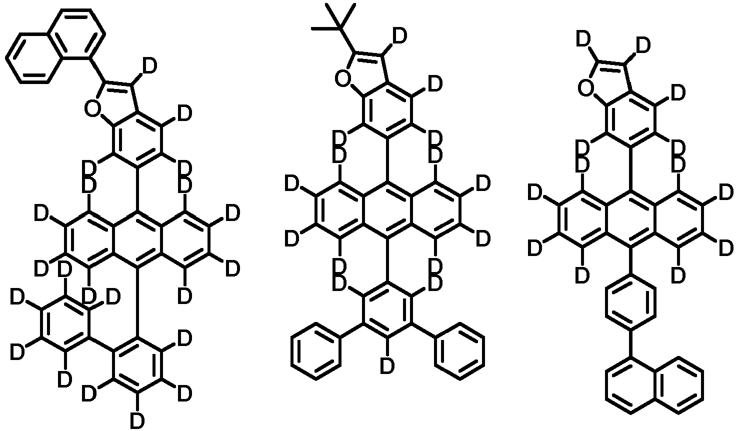

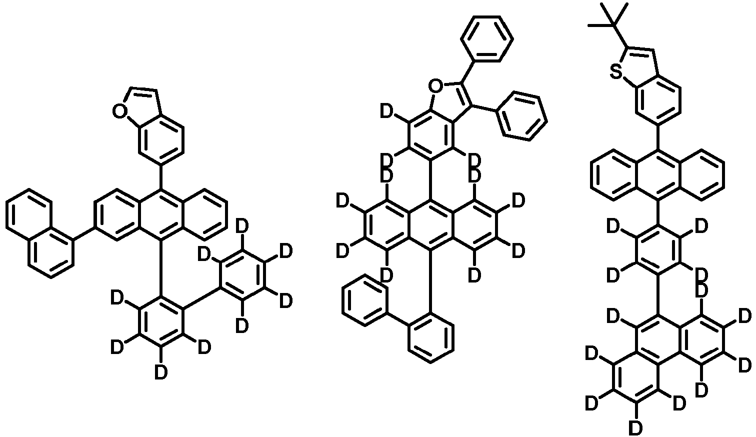

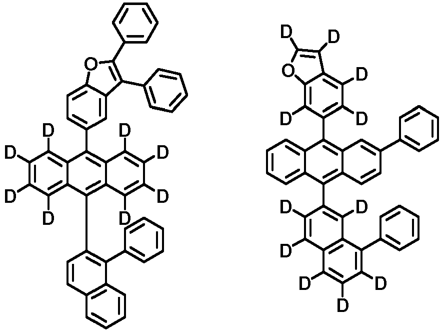

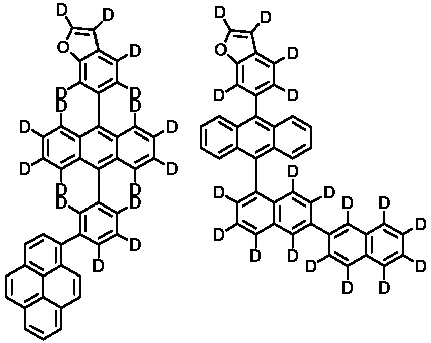

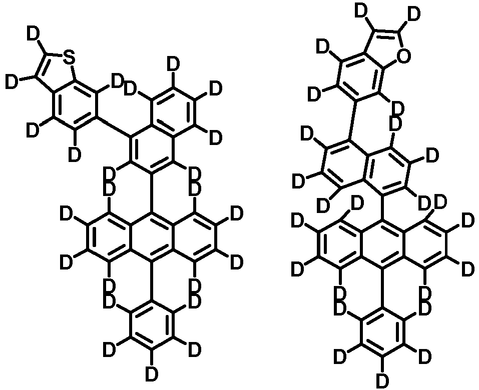

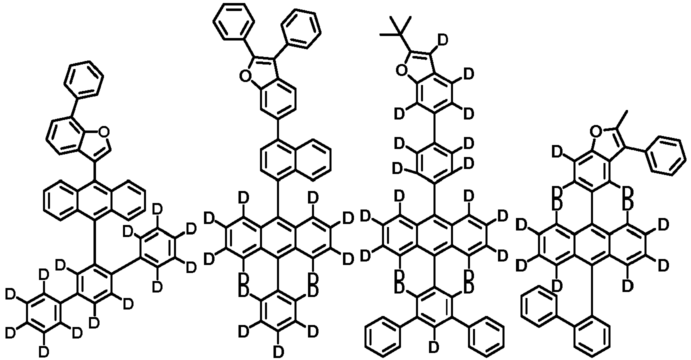

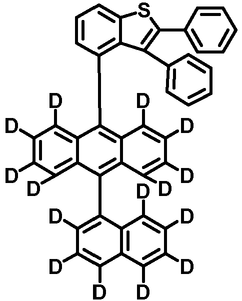

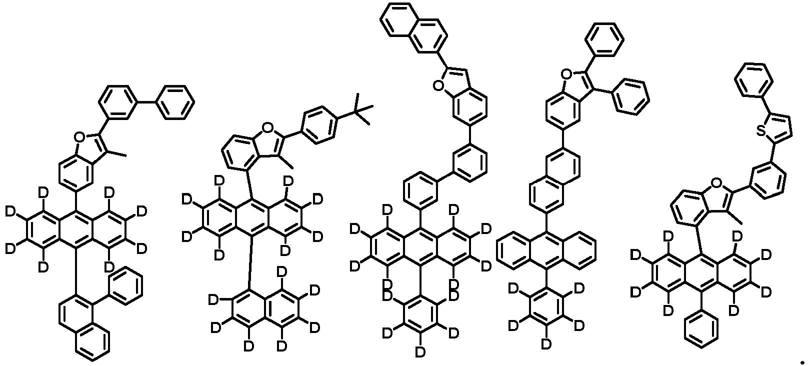

2. An organic light emitting device comprising a first electrode, a second electrode, and one or more organic material layers provided between the first electrode and the second electrode;
wherein the one or more organic material layers comprise a light emitting layer and an electron transport layer;
the light emitting layer comprises a compound of any one of the following Chemical Formulae 1-1 to 1-5 as a host compound; and
the electron transport layer comprises a compound of the following Chemical Formula ET-1: 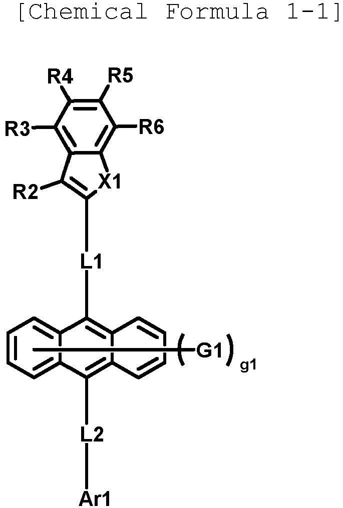 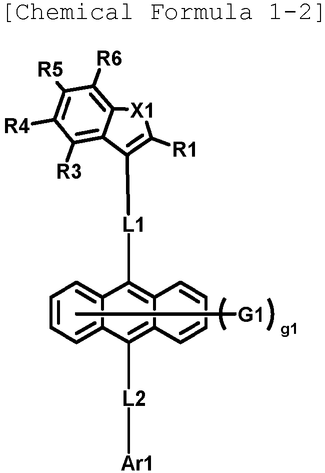 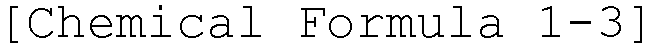 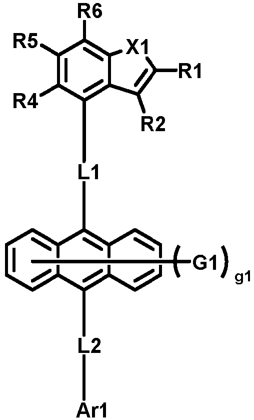 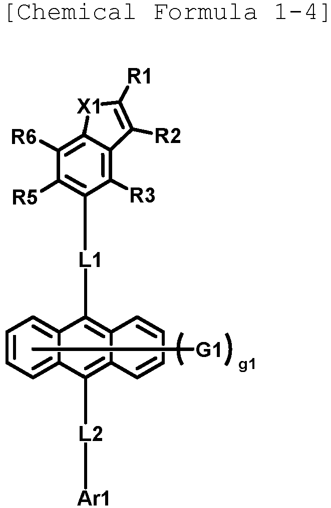 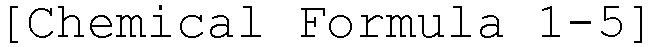 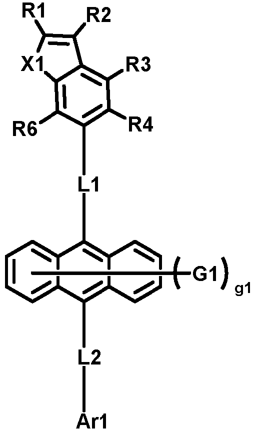
in Chemical Formulae 1-1 to 1-5,
R1 to R6 are the same as or different from each other, and are each independently hydrogen, deuterium, a halogen group, a cyano group, a substituted or unsubstituted alkyl group, a substituted or unsubstituted cycloalkyl group, a substituted or unsubstituted alkoxy group, a substituted or unsubstituted alkenyl group, a substituted or unsubstituted silyl group, a substituted or unsubstituted aryl group, or a substituted or unsubstituted heterocyclic group;
L1 and L2 are the same as or different from each other, and are each independently a direct bond, a substituted or unsubstituted arylene group, or a substituted or unsubstituted divalent heterocyclic group;
Ar1 is a substituted or unsubstituted aryl group; each G1 is independently hydrogen, deuterium, a halogen group, a cyano group, a substituted or unsubstituted alkyl group, a substituted or unsubstituted cycloalkyl group, a substituted or unsubstituted alkoxy group, a substituted or unsubstituted alkenyl group, a substituted or unsubstituted silyl group, a substituted or unsubstituted aryl group, or a substituted or unsubstituted heterocyclic group;
g1 is 8;
G1's are the same as or different from each other;
in Chemical Formulae 1-2 to 1-6, X1 is O or S;
in Chemical Formula 1-1, X1 is S;
in Chemical Formulae 1-3 and 1-4, R2 is a substituted or unsubstituted alkyl group, a substituted or unsubstituted cycloalkyl group, a substituted or unsubstituted alkoxy group, a substituted or unsubstituted alkenyl group, a substituted or unsubstituted silyl group, a substituted or unsubstituted aryl group, or a substituted or unsubstituted heterocyclic group; and
Chemical Formulae 1-1 to 1-5 comprise deuterium, wherein the deuterium substitution rate is 40% to 100%; 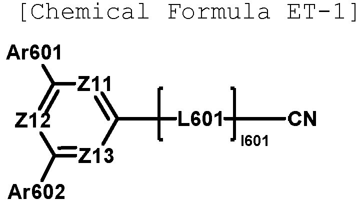
in Chemical Formula ET-1,
at least one of Z11 to Z13 is N, and the others are CH;
L601 is a direct bond, a substituted or unsubstituted arylene group, or a substituted or unsubstituted heteroarylene group;
Ar601 and Ar602 are the same as or different from each other, and are each independently a substituted or unsubstituted aryl group, or a substituted or unsubstituted heteroaryl group; and
l601 is an integer from 1 to 5, and when l601 is 2 or higher, the two or more L601s are the same as or different from each other.

3. The organic light emitting device of claim 2, wherein the light emitting layer comprises a fluorescent dopant which comprises one or more of a pyrene-based compound and a boron-based compound.

4. The organic light emitting device of claim 2, wherein the light emitting layer further comprises one or more hosts different from the compound of any one of Chemical Formula 1-1 to 1-5.

5. The organic light emitting device of claim 2, wherein
R1 to R6 are the same as or different from each other, and are each independently hydrogen, deuterium; a straight-chained or branched alkyl group having 1 to 30 carbon atoms, which is unsubstituted or substituted with deuterium or a monocyclic or polycyclic aryl group having 6 to 30 carbon atoms; a monocyclic or polycyclic cycloalkyl group having 3 to 30 carbon atoms, which is unsubstituted or substituted with deuterium; a straight-chained or branched alkylsilyl group having 1 to 30 carbon atoms; a monocyclic or polycyclic aryl group having 6 to 30 carbon atoms, which is unsubstituted or substituted with deuterium, a cyano group, a straight-chained or branched alkyl group having 1 to 30 carbon atoms, or a monocyclic or polycyclic heterocyclic group having 2 to 30 carbon atoms, which is unsubstituted or substituted with a monocyclic or polycyclic aryl group having 6 to 30 carbon atoms; or a monocyclic or polycyclic heteroaryl group having 2 to 30 carbon atoms, which is unsubstituted or substituted with deuterium;
L1 and L2 are the same as or different from each other, and are each independently a direct bond or a monocyclic or polycyclic arylene group having 6 to 30 carbon atoms, which is unsubstituted or substituted with deuterium,
G1 is hydrogen, deuterium, or a monocyclic or polycyclic aryl group having 6 to 30 carbon atoms, which is unsubstituted or substituted with deuterium;
Ar1 is a monocyclic or polycyclic aryl group having 6 to 30 carbon atoms, which is unsubstituted or substituted with deuterium; and
in Chemical Formulae 1-3 and 1-4, R2 is a straight-chained or branched alkyl group having 1 to 30 carbon atoms, which is unsubstituted or substituted with deuterium or a monocyclic or polycyclic aryl group having 6 to 30 carbon atoms; a monocyclic or polycyclic cycloalkyl group having 3 to 30 carbon atoms, which is unsubstituted or substituted with deuterium; a straight-chained or branched alkylsilyl group having 1 to 30 carbon atoms; a monocyclic or polycyclic aryl group having 6 to 30 carbon atoms, which is unsubstituted or substituted with deuterium, a cyano group, or a straight-chained or branched alkyl group having 1 to 30 carbon atoms; or a monocyclic or polycyclic heteroaryl group having 2 to 30 carbon atoms, which is unsubstituted or substituted with deuterium.

6. The organic light emitting device of claim 2, wherein the compound of any one of Chemical Formulae 1-1 to 1-5 is selected from the following compounds:

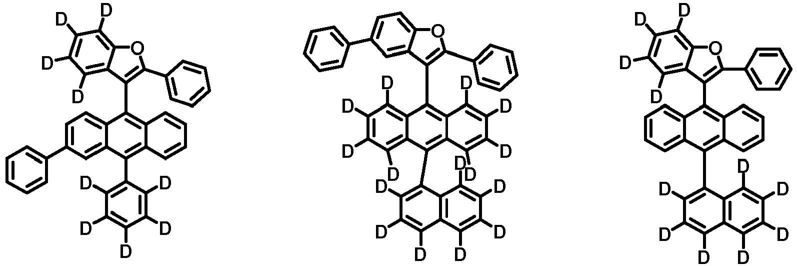

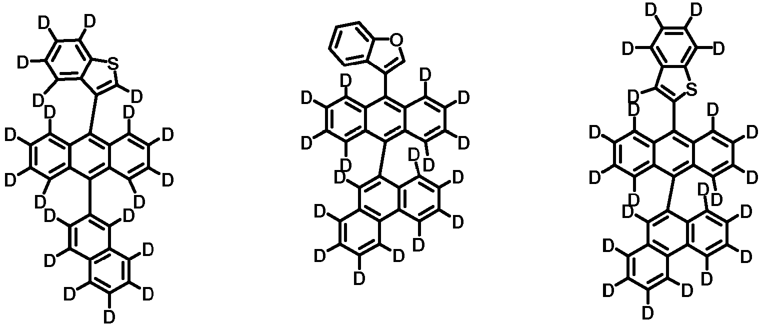

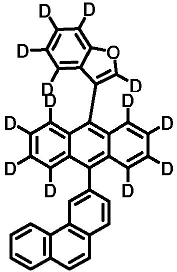

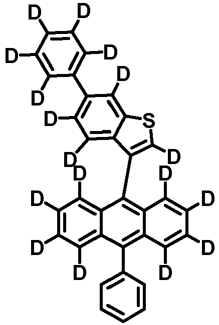

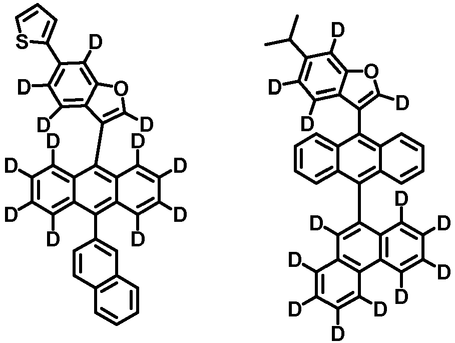

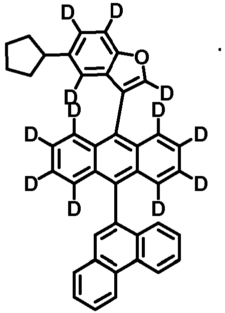

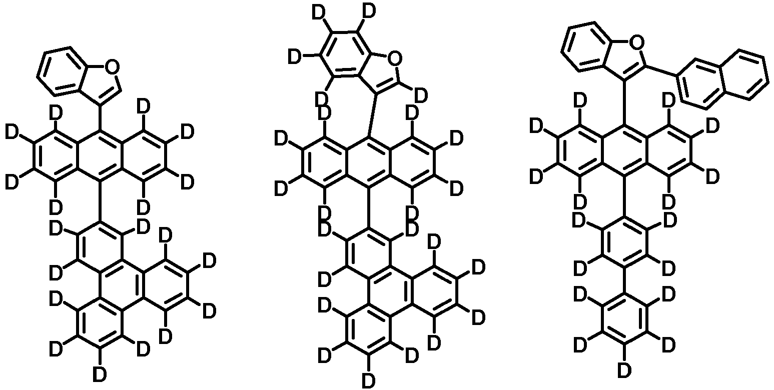

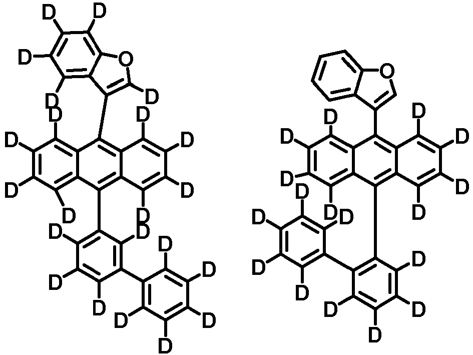

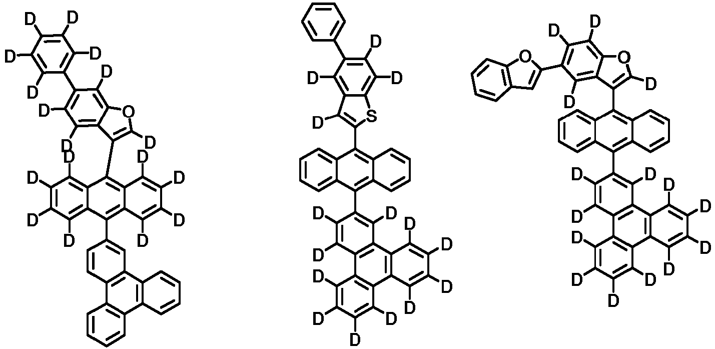

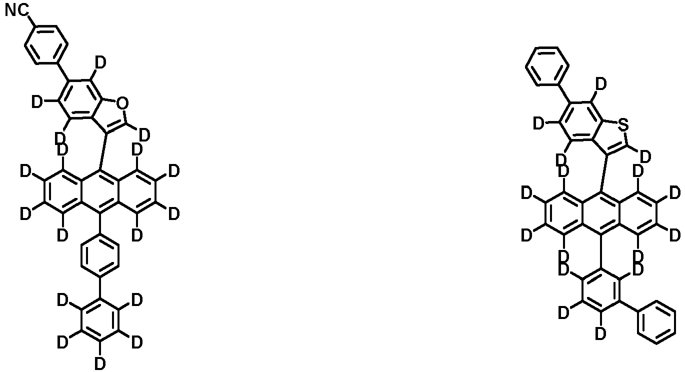

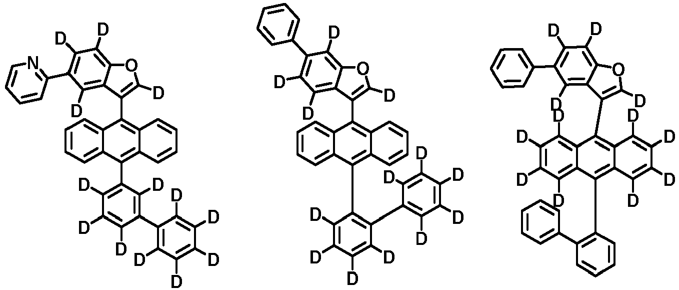

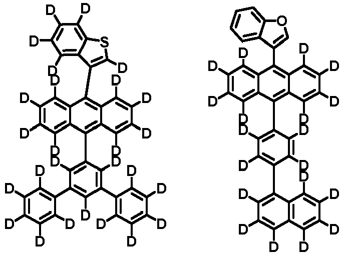

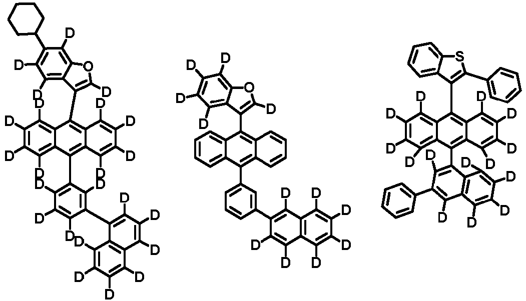

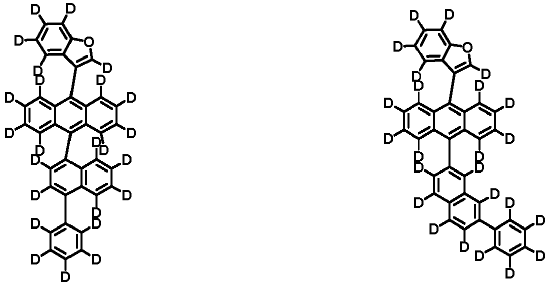

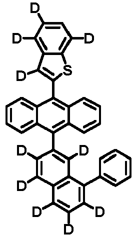

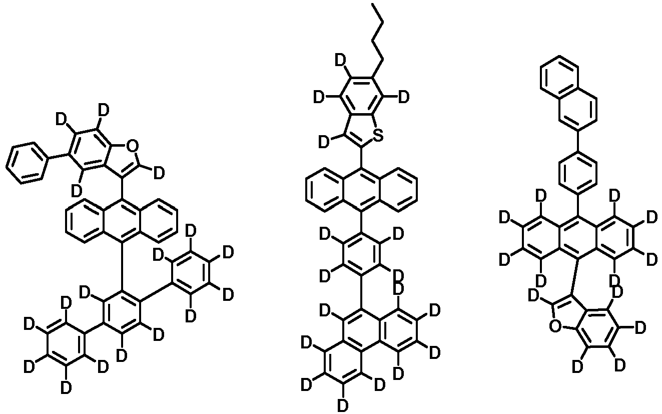

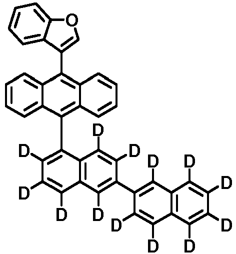

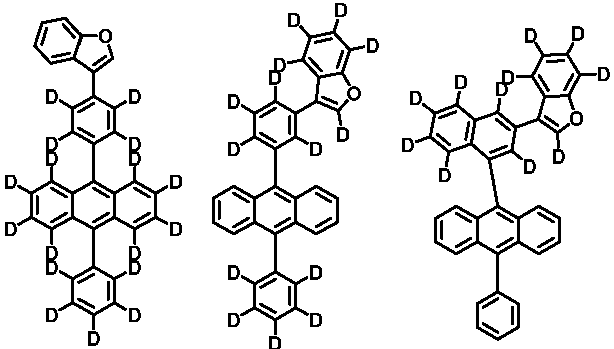

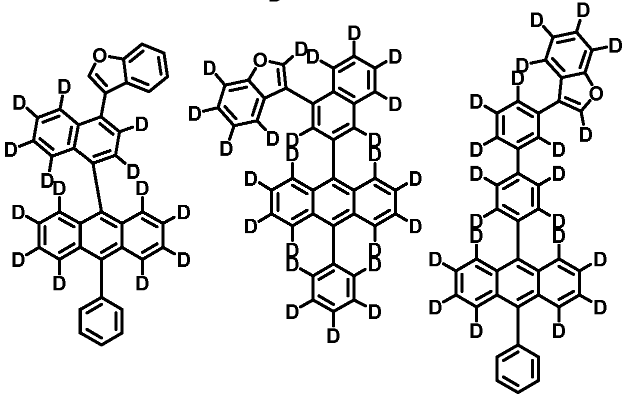

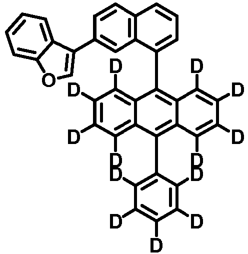

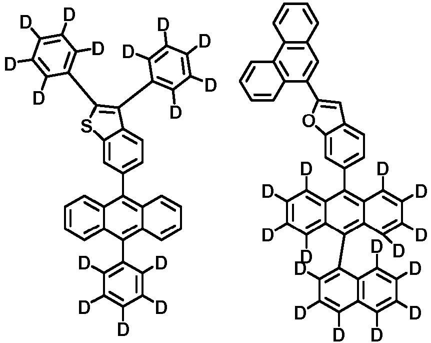

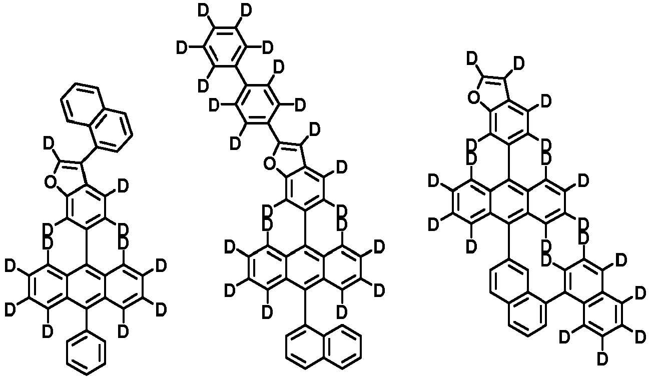

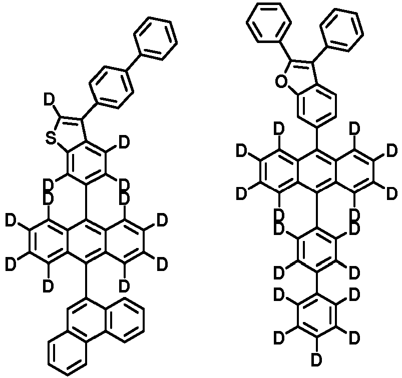

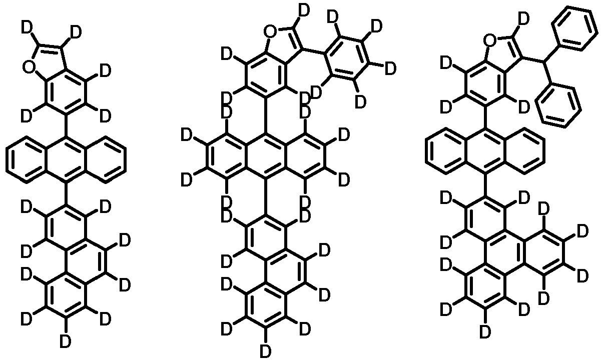

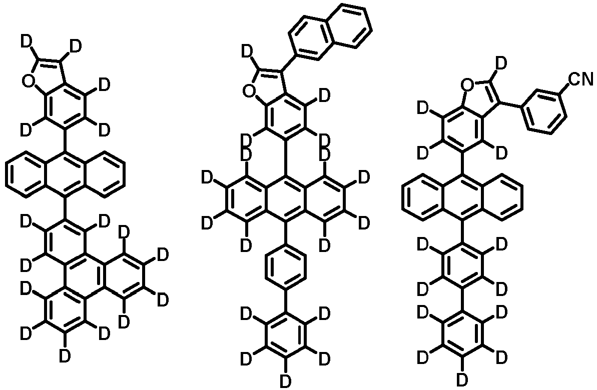

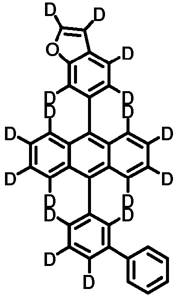

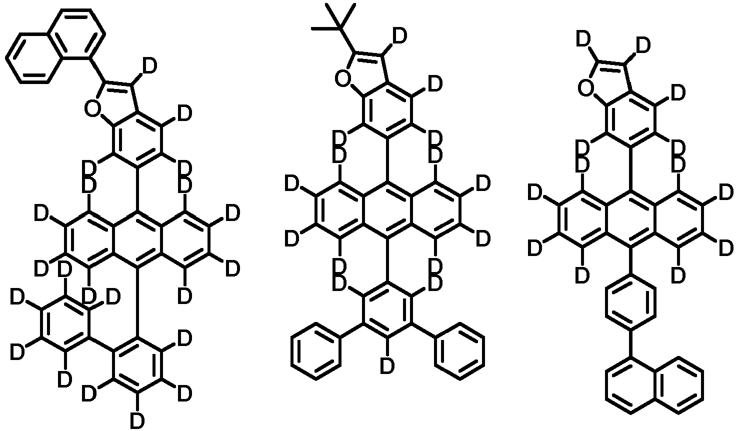

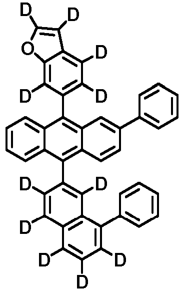

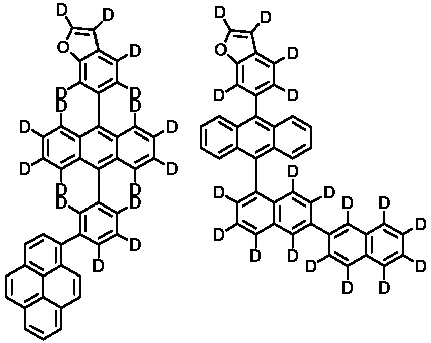

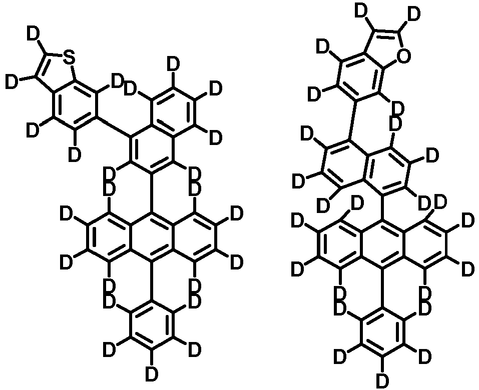

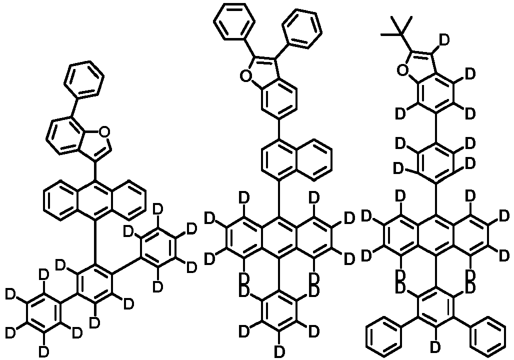

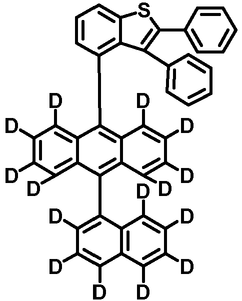

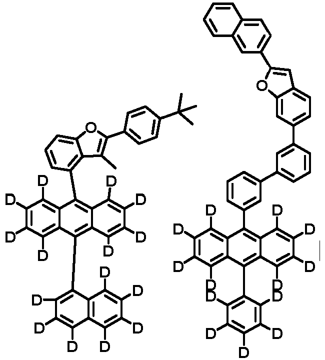

## Patentansprüche

1. Verbindung, ausgewählt aus den Folgenden:

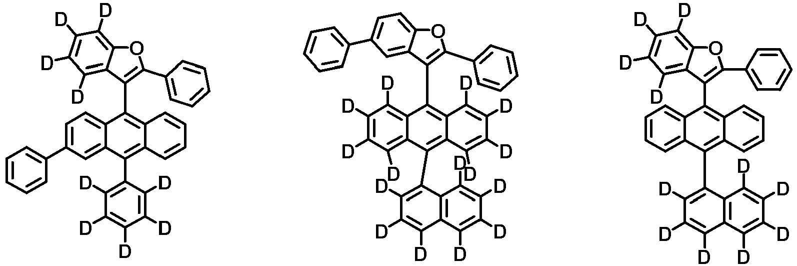

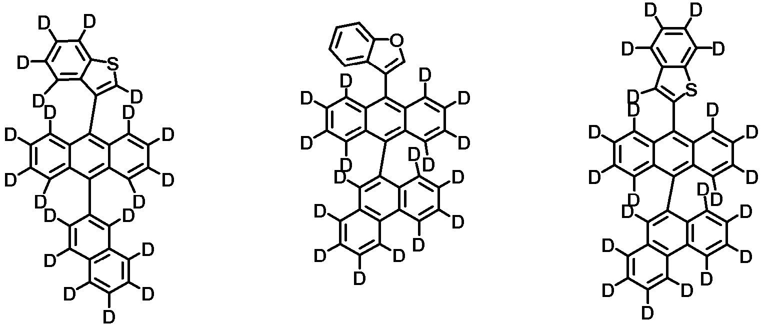

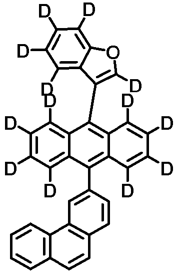

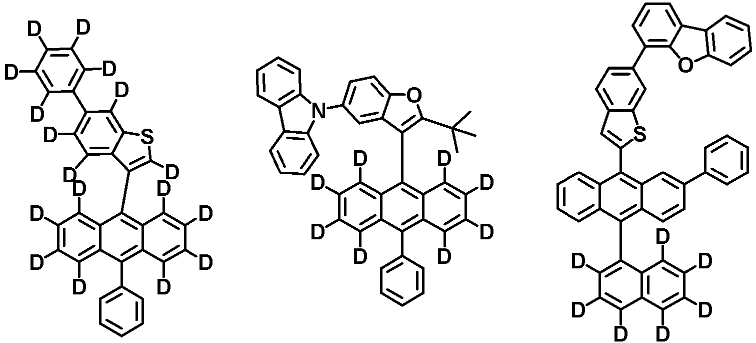

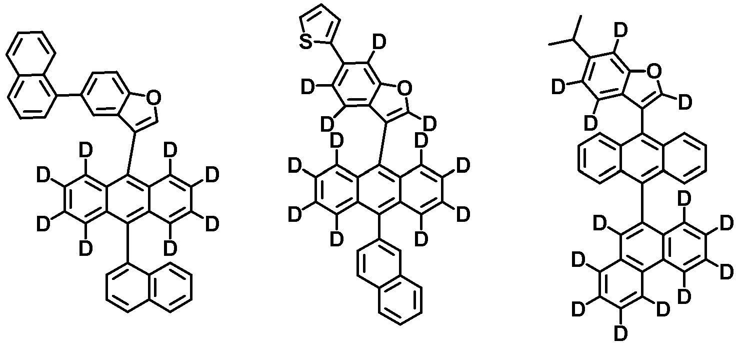

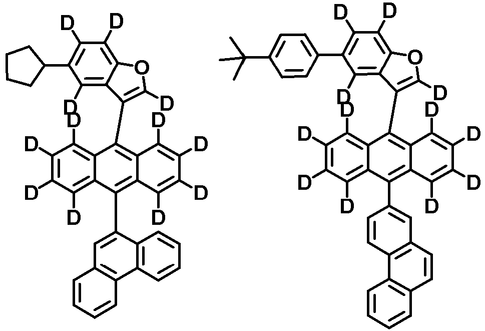

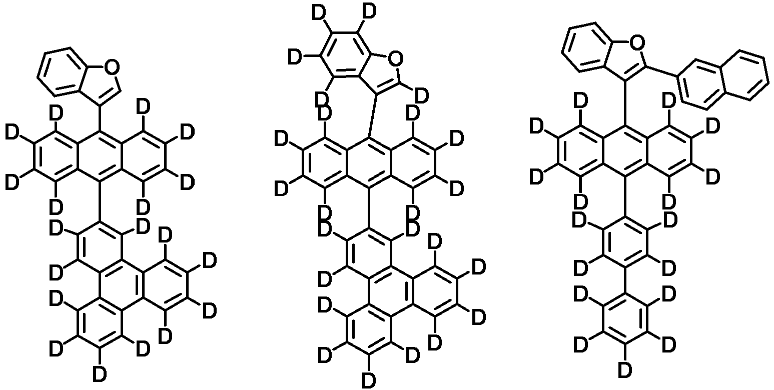

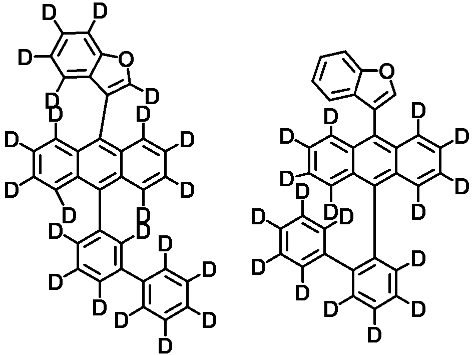

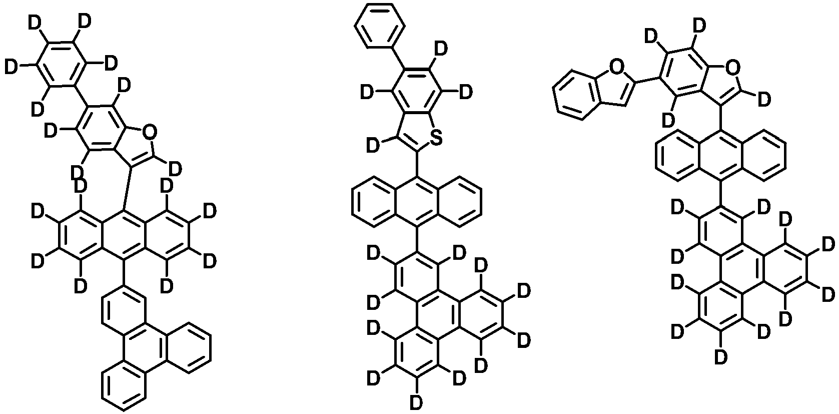

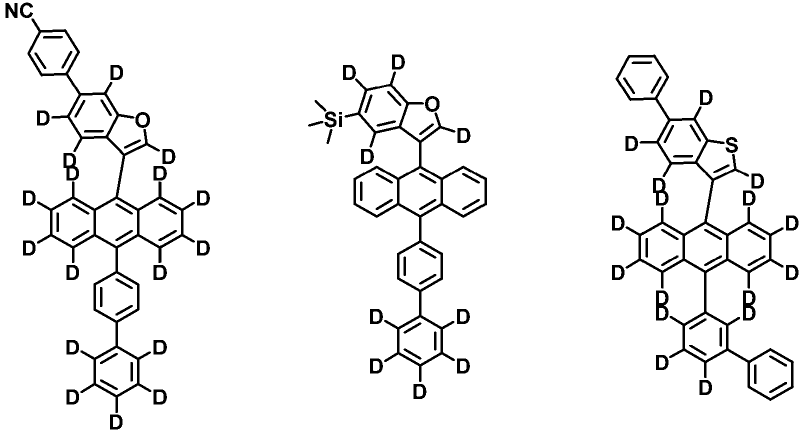

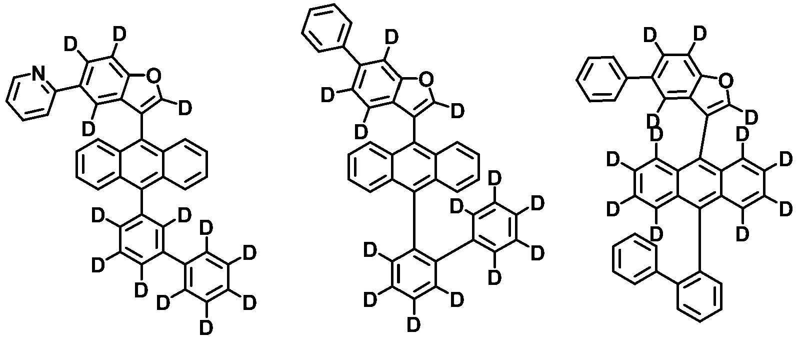

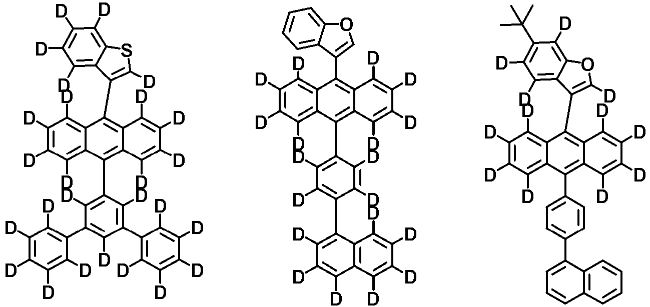

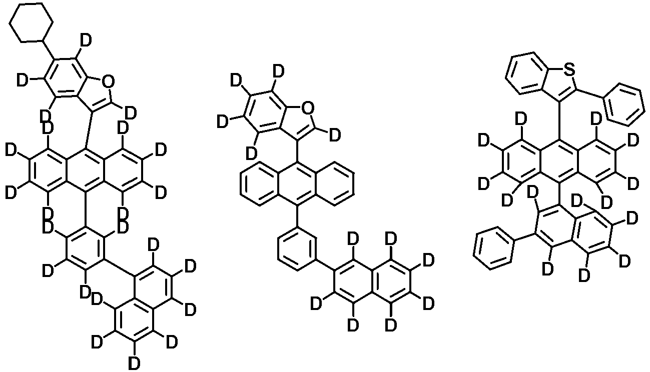

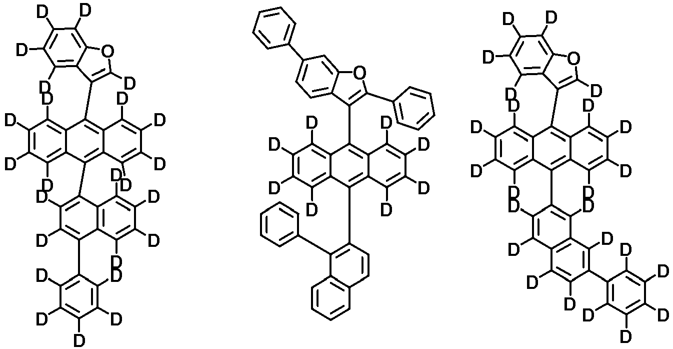

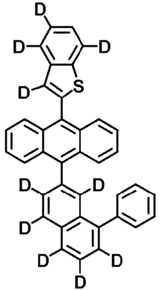

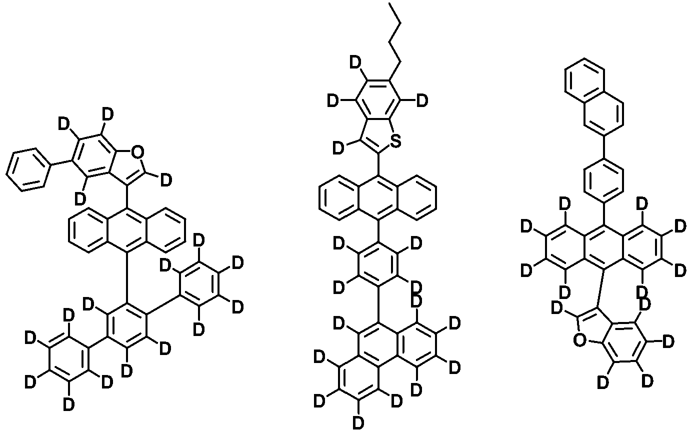

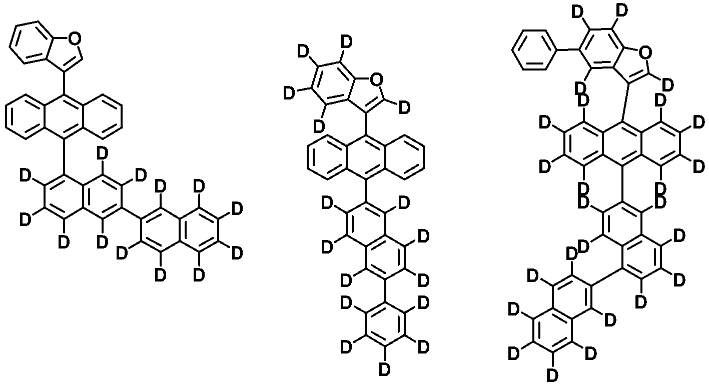

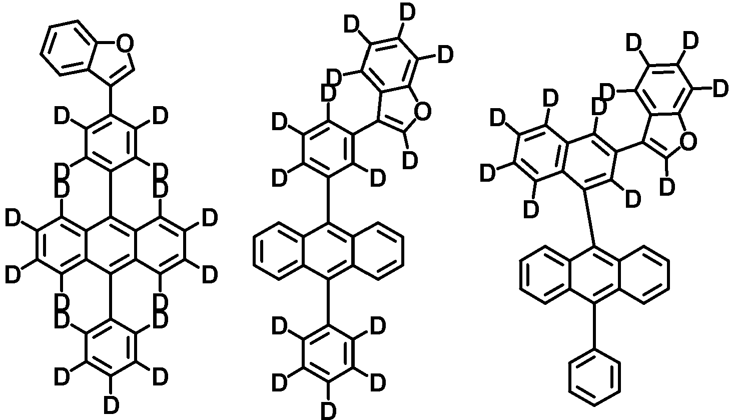

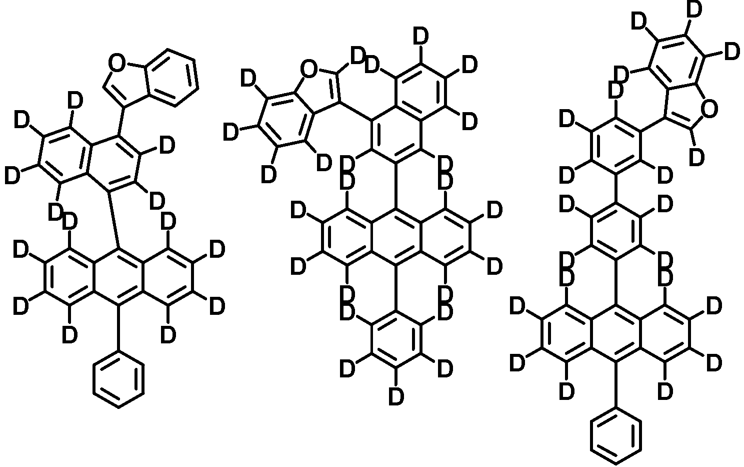

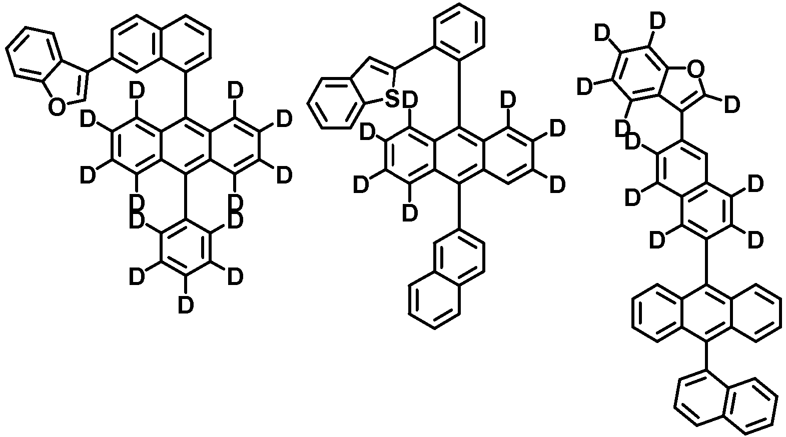

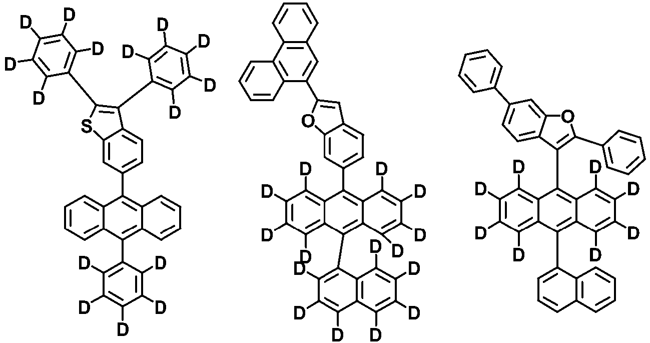

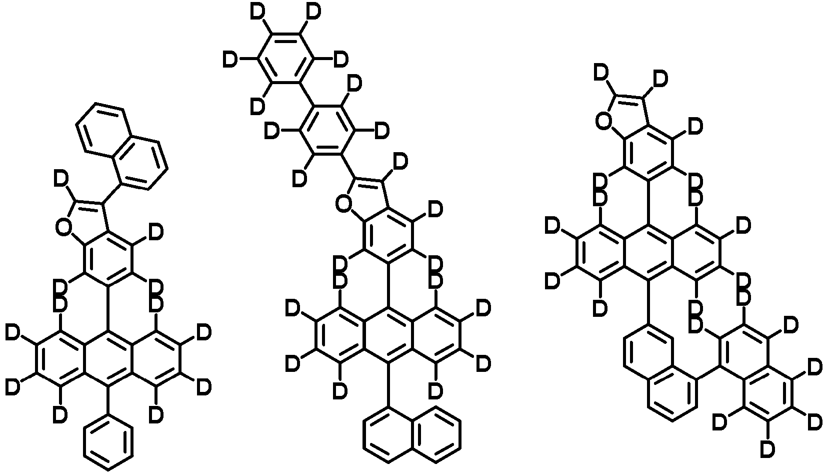

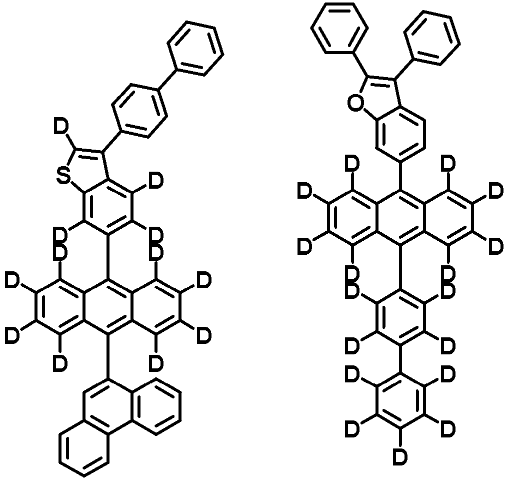

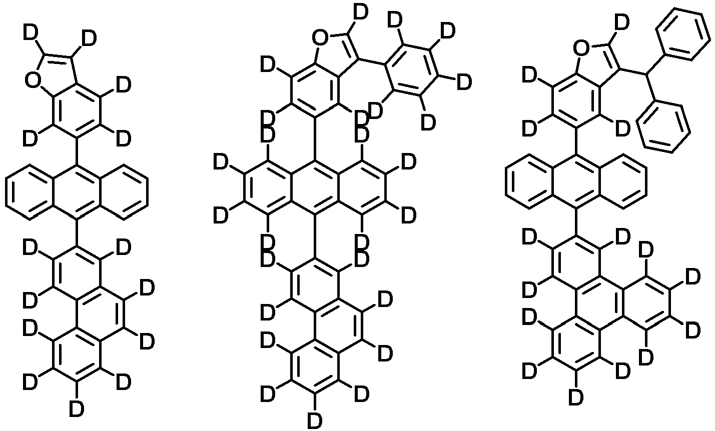

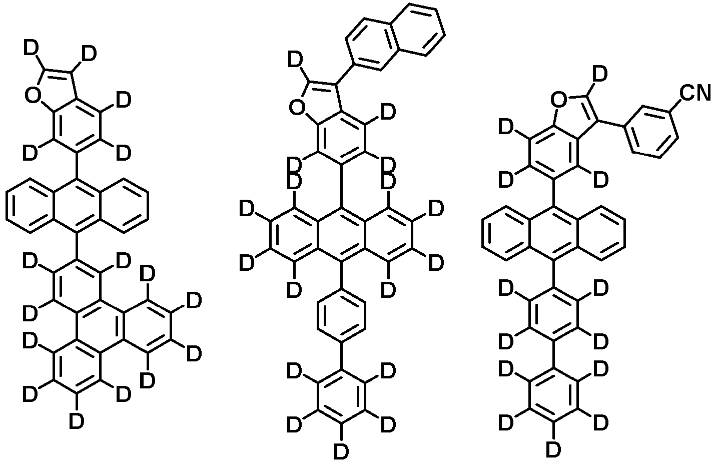

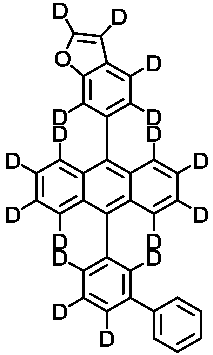

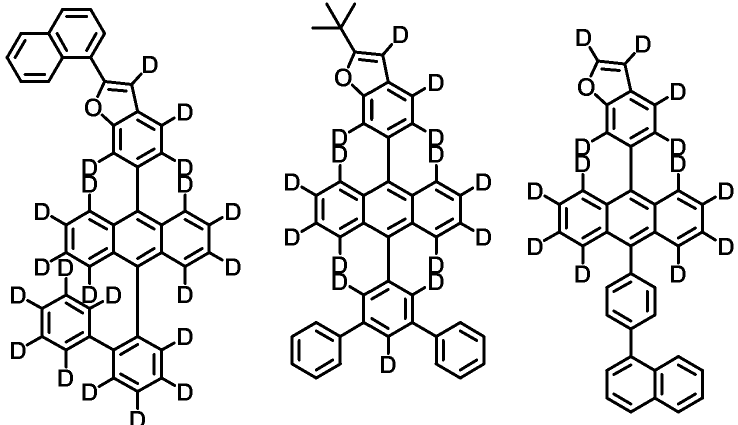

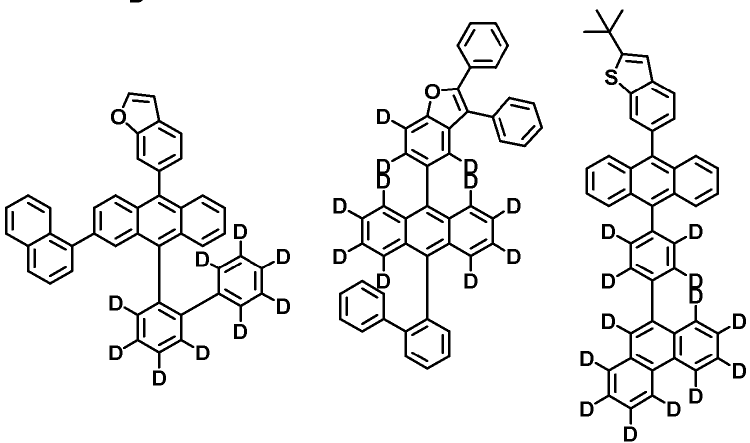

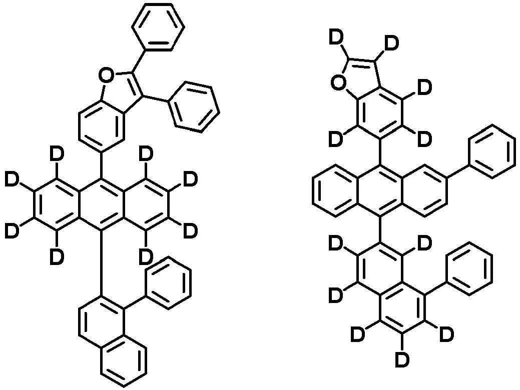

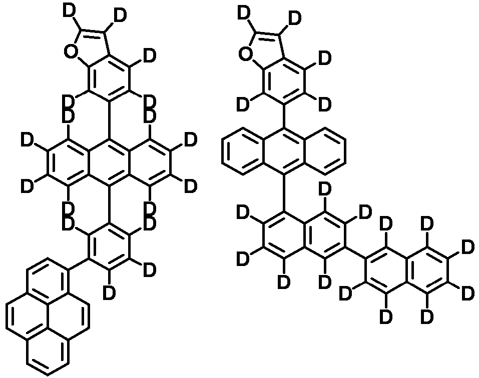

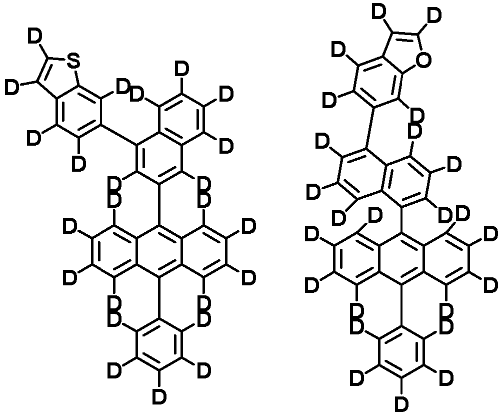

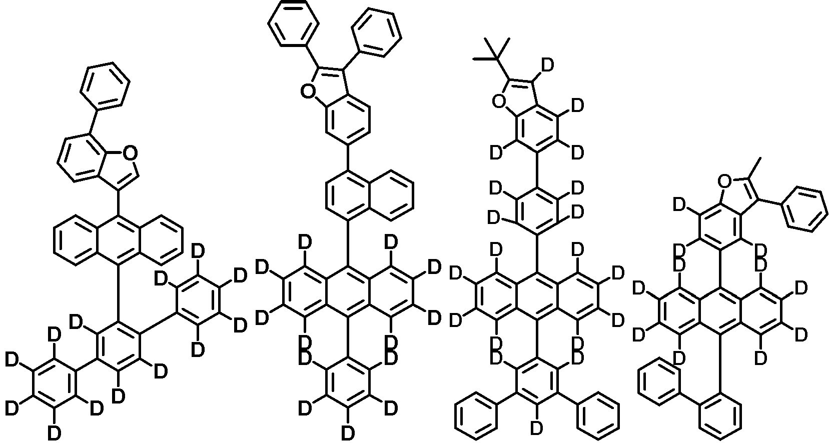

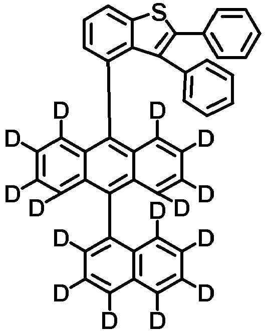

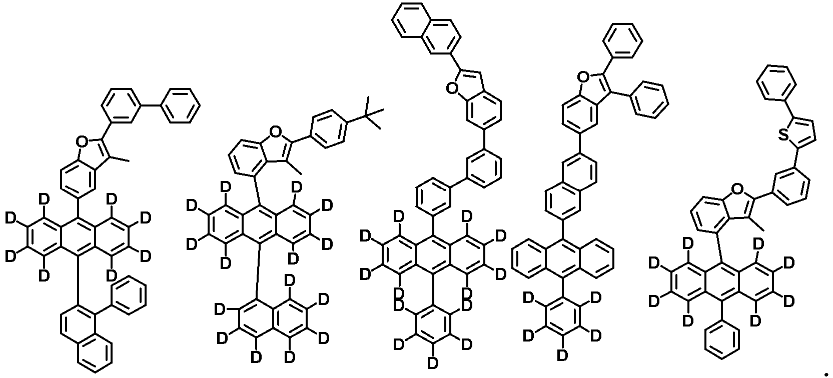

2. Organische lichtemittierende Vorrichtung, umfassend eine erste Elektrode, eine zweite Elektrode und eine oder mehrere organische Materialschichten, die zwischen der ersten Elektrode und der zweiten Elektrode angeordnet sind,
wobei die eine oder mehreren organischen Materialschichten eine lichtemittierende Schicht und eine Elektronentransportschicht umfassen,
die lichtemittierende Schicht eine Verbindung einer der folgenden chemischen Formeln 1-1 bis 1-5 als eine Wirtsverbindung umfasst und
die Elektronentransportschicht eine Verbindung der folgenden chemischen Formel ET-1 umfasst: 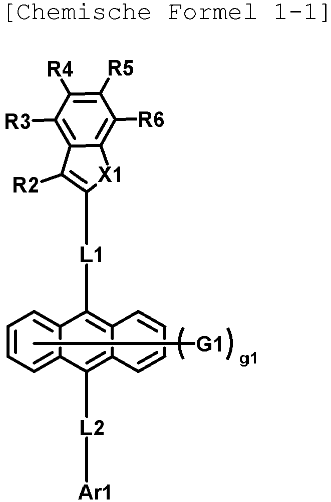 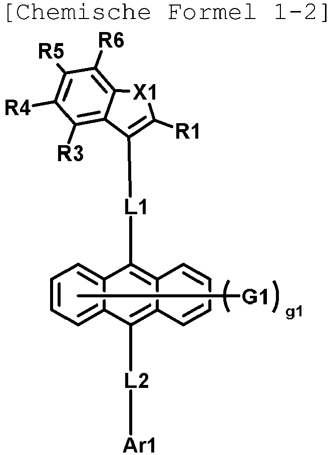 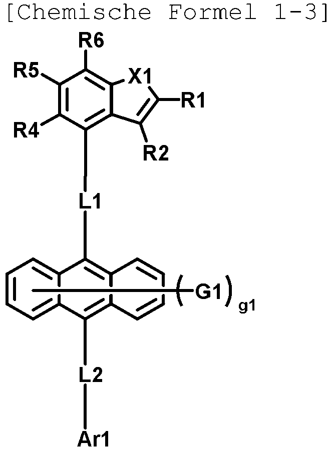 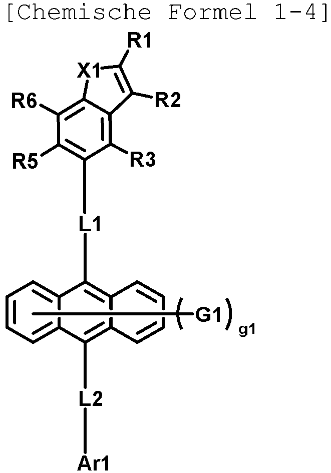 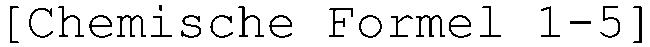 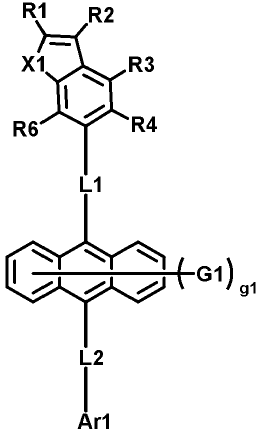
in den chemischen Formeln 1-1 bis 1-5
R1 bis R6 gleich sind oder sich voneinander unterscheiden und jeweils unabhängig Wasserstoff, Deuterium, eine Halogengruppe, eine Cyanogruppe, eine substituierte oder unsubstituierte Alkylgruppe, eine substituierte oder unsubstituierte Cycloalkylgruppe, eine substituierte oder unsubstituierte Alkoxygruppe, eine substituierte oder unsubstituierte Alkenylgruppe, eine substituierte oder unsubstituierte Silylgruppe, eine substituierte oder unsubstituierte Arylgruppe oder eine substituierte oder unsubstituierte heterocyclische Gruppe sind,
L1 und L2 gleich sind oder sich voneinander unterscheiden und jeweils unabhängig eine direkte Bindung, eine substituierte oder unsubstituierte Arylengruppe oder eine substituierte oder unsubstituierte zweiwertige heterocyclische Gruppe sind, Ar1 eine substituierte oder unsubstituierte Arylgruppe ist,
jedes G1 unabhängig Wasserstoff, Deuterium, eine Halogengruppe, eine Cyanogruppe, eine substituierte oder unsubstituierte Alkylgruppe, eine substituierte oder unsubstituierte Cycloalkylgruppe, eine substituierte oder unsubstituierte Alkoxygruppe, eine substituierte oder unsubstituierte Alkenylgruppe, eine substituierte oder unsubstituierte Silylgruppe, eine substituierte oder unsubstituierte Arylgruppe oder eine substituierte oder unsubstituierte heterocyclische Gruppe ist,
g1 8 ist,
die G1 gleich sind oder sich voneinander unterscheiden,
in den chemischen Formeln 1-2 bis 1-6 X1 O oder S ist,
in der chemischen Formel 1-1 X1 S ist,
in den chemischen Formeln 1-3 und 1-4 R2 eine substituierte oder unsubstituierte Alkylgruppe, eine substituierte oder unsubstituierte Cycloalkylgruppe, eine substituierte oder unsubstituierte Alkoxygruppe eine substituierte oder unsubstituierte Alkenylgruppe, eine substituierte oder unsubstituierte Silylgruppe, eine substituierte oder unsubstituierte Arylgruppe oder eine substituierte oder unsubstituierte heterocyclische Gruppe ist und
die chemischen Formeln 1-1 bis 1-5 Deuterium umfassen, wobei die Deuteriumssubstitutionsrate 40 % bis 100 % beträgt, 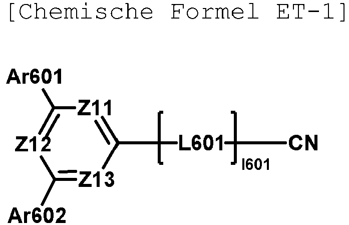
in der chemischen Formel ET-1
mindestens eines von Z11 bis Z13 N ist und die anderen CH sind,
L601 eine direkte Bindung, eine substituierte oder unsubstituierte Arylengruppe oder eine substituierte oder unsubstituierte Heteroarylengruppe ist,
Ar601 und Ar602 gleich sind oder sich voneinander unterscheiden und jeweils unabhängig eine substituierte oder unsubstituierte Arylgruppe oder eine substituierte oder unsubstituierte Heteroarylgruppe sind und
l601 eine ganze Zahl von 1 bis 5 ist, und wenn l601 2 oder größer ist, die zwei oder mehr L601 gleich sind oder sich voneinander unterscheiden.

3. Organische lichtemittierende Vorrichtung nach Anspruch 2, wobei die lichtemittierende Schicht ein fluoreszierendes Dotierungsmittel umfasst, das eine oder mehrere von einer pyrenbasierten Verbindung und einer borbasierten Verbindung umfasst.

4. Organische lichtemittierende Vorrichtung nach Anspruch 2, wobei die lichtemittierende Schicht ferner einen oder mehrere Wirte umfasst, die sich von der Verbindung einer der chemischen Formeln 1-1 bis 1-5 unterscheiden.

5. Organische lichtemittierende Vorrichtung nach Anspruch 2, wobei
R1 bis R6 gleich sind oder sich voneinander unterscheiden und jeweils unabhängig Wasserstoff, Deuterium, eine geradkettige oder verzweigte Alkylgruppe mit 1 bis 30 Kohlenstoffatomen, die unsubstituiert oder mit Deuterium oder einer monocyclischen oder polycyclischen Arylgruppe mit 6 bis 30 Kohlenstoffatomen substituiert ist, eine monocyclische oder polycyclische Cycloalkylgruppe mit 3 bis 30 Kohlenstoffatomen, die unsubstituiert oder mit Deuterium substituiert ist, eine geradkettige oder verzweigte Alkylsilylgruppe mit 1 bis 30 Kohlenstoffatomen, eine monocyclische oder polycyclische Arylgruppe mit 6 bis 30 Kohlenstoffatomen, die unsubstituiert oder mit Deuterium substituiert ist oder mit Deuterium, einer Cyanogruppe, einer geradkettigen oder verzweigten Alkylgruppe mit 1 bis 30 Kohlenstoffatomen oder einer monocyclischen oder polycyclischen heterocyclischen Gruppe mit 2 bis 30 Kohlenstoffatomen substituiert ist, die unsubstituiert oder mit einer monocyclischen oder polycyclischen Arylgruppe mit 6 bis 30 Kohlenstoffatomen substituiert ist, oder eine monocyclische oder polycyclische Heteroarylgruppe mit 2 bis 30 Kohlenstoffatomen, die unsubstituiert oder mit Deuterium substituiert ist, sind,
L1 und L2 gleich sind oder sich voneinander unterscheiden und jeweils unabhängig eine direkte Bindung oder eine monocyclische oder polycyclische Arylengruppe mit 6 bis 30 Kohlenstoffatomen, die unsubstituiert oder mit Deuterium substituiert ist, sind,
G1 Wasserstoff, Deuterium oder eine monocyclische oder polycyclische Arylgruppe mit 6 bis 30 Kohlenstoffatomen,
die unsubstituiert oder mit Deuterium substituiert ist, ist,
Ar1 eine monocyclische oder polycyclische Arylgruppe mit 6 bis 30 Kohlenstoffatomen, die unsubstituiert oder mit Deuterium substituiert ist, ist, und
in den chemischen Formeln 1-3 und 1-4 R2 eine geradkettige oder verzweigte Alkylgruppe mit 1 bis 30 Kohlenstoffatomen, die unsubstituiert oder mit Deuterium oder einer monocyclischen oder polycyclischen Arylgruppe mit 6 bis 30 Kohlenstoffatomen substituiert ist, eine monocyclische oder polycyclische Cycloalkylgruppe mit 3 bis 30 Kohlenstoffatomen, die unsubstituiert oder mit Deuterium substituiert ist, eine geradkettige oder verzweigte Alkylsilylgruppe mit 1 bis 30 Kohlenstoffatomen, eine monocyclische oder polycyclische Arylgruppe mit 6 bis 30 Kohlenstoffatomen, die unsubstituiert oder mit Deuterium, einer Cyanogruppe oder einer geradkettigen oder verzweigten Alkylgruppe mit 1 bis 30 Kohlenstoffatomen substituiert ist, oder eine monocyclische oder polycyclische Heteroarylgruppe mit 2 bis 30 Kohlenstoffatomen, die unsubstituiert oder mit Deuterium substituiert ist, ist.

6. Organische lichtemittierende Vorrichtung nach Anspruch 2, wobei die Verbindung einer der chemischen Formeln 1-1 bis 1-5 aus den folgenden Verbindungen ausgewählt ist:

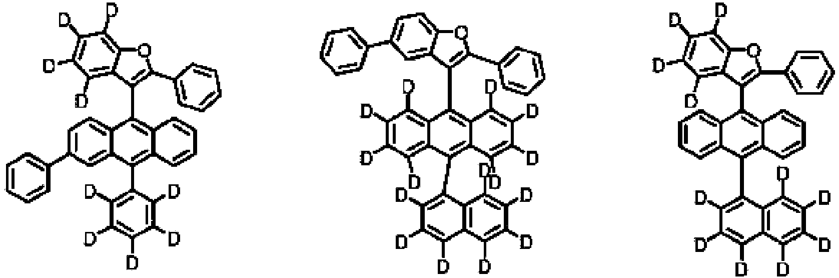

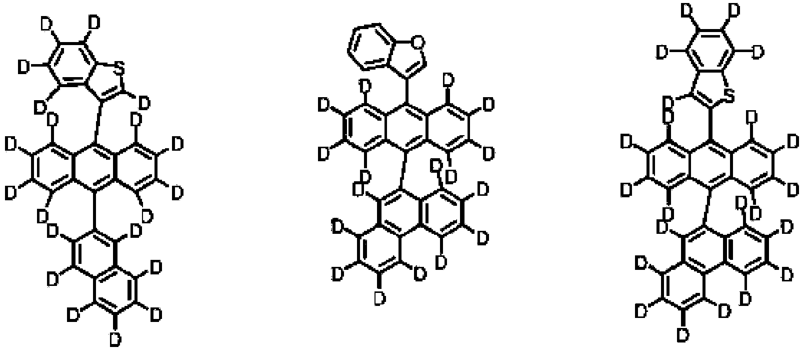

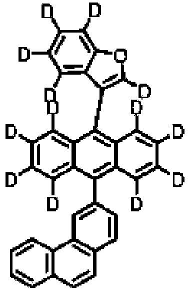

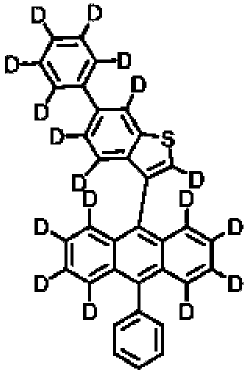

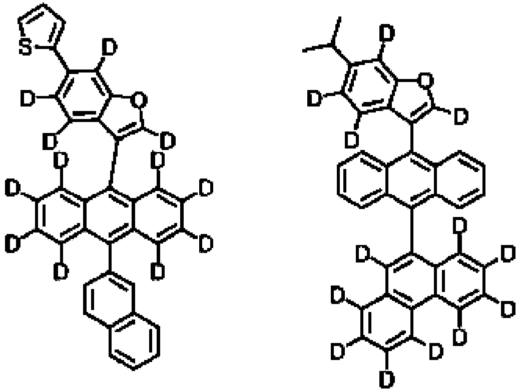

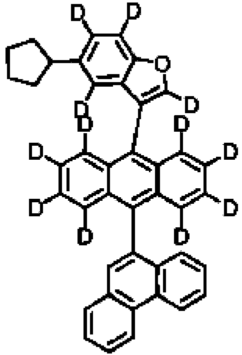

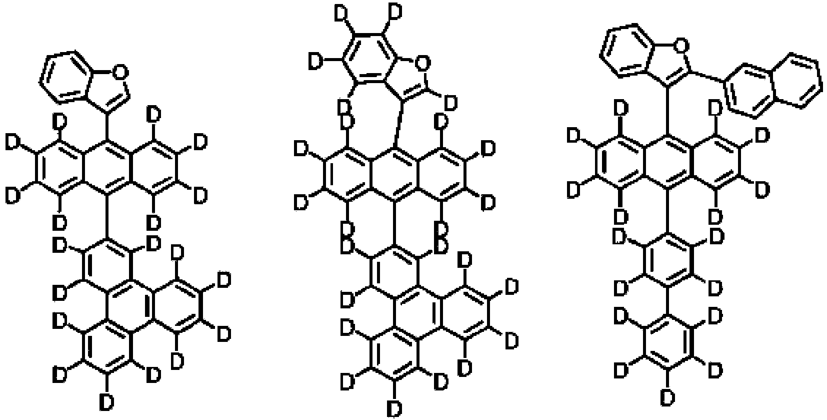

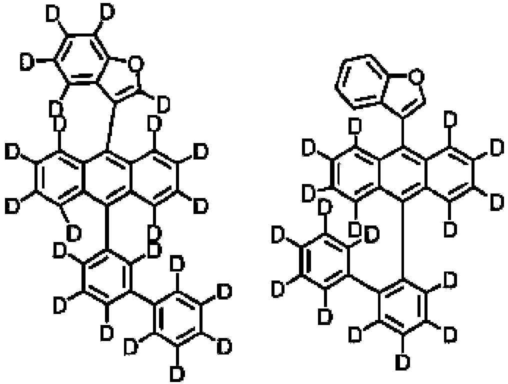

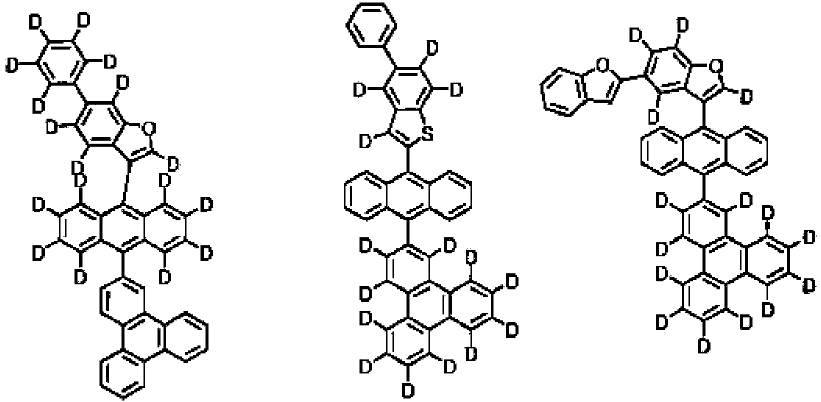

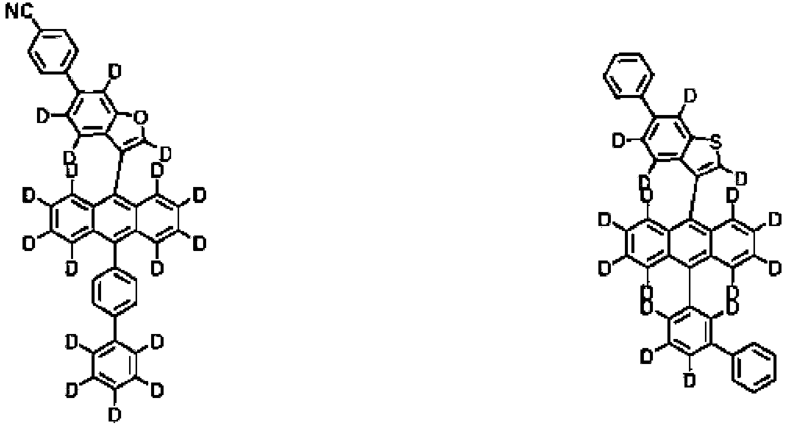

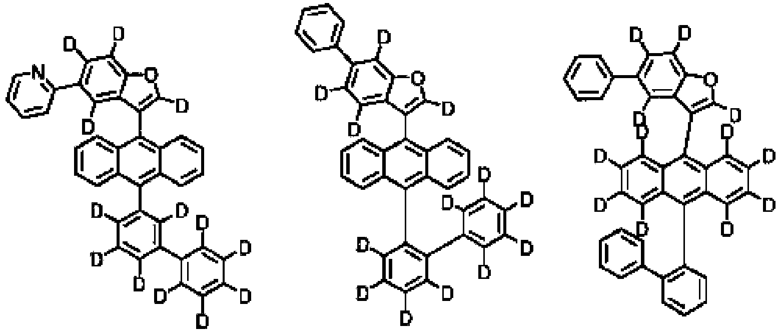

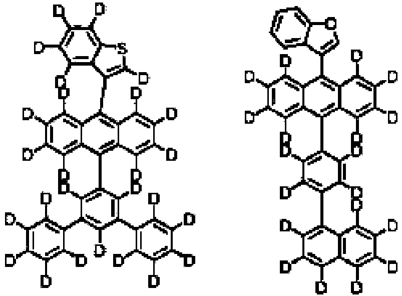

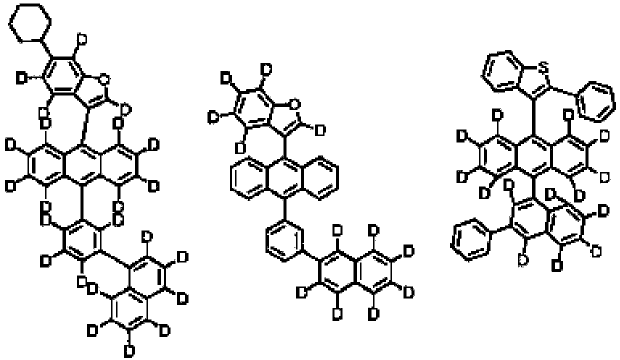

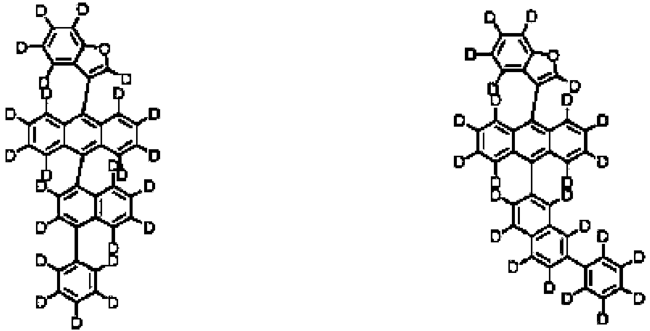

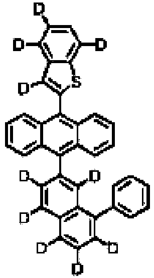

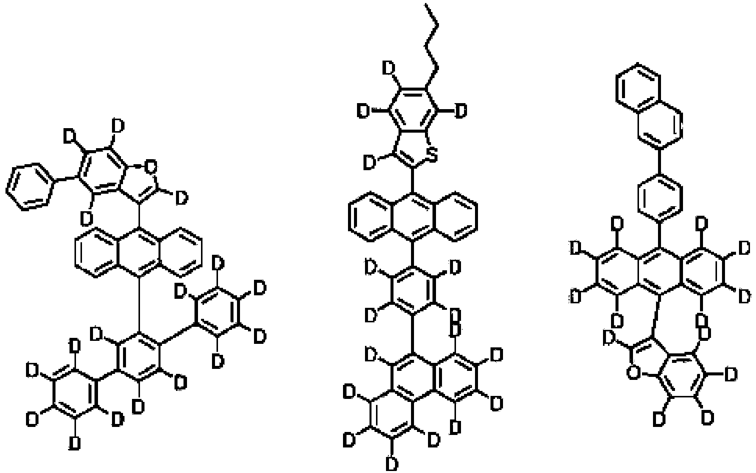

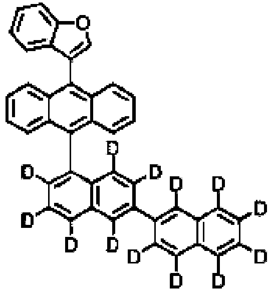

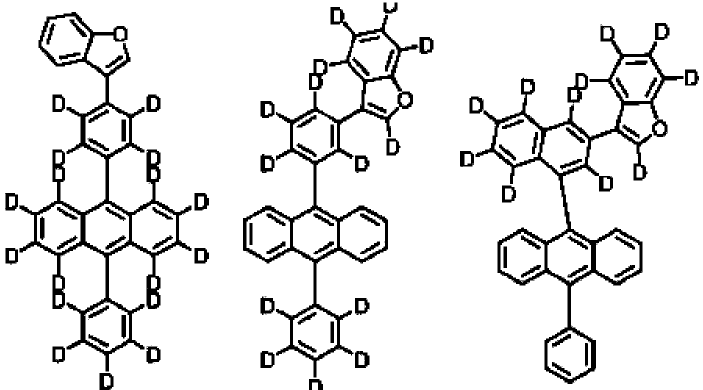

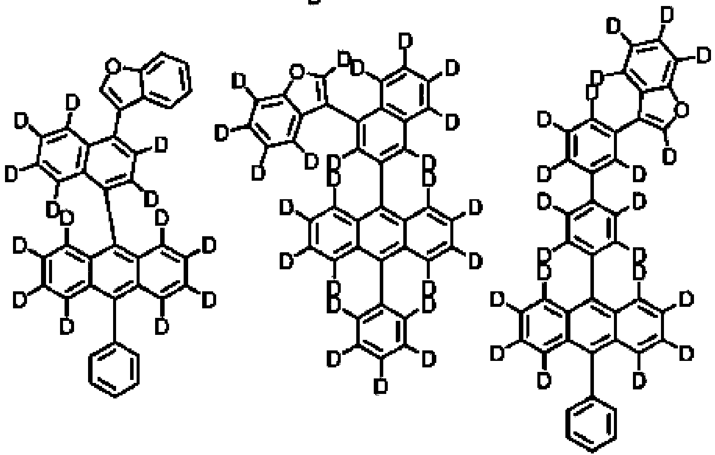

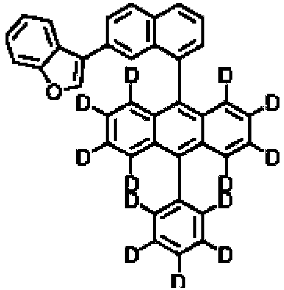

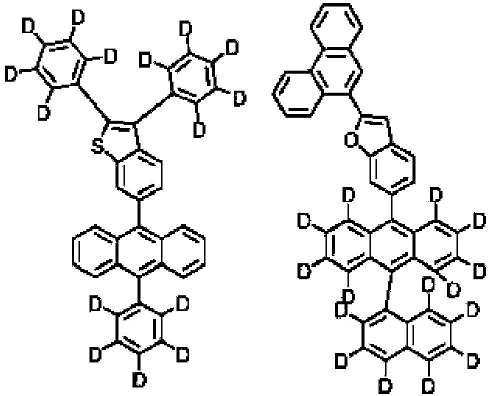

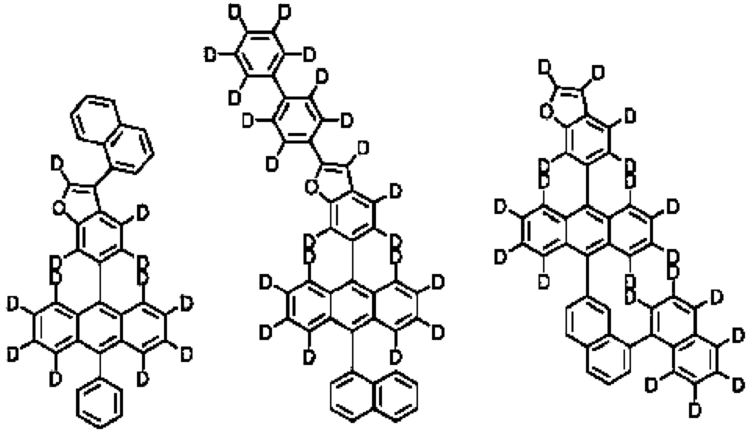

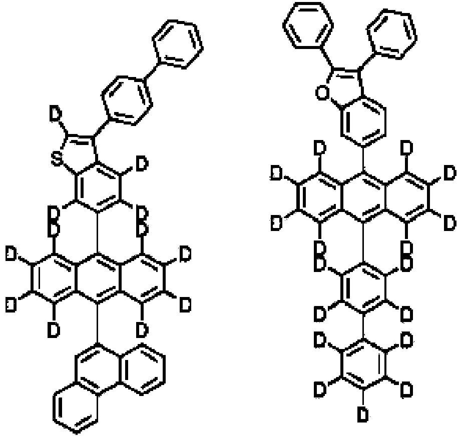

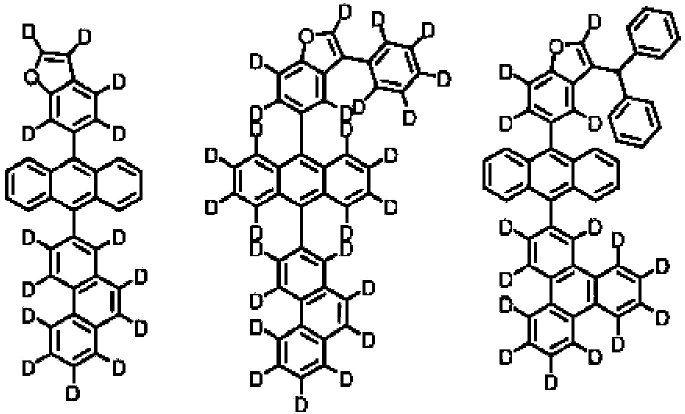

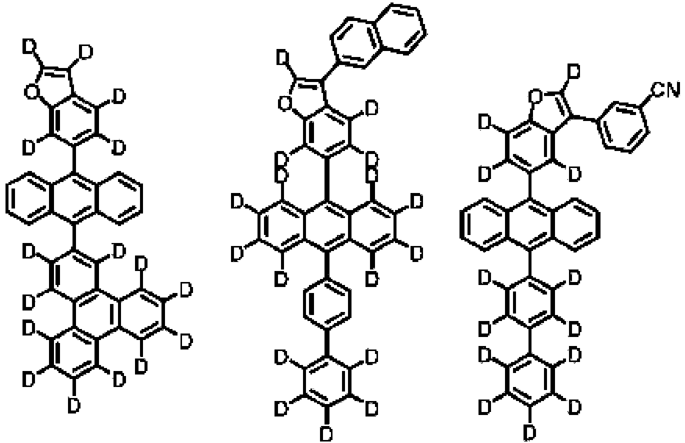

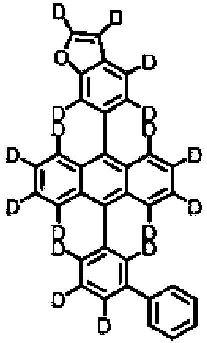

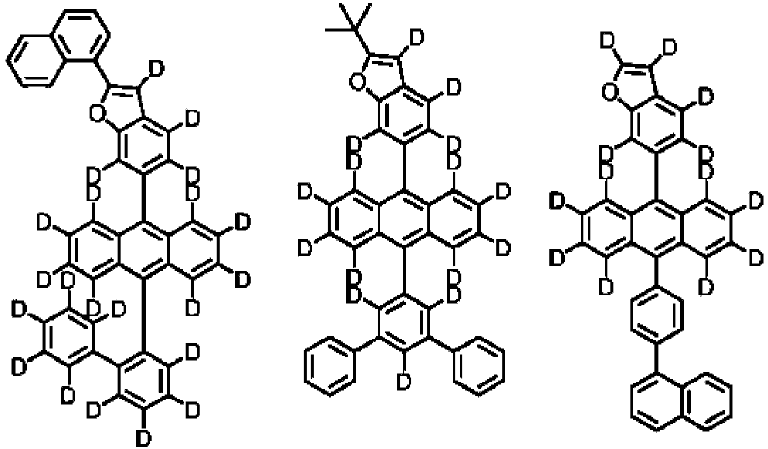

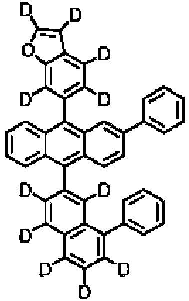

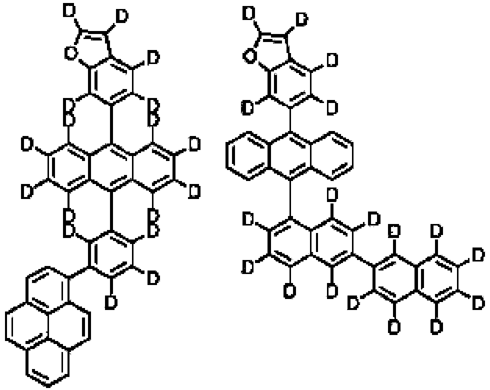

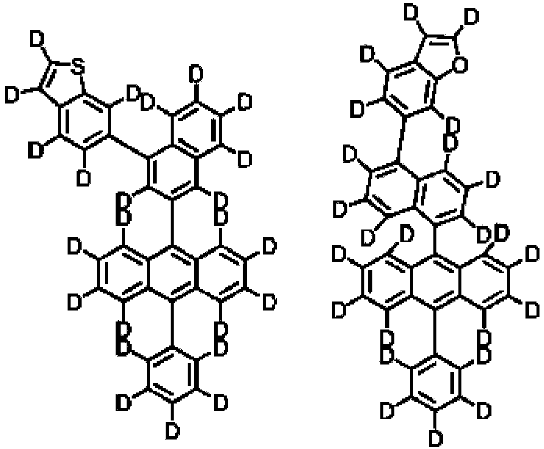

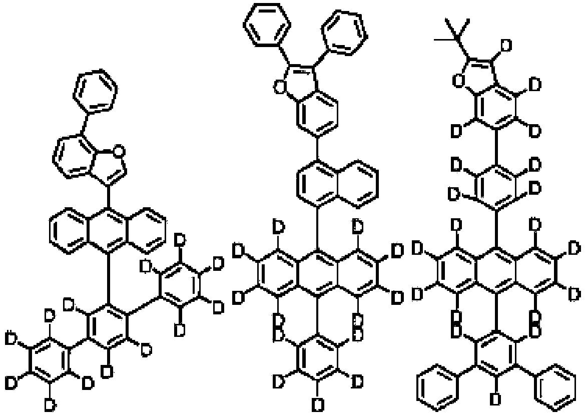

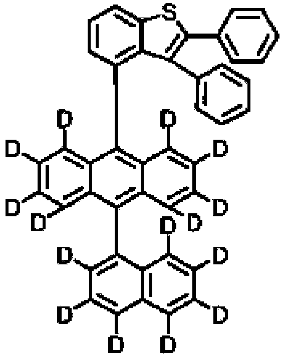

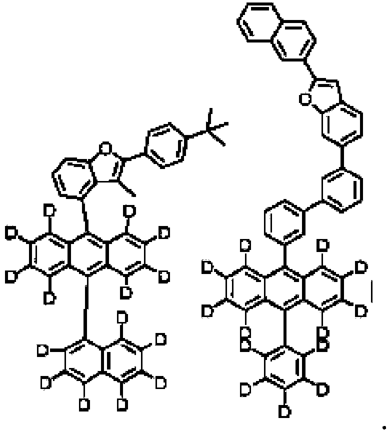

## Revendications

1. Composé sélectionné parmi les suivants :

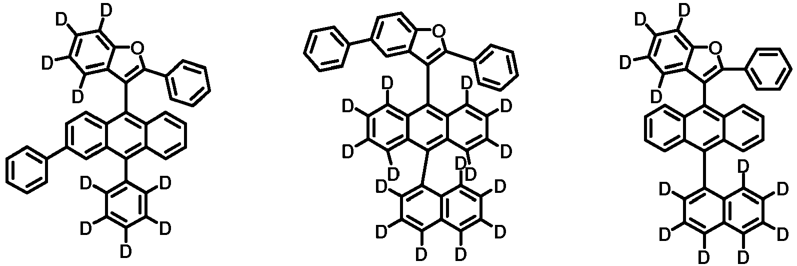

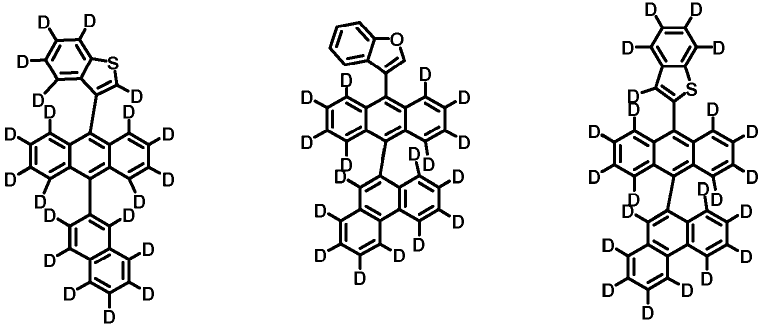

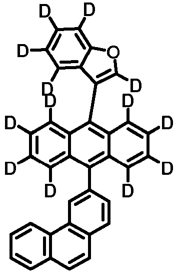

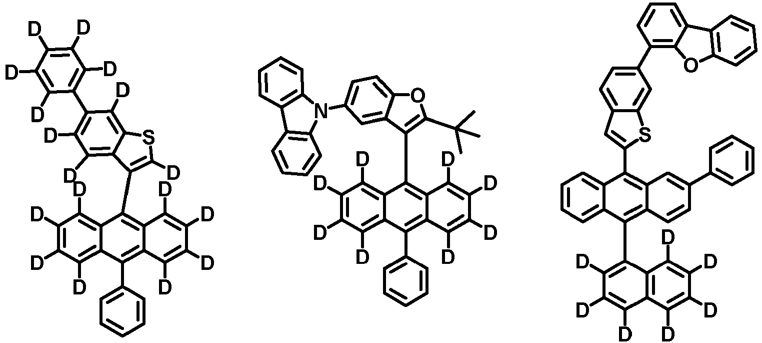

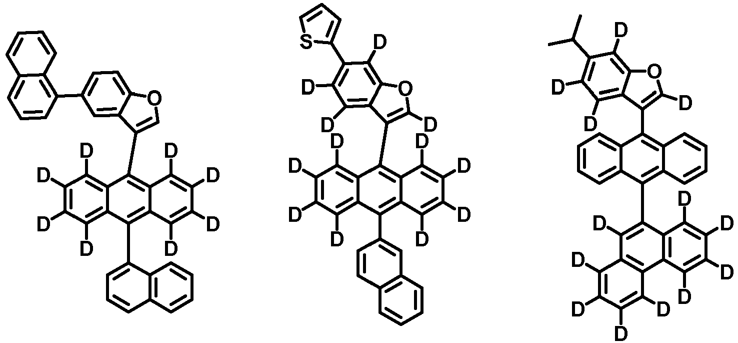

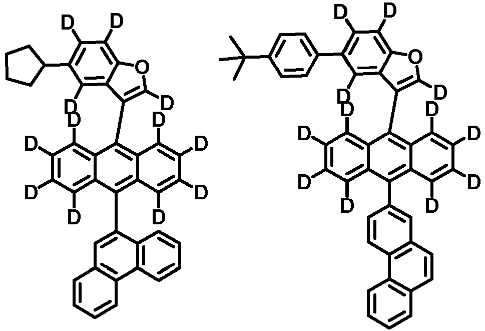

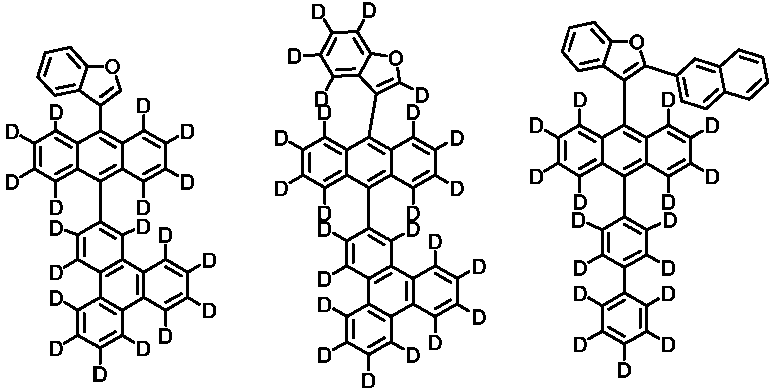

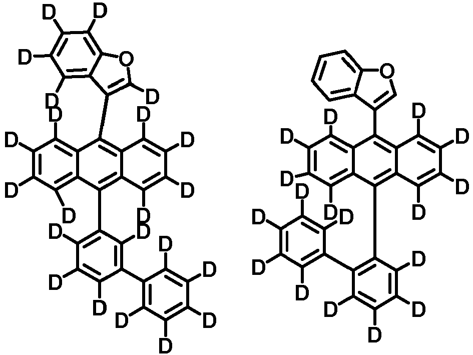

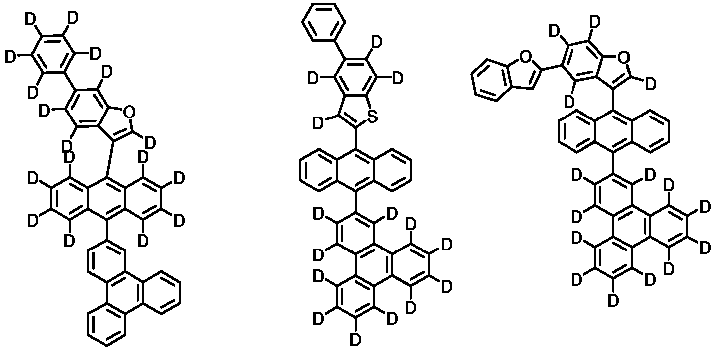

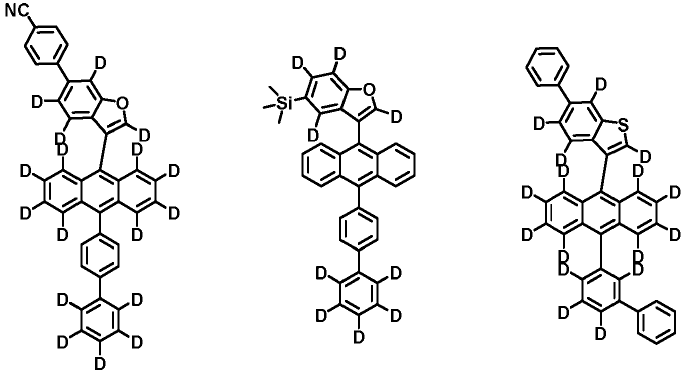

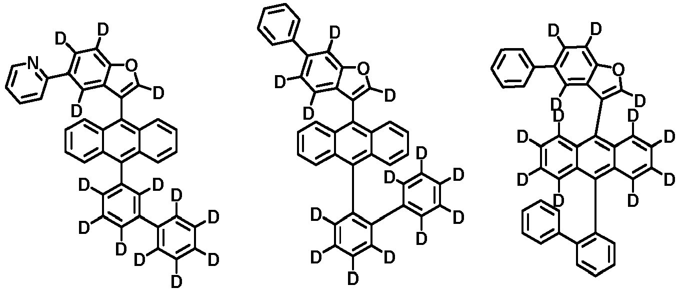

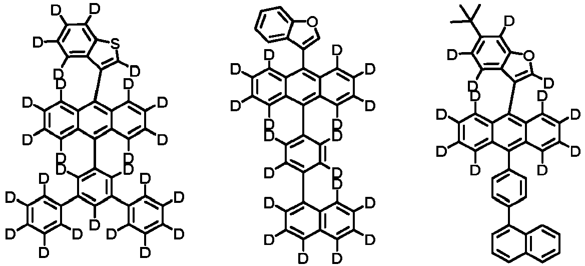

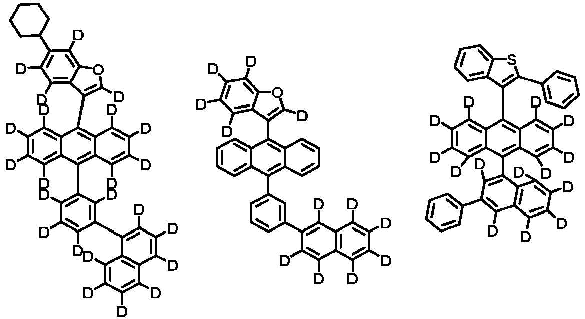

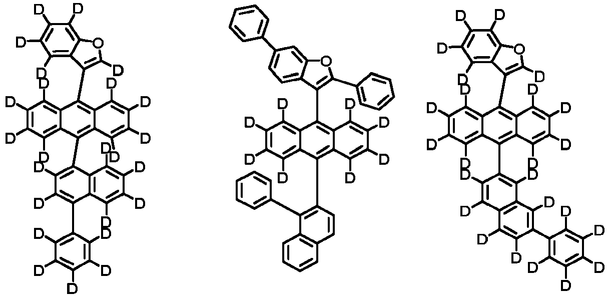

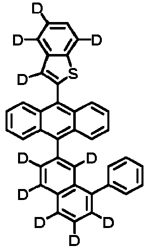

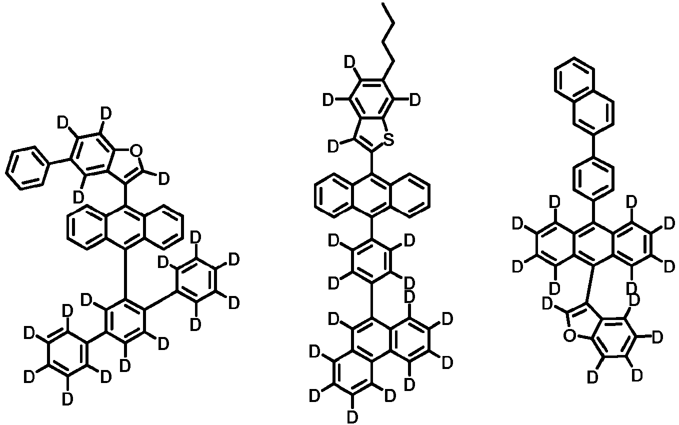

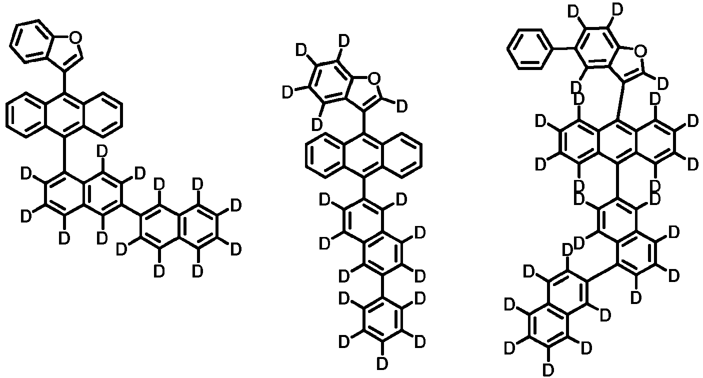

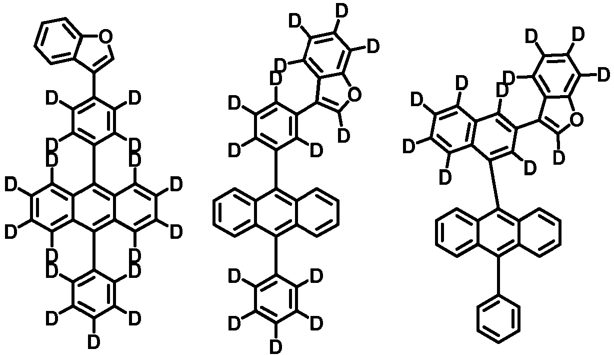

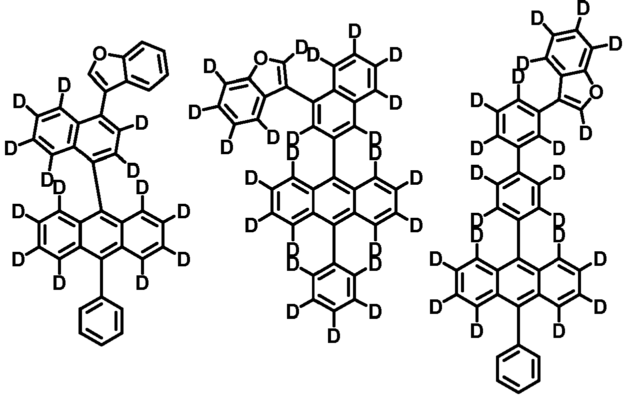

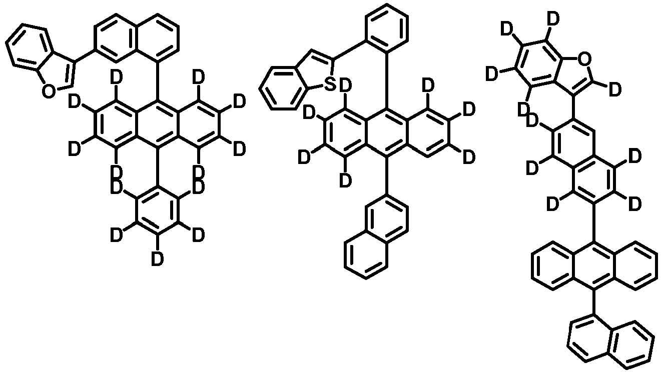

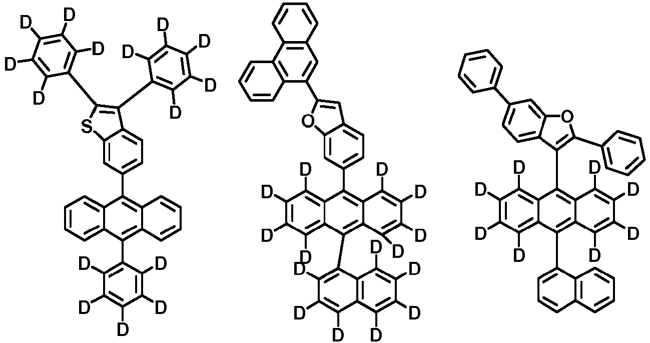

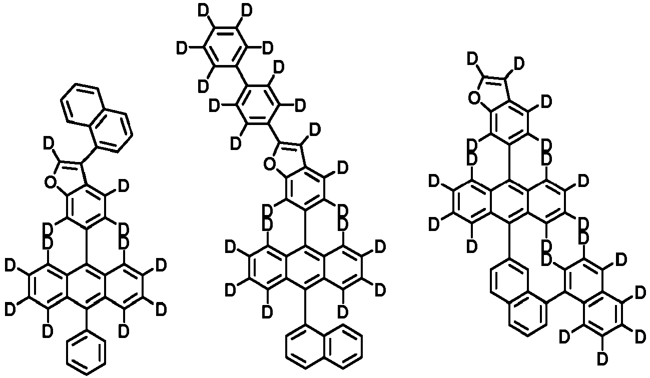

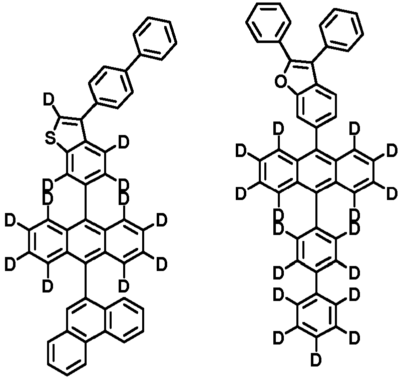

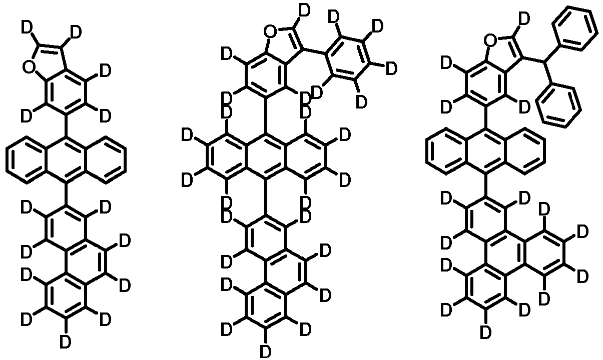

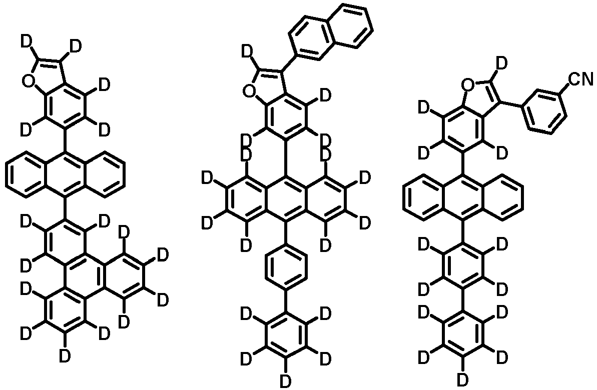

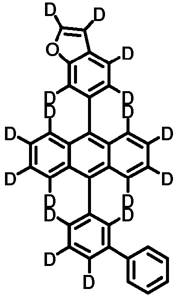

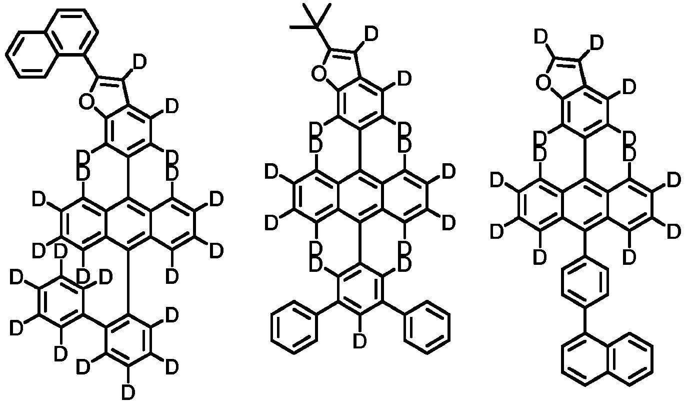

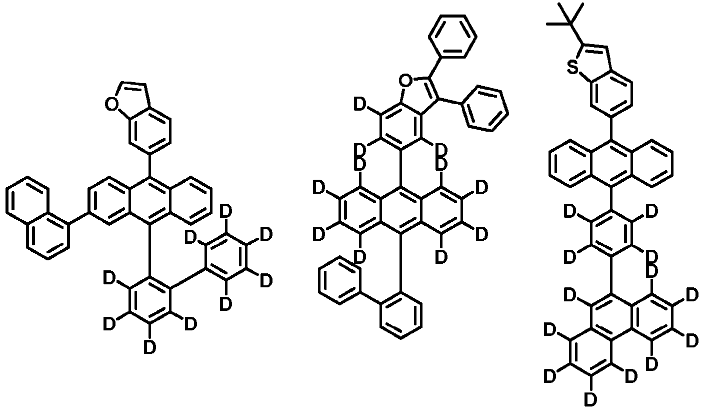

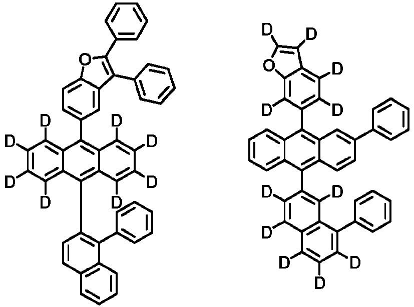

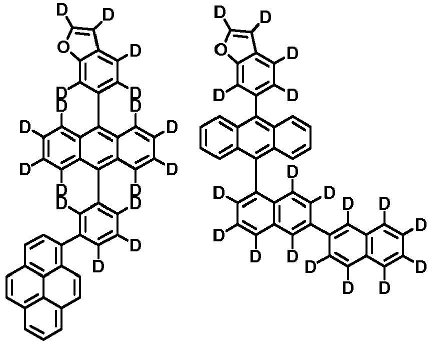

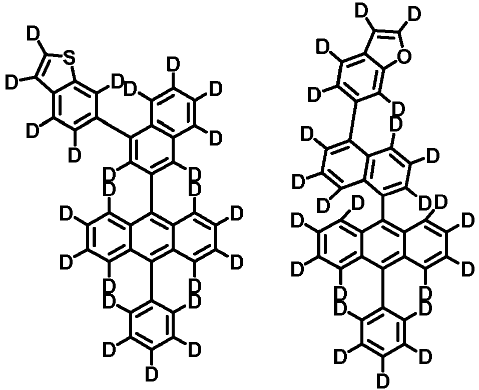

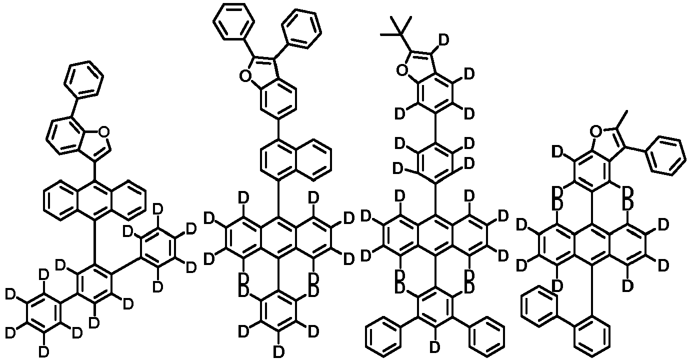

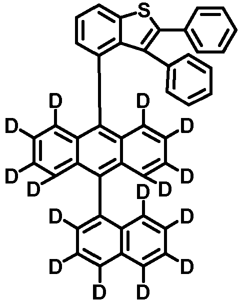

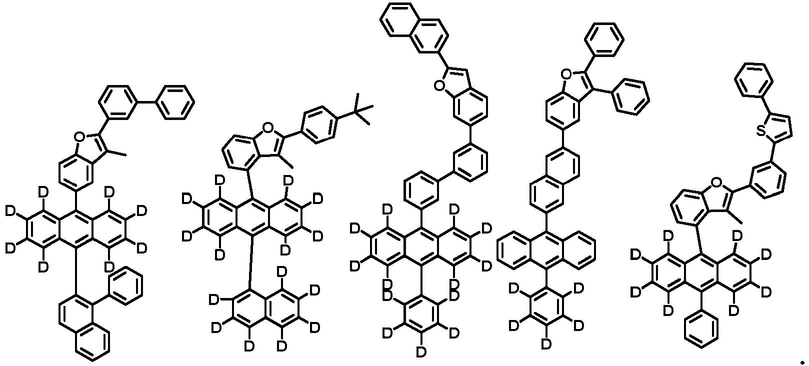

2. Dispositif électroluminescent organique comprenant une première électrode, une deuxième électrode, et une ou plusieurs couches de matériau organique disposées entre la première électrode et la deuxième électrode ;
dans lequel lesdites une ou plusieurs couches de matériau organique comprennent une couche électroluminescente et une couche de transport d'électrons ;
la couche électroluminescente comprend un composé de l'une quelconque des Formules chimiques 1-1 à 1-5 suivantes en tant que composé hôte ; et
la couche de transport d'électrons comprend un composé de la Formule chimique ET-1 suivante : 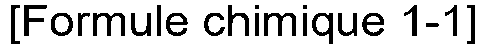 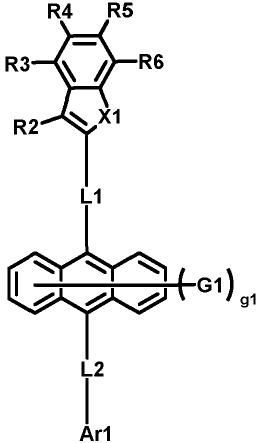 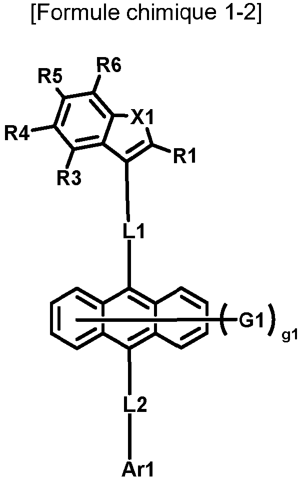 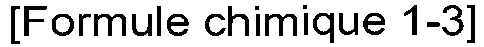 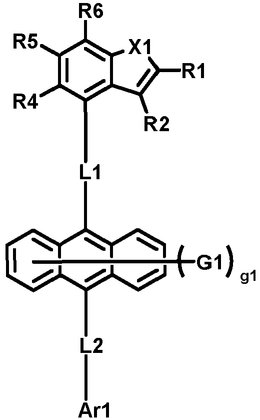 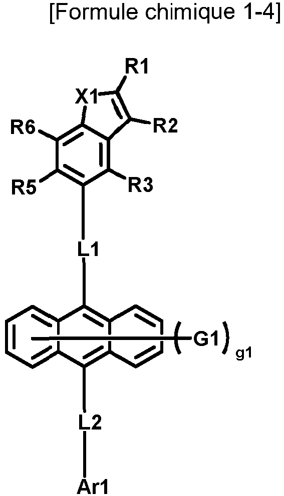 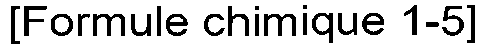 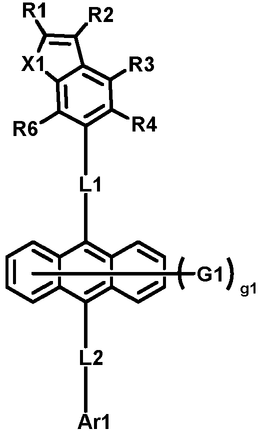
dans les formules chimiques 1-1 à 1-5,
R1 à R6 sont identiques ou différents les uns des autres, et sont chacun indépendamment hydrogène, deutérium, un groupe halogène, un groupe cyano, un groupe alkyle substitué ou non substitué, un groupe cycloalkyle substitué ou non substitué, un groupe alcoxy substitué ou non substitué, un groupe alcényle substitué ou non substitué, un groupe silyle substitué ou non substitué, un groupe aryle substitué ou non substitué, ou un groupe hétérocyclique substitué ou non substitué ;
L1 et L2 sont identiques ou différents les uns des autres, et sont chacun indépendamment une liaison directe, un groupe arylénique substitué ou non substitué, ou un groupe hétérocyclique divalent substitué ou non substitué ;
Ar1 est un groupe aryle substitué ou non substitué ;
chaque G1 est indépendamment hydrogène, deutérium, un groupe halogène, un groupe cyano, un groupe alkyle substitué ou non substitué, un groupe cycloalkyle substitué ou non substitué, un groupe alcoxy substitué ou non substitué, un groupe alcényle substitué ou non substitué, un groupe silyle substitué ou non substitué, un groupe aryle substitué ou non substitué, ou un groupe hétérocyclique substitué ou non substitué ;
g1 est 8 ;
les G1 sont identiques ou différents les uns des autres ;
dans les Formules chimiques 1-2 à 1-6, X1 est O ou S ;
dans la Formule chimique 1-1, X1 est S ;
dans les Formules chimiques 1-3 et 1-4, R2 est un groupe alkyle substitué ou non substitué, un groupe cycloalkyle substitué ou non substitué, un groupe alcoxy substitué ou non substitué, un groupe alcényle substitué ou non substitué, un groupe silyle substitué ou non substitué, un groupe aryle substitué ou non substitué, ou un groupe hétérocyclique substitué ou non substitué ; et
les Formules chimiques 1-1 à 1-5 comprennent du deutérium, le taux de substitution en deutérium étant de 40 % à 100 % ; 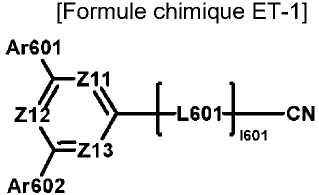
dans la Formule chimique ET-1,
au moins un parmi Z11 à Z13 est N, et les autres sont CH ;
L601 est une liaison directe, un groupe arylénique substitué ou non substitué, ou un groupe hétéroarylène substitué ou non substitué ;
Ar601 et Ar602 sont identiques ou différents les uns des autres, et sont chacun indépendamment un groupe aryle substitué ou non substitué, ou un groupe hétéroaryle substitué ou non substitué ; et
l601 est un nombre entier de 1 à 5, et lorsque l601 est 2 ou supérieur, les deux L601 ou plus sont identiques ou différents les uns des autres.

3. Dispositif électroluminescent organique selon la revendication 2, dans lequel la couche électroluminescente comprend un dopant fluorescent qui comprend un ou plusieurs composés parmi un composé à base de pyrène et un composé à base de bore.

4. Dispositif électroluminescent organique selon la revendication 2, dans lequel la couche électroluminescente comprend en outre un ou plusieurs hôtes différents du composé de l'une quelconque des Formules chimiques 1-1 à 1-5.

5. Dispositif électroluminescent organique selon la revendication 2, dans lequel
R1 à R6 sont identiques ou différents les uns des autres, et sont chacun indépendamment hydrogène, deutérium ; un groupe alkyle à chaîne linéaire ou ramifiée ayant 1 à 30 atomes de carbone, qui est non substitué ou substitué par du deutérium ou un groupe aryle monocyclique ou polycyclique ayant 6 à 30 atomes de carbone ; un groupe cycloalkyle monocyclique ou polycyclique ayant 3 à 30 atomes de carbone, qui est non substitué ou substitué par du deutérium ;
un groupe alkylsilyle à chaîne linéaire ou ramifiée ayant 1 à 30 atomes de carbone ; un groupe aryle monocyclique ou polycyclique ayant 6 à 30 atomes de carbone, qui est non substitué ou substitué par du deutérium, un groupe cyano, un groupe alkyle à chaîne linéaire ou ramifiée ayant 1 à 30 atomes de carbone, ou un groupe hétérocyclique monocyclique ou polycyclique ayant 2 à 30 atomes de carbone, qui est non substitué ou substitué par un groupe aryle monocyclique ou polycyclique ayant 6 à 30 atomes de carbone ; ou un groupe hétéroaryle monocyclique ou polycyclique ayant 2 à 30 atomes de carbone, qui est non substitué ou substitué par du deutérium ;
L1 et L2 sont identiques ou différents les uns des autres, et sont chacun indépendamment une liaison directe ou un groupe arylénique monocyclique ou polycyclique ayant 6 à 30 atomes de carbone, qui est non substitué ou substitué par du deutérium,
G1 est hydrogène, deutérium, ou un groupe aryle monocyclique ou polycyclique ayant 6 à 30 atomes de carbone, qui est non substitué ou substitué par du deutérium ;
Ar1 est un groupe aryle monocyclique ou polycyclique ayant 6 à 30 atomes de carbone, qui est non substitué ou substitué par du deutérium ; et
dans les Formules chimiques 1-3 et 1-4, R2 est un groupe alkyle à chaîne linéaire ou ramifiée ayant 1 à 30 atomes de carbone, qui est non substitué ou substitué par du deutérium ou un groupe aryle monocyclique ou polycyclique ayant 6 à 30 atomes de carbone ; un groupe cycloalkyle monocyclique ou polycyclique ayant 3 à 30 atomes de carbone, qui est non substitué ou substitué par du deutérium ; un groupe alkylsilyle à chaîne linéaire ou ramifiée ayant 1 à 30 atomes de carbone ;
un groupe aryle monocyclique ou polycyclique ayant 6 à 30 atomes de carbone, qui est non substitué ou substitué par du deutérium, un groupe cyano, ou un groupe alkyle à chaîne linéaire ou ramifiée ayant 1 à 30 atomes de carbone ; ou un groupe hétéroaryle monocyclique ou polycyclique ayant 2 à 30 atomes de carbone, qui est non substitué ou substitué par du deutérium.

6. Dispositif électroluminescent organique selon la revendication 2, dans lequel le composé de l'une quelconque des Formules chimiques 1-1 à 1-5 est sélectionné parmi les composés suivants :
